(19) Europäisches Patentamt
European Patent Office
Office européen des brevets

(11) EP 3 153 592 A1

(12) **EUROPEAN PATENT APPLICATION**

(43) Date of publication:
**12.04.2017 Bulletin 2017/15**

(51) Int Cl.:
***C12Q 1/68*** *(2006.01)*

(21) Application number: **16194527.4**

(22) Date of filing: **03.08.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **04.08.2009 EP 09167184**

(62) Document number(s) of the earlier application(s) in accordance with Art. 76 EPC:
**15151581.4 / 2 894 231**
**10744513.2 / 2 462 242**

(71) Applicants:
- **F. Hoffmann-La Roche AG**
  **4070 Basel (CH)**
- **VIB vzw**
  **9052 Gent (BE)**
- **Life Sciences Research Partners VZW**
  **3000 Leuven (BE)**

(72) Inventors:
- **FOERNZLER, Dorothee**
  **5600 Lenzburg (CH)**
- **DELMAR, Paul**
  **4057 Basel (CH)**
- **SCHERER, Stefan**
  **4053 Basel (CH)**
- **LAMBRECHTS, Diether**
  **3010 Kessel-Lo (BE)**
- **CARMELIET, Peter**
  **3052 Blanden (BE)**

(74) Representative: **Vossius & Partner**
**Patentanwälte Rechtsanwälte mbB**
**Siebertstrasse 3**
**81675 München (DE)**

Remarks:
This application was filed on 19.10.2016 as a divisional application to the application mentioned under INID code 62.

# (54) RESPONSIVENESS TO ANGIOGENESIS INHIBITORS

(57) The present invention relates to methods for improving the overall survival of a patient suffering from a malignant disease or a disease involving physiological and pathological angiogenesis by treatment with an angiogenesis inhibitor, such as bevacizumab, by determining the presence of one or more variant alleles of the vascular endothelial growth factor receptor 1 (VEGFR-1) gene. The present invention further provides methods for improving the progression-free survival of a patient suffering from a malignant disease or a disease involving physiological and pathological angiogenesis by treatment with an angiogenesis inhibitor, such as bevacizumab, by determining the presence of one or more variant alleles of the VEGFR-1 gene. The present invention also provides for methods for assessing the responsiveness of a patient to an angiogenesis inhibitor by determining the presence of one or more variant alleles of the VEGFR-1 gene.



Printed by Jouve, 75001 PARIS (FR)

## Description

The present invention relates to a method for improving overall survival of a patient suffering from a malignant disease or a disease involving physiological and pathological angiogenesis by treatment with an angiogenesis inhibitor, such as bevacizumab, by determining the presence of one or more variant alleles of the vascular endothelial growth factor receptor 1 (VEGFR-1) gene. The present invention also relates to a method for improving progression-free survival of a patient suffering from a malignant disease or a disease involving physiological and pathological angiogenesis by treatment with an angiogenesis inhibitor, such as bevacizumab, by determining the presence of one or more variant alleles of the vascular endothelial growth factor receptor 1 (VEGFR-1) gene. The present invention further provides for methods for assessing the responsiveness of a patient to an angiogenesis inhibitor by determining the presence of one or more variant alleles of the vascular endothelial growth factor receptor 1 (VEGFR-1) gene.

Angiogenesis contributes to benign and malignant diseases such as cancer development and, especially in cancer, is necessary for primary tumor growth, invasiveness and metastasis. In order to grow, a tumor must undergo an angiogenic switch. Vascular endothelial growth factor (VEGF) is required to induce this angiogenic switch. VEGF and the genes in the VEGF pathway are considered important mediators of cancer progression. The VEGF gene family includes the VEGF gene, also referred to as VEGFA, homologues to VEGF including, placenta growth factor (P1GF), VEGFB, VEGFC, VEGFD, the VEGF receptors, including VEGFR-1 and VEGFR-2 (also referred to as FLT1 and FLK1/KDR, respectively), the VEGF inducers, including hypoxia-inducible factors HIF1$\alpha$, HIF2$\alpha$, and the oxygen sensors PHD1, PHD2 and PHD3.

The importance of this pathway in cancer cell growth and metastasis has led to the development of anti-angiogenesis agents for use in cancer therapy. These therapies include, among others, bevacizumab, pegaptanib, sunitinib, sorafenib and vatalanib. Despite significantly prolonged survival obtained with angiogenesis inhibitors, such as bevacizumab, patients still succumb to cancer. Further, not all patients respond to angiogenesis inhibitor therapy. The mechanism underlying the non-responsiveness remains unknown. Moreover, angiogenesis inhibitor therapy is associated with side effects, such as gastrointestinal perforation, thrombosis, bleeding, hypertension and proteinuria.

Accordingly, there is a need for methods of determining which patients respond particular well to angiogenesis inhibitor therapy.

The present invention, therefore, provides a method for improving the overall survival of a patient suffering from a malignant disease or a disease involving physiological and pathological angiogenesis by treatment with an angiogenesis inhibitor, said method comprising the following steps:

(a) determining from a patient sample if the patient suffering from a malignant disease or a disease involving physiological and pathological angiogenesis has one or more variant alleles of the VEGFR-1 gene; and
(b) administering an angiogenesis inhibitor to the patient having one or more variant alleles of the VEGFR-1 gene.

The malignant disease or a disease involving physiological and pathological angiogenesis may be pancreatic cancer. The angiogenesis inhibitor may be bevacizumab.

Accordingly, the present invention relates to a method for improving the overall survival of a patient suffering from a malignant disease or a disease involving physiological and pathological angiogenesis by treatment with an angiogenesis inhibitor, said method comprising the following steps:

(a) obtaining a sample from a patient suffering from a malignant disease or a disease involving physiological and pathological angiogenesis;
(b) determining if the patient has one or more variant alleles of the VEGFR-1 gene; and
(c) administering an angiogenesis inhibitor to the patient having one or more variant alleles of the VEGFR-1 gene.

The malignant disease or a disease involving physiological and pathological angiogenesis may be pancreatic cancer. The angiogenesis inhibitor may be bevacizumab.

Therefore, the present invention relates to a method for improving the progression-free survival of a patient suffering from a malignant disease or a disease involving physiological and pathological angiogenesis by treatment with an angiogenesis inhibitor, said method comprising the following steps:

(a) determining from a patient sample if the patient suffering from a malignant disease or a disease involving physiological and pathological angiogenesis has one or more variant alleles of the VEGFR-1 gene; and
(b) administering an angiogenesis inhibitor to the patient having one or more variant alleles of the VEGFR-1 gene.

The malignant disease or a disease involving physiological and pathological angiogenesis may be pancreatic cancer or renal cell cancer. The angiogenesis inhibitor may be bevacizumab.

The present invention relates to a method for improving the progression-free survival of a patient suffering from a malignant disease or a disease involving physiological and pathological angiogenesis by treatment with an angiogenesis inhibitor, said method comprising the following steps:

(a) obtaining a sample from a patient suffering from a malignant disease or a disease involving physiological and pathological angiogenesis;
(b) determining if the patient has one or more variant alleles of the VEGFR-1 gene; and
(c) administering an angiogenesis inhibitor to the patient having one or more variant alleles of the VEGFR-1 gene.

The malignant disease or a disease involving physiological and pathological angiogenesis may be pancreatic cancer or renal cell cancer. The angiogenesis inhibitor may be bevacizumab.

The present invention provides an in vitro method for the identification of a responder for or a patient sensitive to an angiogenesis inhibitor, said method comprising determining from a patient sample if the patient suffering from a malignant disease or a disease involving physiological and pathological angiogenesis has one or more variant alleles of the VEGFR-1 gene whereby the presence of the one or more variant alleles of the VEGFR-1 gene is indicative for a responding patient or is indicative of a sensitivity of the patient to the angiogenesis inhibitor. The malignant disease or a disease involving physiological and pathological angiogenesis may be pancreatic cancer or renal cell cancer. The angiogenesis inhibitor may be bevacizumab.

Accordingly, the present invention relates to an in vitro method for the identification of a responder for or a patient sensitive to an angiogenesis inhibitor, said method comprising the following steps:

(a) obtaining a sample from a patient suffering from a malignant disease or a disease involving physiological and pathological angiogenesis; and
(b) determining if the patient has one or more variant alleles of the VEGFR-1 gene;

whereby the presence of the one or more variant alleles of the VEGFR-1 gene is indicative for a responding patient or is indicative for a sensitivity for the patient to the angiogenesis inhibitor. The malignant disease or a disease involving physiological and pathological angiogenesis may be pancreatic cancer or renal cell cancer. The angiogenesis inhibitor may be bevacizumab.

In accordance with the embodiments herein disclosed, the present invention provides the means and methods of identification of a patient or group of patients suffering from a malignant disease or a disease involving physiological and pathological angiogenesis who benefit from treatment with angiogenesis inhibitors, in particular to treatment with bevacizumab.

In the present invention, variations in the VEGFR-1 gene were surprisingly identified as markers/predictors for overall survival and/or progression-free survival to treatment with an angiogenesis inhibitor. The terms "marker" and "predictor" can be used interchangeably and refer to specific allele variants of genes, in particular the VEGFR-1 gene (see, for example, Ensembl ID ENSG00000102755). The variation or marker may also be referred to as a single nucleotide polymorphism (SNP).

Preferably the one or more variant alleles are in the region encompassing the tyrosine kinase domain of the VEGFR-1 gene. In particular, preferably the one or more variant alleles are in the region encoding the tyrosine kinase domain of the VEGFR-1 gene. More preferable the one or more variant alleles are in the region encompassing exons 25 to 30 of the VEGFR-1 gene. Even more preferably, the one or more variant alleles are in an intron of the VEGFR-1 gene. Even more preferably the one or more variant alleles of the VEGFR-1 gene are selected from the group consisting of rs9582036 (SEQ ID NO. 2), rs9554316 (SEQ ID NO. 1), rs9554320 (SEQ ID NO. 4) and/or rs9513070 (SEQ ID NO. 3). Most preferably, the one or more variant alleles of the VEGFR-1 gene are selected from the group consisting of rs9582036 (SEQ ID NO. 2) and/or rs9554316 (SEQ ID NO. 1) or the group consisting of rs9554316 (SEQ ID NO. 1) and/or rs9513070 (SEQ ID NO. 3).

Accordingly, the invention provides a method for improving overall survival of a patient suffering from a metastatic pancreatic cancer by treatment with an angiogenesis inhibitor comprising the following steps:

(a) determining from a patient sample if the patient suffering metastatic pancreatic cancer has one or more variant alleles of the VEGFR-1 gene, wherein the one or more variant alleles are selected from the group consisting of SNPs rs9554316 (SEQ ID NO. 1), rs9582036 (SEQ ID NO. 2), rs9513070 (SEQ ID NO. 3) and rs9554320 (SEQ ID NO.4); and
(b) administering the angiogenesis inhibitor to the patient having one or more variant alleles of the VEGFR-1 gene.

The angiogenesis inhibitor may be bevacizumab.

The invention relates to a method for improving overall survival of a patient suffering from a metastatic pancreatic cancer by treatment with an angiogenesis inhibitor comprising the following steps:

(a) obtaining a sample from a patient suffering from metastatic pancreatic cancer;
(b) determining from a patient sample if the patient has one or more variant alleles of the VEGFR-1 gene wherein the one or more variant alleles are selected from the group consisting of SNPs rs9554316 (SEQ ID NO. 1), rs9582036 (SEQ ID NO. 2), rs9513070 (SEQ ID NO. 3) and rs9554320 (SEQ ID NO.4); and
(c) administering the angiogenesis inhibitor to the patient having one or more variant alleles of the VEGFR-1 gene.

The angiogenesis inhibitor may be bevacizumab.

The invention relates to a method for improving progression-free survival of a patient suffering from a metastatic pancreatic cancer by treatment with an angiogenesis inhibitor comprising the following steps:

(a) determining from a patient sample if the patient suffering metastatic pancreatic cancer has one or more variant alleles of the VEGFR-1 gene wherein the one or more variant alleles are selected from the group consisting of rs9554316 (SEQ ID NO. 1), SNPs rs9582036 (SEQ ID NO. 2), rs9513070 (SEQ ID NO. 3) and rs9554320 (SEQ ID NO.4); and
(b) administering the angiogenesis inhibitor to the patient having one or more variant alleles of the VEGFR-1 gene.

The angiogenesis inhibitor may be bevacizumab.

The invention relates to a method for improving progression-free survival of a patient suffering from a metastatic pancreatic cancer by treatment with an angiogenesis inhibitor comprising the following steps:

(a) obtaining a sample from a patient suffering from metastatic pancreatic cancer;
(b) determining from a patient sample if the patient has one or more variant alleles of the VEGFR-1 gene wherein the one or more variant alleles are selected from the group consisting of SNPs rs9554316 (SEQ ID NO. 1), rs9582036 (SEQ ID NO. 2), rs9513070 (SEQ ID NO. 3) and rs9554320 (SEQ ID NO.4); and
(c) administering the angiogenesis inhibitor to the patient having one or more variant alleles of the VEGFR-1 gene.

The angiogenesis inhibitor may be bevacizumab.

Accordingly, the invention provides a method for improving progression-free survival of a patient suffering from a renal cell cancer by treatment with an angiogenesis inhibitor comprising the following steps:

(a) determining from a patient sample if the patient renal cell cancer has one or more variant alleles of the VEGFR-1 gene wherein the one or more variant alleles are selected from the group consisting of rs9554316 (SEQ ID NO. 1) and rs9513070 (SEQ ID NO. 3); and
(b) administering the angiogenesis inhibitor to the patient having one or more variant alleles of the VEGFR-1 gene.

The angiogenesis inhibitor may be bevacizumab.

The invention relates a method for improving progression-free survival of a patient suffering from a renal cell cancer by treatment with an angiogenesis inhibitor comprising the following steps:

(a) obtaining a sample from a patient suffering from renal cell cancer;
(b) determining from a patient sample if the patient has one or more variant alleles of the VEGFR-1 gene wherein the one or more variant alleles are selected from the group consisting of rs9554316 (SEQ ID NO. 1) and rs9513070 (SEQ ID NO. 3); and
(c) administering the angiogenesis inhibitor to the patient having one or more variant alleles of the VEGFR-1 gene.

The angiogenesis inhibitor may be bevacizumab.

Besides the SNPs herein described, also with the scope of this invention are SNPs that are linked to the SNPs herein described. Linkage disequilibrium is defined in the context of the present invention as having a D'>0.8. To determine these estimates of disequilibrium ($D_{ij}$) between pair-wise combinations of alleles the following definition was used: $D_{ij} = h_{ij} - p_i q_j$ whereby $h_{ij}$ represents the frequency of the observed haplotype with allele i at locus 1 and allele j at locus 2, and $p_i$ and $q_j$ the frequencies of both alleles. The linkage disequilibrium coefficient $D'_{ij}$ is standardized by its maximum value as $D' = D/D_{max}$. If D < 0, then $D_{max}$ = - pq; if D > 0, then $D_{max}$ = p (1- q). The linked SNPs identified according to this definition may be located within the region encompassing exons 25 to 30 of the VEGFR-1 gene but also upstream or downstream from the region encompassing exons 25 to 30 of the VEGFR-1 gene. For example, using this method of defining linkage disequilibrium, linked SNPs include rs17619037 (SEQ ID NO. 5), rs9579177 (SEQ ID NO. 6), rs9579176 (SEQ ID NO. 7), rs9554319 (SEQ ID NO. 8), rs7996030 (SEQ ID NO. 9), rs9513075 (SEQ ID NO. 10), rs7993418 (SEQ ID NO. 11), rs9513071 (SEQ ID NO. 12), rs11620238 (SEQ ID NO. 13), rs17618631 (SEQ ID NO. 14), rs12429309 (SEQ ID NO. 15), rs7982251 (SEQ ID NO 16), rs9508016 (SEQ ID NO. 17), rs7982957 (SEQ ID NO. 18) and rs3794400 (SEQ ID NO. 19).

The person skilled in the art will understand that other well-accepted and/or related methods of determining linkage equilibrium may be applied as well to determine additional SNPs linked to the SNPs herein disclosed, e.g., SNPs rs9554316 (SEQ ID NO. 1), rs9582036 (SEQ ID NO. 2), rs9513070 (SEQ ID NO. 3) and/or rs9554320 (SEQ ID NO.4). In the context of the present invention, for example, the correlation coefficient $r^2$ was used as an additional measure. To determine estimates of correlation between pair-wise combinations of alleles the following definition can be used: D = h - $p_1q_1$ whereby h represents the frequency of the observed haplotype with allele 1 at locus 1 and allele 1 at locus 2, and $p_1$ and $q_1$ are the frequencies of both alleles. When $p_2$ and $q_2$ are respectively defined as (1-$p_1$) and (1-$q_1$), the correlation coefficient between pairs of loci can be defined as

$$r = \frac{D}{\sqrt{p_1 p_2 q_1 q_2}}$$

Accordingly, using this definition, linkage disequilibrium may be defined as having an $r^2$ equal or larger that 0.12 to rs9554316 (SEQ ID NO. 1), rs9582036 (SEQ ID NO. 2), rs9513070 (SEQ ID NO. 3) or rs9554320 (SEQ ID NO.4), for example. The threshold of $r^2$ equal or larger than 0.12 was applied because this $r^2$ value represents the lowest level of linkage disequilibrium between the four SNPs (SEQ ID NOs. 1 to 4) described within the scope of this invention and identified as markers/predictors for overall survival and/or progression-free survival to treatment with an angiogenesis inhibitor. Accordingly, using this alternative method of defining linkage disequilibrium, linked SNPs include, in addition to those identified above (i.e, SEQ ID NOs. 5 to 19), rs45455097 (SEQ ID NO. 40), rs1886233 (SEQ ID NO. 41), rs9554309 (SEQ ID NO. 42), rs11619230 (SEQ ID NO. 43), rs9554311 (SEQ ID NO. 44), rs4771233 (SEQ ID NO. 45), rs6491274 (SEQ ID NO. 46), rs7982639 (SEQ ID NO. 47), rs12877718 (SEQ ID NO. 48), rs10507382 (SEQ ID NO. 49), rs57354941 (SEQ ID NO. 50), rs17086497 (SEQ ID NO. 51), rs3794395 (SEQ ID NO. 52), rs9554317 (SEQ ID NO. 53), rs9513073 (SEQ ID NO. 54), rs9513074 (SEQ ID NO. 55), rs9508015 (SEQ ID NO. 56), rs2011950 (SEQ ID NO. 57), rs9513110 (SEQ ID NO. 58), rs9513112 (SEQ ID NO. 59), rs9513113 (SEQ ID NO. 60), rs9551471 (SEQ ID NO. 61), rs2296285 (SEQ ID NO. 62), rs9513116 (SEQ ID NO. 63), rs9551473 (SEQ ID NO. 64), rs7330109 (SEQ ID NO. 65), rs9508037 (SEQ ID NO. 66), rs1924981 (SEQ ID NO. 67), rs34140996 (SEQ ID NO. 68), rs7985584 (SEQ ID NO. 69), rs7992940 (SEQ ID NO. 70) and rs718273 (SEQ ID NO. 71).

In the context of the herein described invention, the linked SNPs herein described may be used alone, in combination with SNPs rs9554316 (SEQ ID NO. 1), rs9582036 (SEQ ID NO. 2), rs9513070 (SEQ ID NO. 3) and/or rs9554320 (SEQ ID NO.4) or in combination with other linked SNPs herein described in the methods and or uses herein described.

Accordingly, the present invention relates to variations in the VEGFR-1 gene including nucleotide substitution(s), deletion(s) or addition(s) at the position corresponding to the SNP. For example, for rs9582036 (SEQ ID NO. 2), rs9554316 (SEQ ID NO. 1), rs9554320 (SEQ ID NO. 4) and/or rs9513070 (SEQ ID NO. 3), the variations include a substitution(s), deletion(s) or addition(s) at position 51 of the rs9554316 (SEQ ID NO. 1), rs9582036 (SEQ ID NO. 2), rs9513070 (SEQ ID NO. 3) and/or rs9554320 (SEQ ID NO. 4). Accordingly, in a further embodiment of the present invention the one or more variant alleles of the VEGFR-1 gene are selected from the group consisting of:

(a) a sequence corresponding to rs9554316 (SEQ ID NO. 1) or having at least 80%, at least 85%, or more preferably at least 90%, or even more preferably, at least 95%, at least 96%, at least 97%, at least 98%, or at least 99% homology to rs9554316 (SEQ ID NO. 1) having a substitution, deletion or addition of at least one nucleotide corresponding to position 51 of rs9554316 (SEQ ID NO. 1);
(b) a sequence corresponding to rs9582036 (SEQ ID NO. 2) or having at least 80%, at least 85%, or more preferably at least 90%, or even more preferably, at least 95%, at least 96%, at least 97%, at least 98%, or at least 99% homology to rs9582036 (SEQ ID NO. 2) having a substitution, deletion or addition of at least one nucleotide corresponding to position 51 of rs9582036 (SEQ ID NO. 2);
(c) a sequence corresponding to rs9513070 (SEQ ID NO. 3) or having at least 80%, at least 85%, or more preferably at least 90%, or even more preferably, at least 95%, at least 96%, at least 97%, at least 98%, or at least 99% homology to rs9513070 (SEQ ID NO. 3) having a substitution, deletion or addition of at least one nucleotide corresponding to position 51 of rs9513070 (SEQ ID NO. 3); and
(d) a sequence corresponding to rs9554320 (SEQ ID NO. 4) or having at least 80%, at least 85%, or more preferably at least 90%, or even more preferably, at least 95%, at least 96%, at least 97%, at least 98%, or at least 99% homology to rs9554320 (SEQ ID NO. 4) having a substitution, deletion or addition of at least one nucleotide corresponding to position 51 of rs9554320 (SEQ ID NO. 4).

In the context of the herein described invention, the above described homology, with regard to the identified SNPs, applies to each embodiment herein described wherein specific SNPs are recited.

Further, the variations can include a substitution(s), deletion(s) or addition(s) at any position from position 1 to 50 and 52 to 101 of the rs9554316 (SEQ ID NO. 1), rs9582036 (SEQ ID NO. 2), rs9513070 (SEQ ID NO. 3) and/or rs9554320 (SEQ ID NO. 4). Accordingly, in a further embodiment of the present invention the one or more variant alleles of the VEGFR-1 gene are selected from the group consisting of:

(a) a sequence corresponding to rs9554316 (SEQ ID NO. 1) or having at least 80%, at least 85%, or more preferably at least 90%, or even more preferably, at least 95%, at least 96%, at least 97%, at least 98%, or at least 99% homology to rs9554316 (SEQ ID NO. 1), wherein position 51 is a T or G and having a substitution, deletion or addition of at least one nucleotide at any position from position 1 to 50 and 52 to 101;
(b) a sequence corresponding to rs9582036 (SEQ ID NO. 2) or having at least 80%, at least 85%, or more preferably at least 90%, or even more preferably, at least 95%, at least 96%, at least 97%, at least 98%, or at least 99% homology to rs9582036 (SEQ ID NO. 2) wherein position 51 is a C or A and having a substitution, deletion or addition of at least one nucleotide at any position from position 1 to 50 and 52 to 101;
(c) a sequence corresponding to rs9513070 (SEQ ID NO. 3) or having at least 80%, at least 85%, or more preferably at least 90%, or even more preferably, at least 95%, at least 96%, at least 97%, at least 98%, or at least 99% homology to rs9513070 (SEQ ID NO. 3) wherein position 51 is a G or A and having a substitution, deletion or addition of at least one nucleotide at any position from position 1 to 50 and 52 to 101; and
(d) a sequence corresponding to rs9554320 (SEQ ID NO. 4) or having at least 80%, at least 85%, or more preferably at least 90%, or even more preferably, at least 95%, at least 96%, at least 97%, at least 98%, or at least 99% homology to rs9554320 (SEQ ID NO. 4) wherein position 51 is a C or A and having a substitution, deletion or addition of at least one nucleotide at any position from position 1 to 50 and 52 to 101.

In the context of the herein described invention, the above described homology, with regard to the identified SNPs, applies to each embodiment herein described wherein specific SNPs are recited.

The present invention also relates to variations in the VEGFR-1 gene including nucleotide substitution(s), deletion(s) or addition(s) at the position corresponding to linked SNPs as well as other linked SNPs nearby but upstream or downstream from the VEGFR-1 gene, and located in the poly(A) specific ribonuclease subunit gene, Ensembl ID ENSG00000152520, ("PAN3 gene") or in intergenic DNA regions. For example, variations in the VEGFR-1 gene include the linked SNPs rs17619037 (SEQ ID NO. 5), rs9579177 (SEQ ID NO. 6), rs9579176 (SEQ ID NO. 7), rs9554319 (SEQ ID NO. 8), rs7996030 (SEQ ID NO. 9), rs9513075 (SEQ ID NO. 10), rs7993418 (SEQ ID NO. 11), rs9513071 (SEQ ID NO. 12), rs12429309 (SEQ ID NO. 15), rs7982251 (SEQ ID NO 16), rs9508016 (SEQ ID NO. 17), rs7982957 (SEQ ID NO. 18), rs3794400 (SEQ ID NO. 19), rs3794395 (SEQ ID NO. 52), rs9554317 (SEQ ID NO. 53), rs9513073 (SEQ ID NO. 54), rs9513074 (SEQ ID NO. 55), rs9508015 (SEQ ID NO. 56), rs2011950 (SEQ ID NO. 57), rs9513110 (SEQ ID NO. 58), rs9513112 (SEQ ID NO. 59), rs9513113 (SEQ ID NO. 60), rs9551471 (SEQ ID NO. 61), rs2296285 (SEQ ID NO. 62), rs9513116 (SEQ ID NO. 63), rs9551473 (SEQ ID NO. 64), rs7330109 (SEQ ID NO. 65), rs9508037 (SEQ ID NO. 66), rs1924981 (SEQ ID NO. 67), rs34140996 (SEQ ID NO. 68), rs7985584 (SEQ ID NO. 69), rs7992940 (SEQ ID NO. 70) and rs718273 (SEQ ID NO. 71), and variations upstream of the VEGFR-1 gene, including the linked SNPs corresponding to variations in the PAN3 gene, such as rs45455097 (SEQ ID NO. 40), rs1886233 (SEQ ID NO. 41), rs9554309 (SEQ ID NO. 42), rs11619230 (SEQ ID NO. 43), and rs9554311 (SEQ ID NO. 44), and the linked SNPs corresponding to variations in the intergenic DNA regions, such as rs11620238 (SEQ ID NO. 13), rs17618631 (SEQ ID NO. 14), rs4771233 (SEQ ID NO. 45), rs6491274 (SEQ ID NO. 46), rs7982639 (SEQ ID NO. 47), rs12877718 (SEQ ID NO. 48), rs10507382 (SEQ ID NO. 49), rs57354941 (SEQ ID NO. 50), and rs17086497 (SEQ ID NO. 51). Accordingly, the variations include a substitution(s), deletion(s) or addition(s) at position 51 of rs17619037 (SEQ ID NO. 5), rs9579177 (SEQ ID NO. 6), rs9579176 (SEQ ID NO. 7), rs9554319 (SEQ ID NO. 8), rs7996030 (SEQ ID NO. 9), rs9513075 (SEQ ID NO. 10), rs7993418 (SEQ ID NO. 11), rs9513071 (SEQ ID NO. 12), rs11620238 (SEQ ID NO. 13), rs17618631 (SEQ ID NO. 14), rs12429309 (SEQ ID NO. 15), rs7982251 (SEQ ID NO 16), rs9508016 (SEQ ID NO. 17), rs7982957 (SEQ ID NO. 18), rs3794400 (SEQ ID NO. 19), rs45455097 (SEQ ID NO. 40), rs1886233 (SEQ ID NO. 41), rs9554309 (SEQ ID NO. 42), rs11619230 (SEQ ID NO. 43), rs9554311 (SEQ ID NO. 44), rs4771233 (SEQ ID NO. 45), rs6491274 (SEQ ID NO. 46), rs7982639 (SEQ ID NO. 47), rs12877718 (SEQ ID NO. 48), rs10507382 (SEQ ID NO. 49), rs57354941 (SEQ ID NO. 50), rs17086497 (SEQ ID NO. 51), rs3794395 (SEQ ID NO. 52), rs9554317 (SEQ ID NO. 53), rs9513073 (SEQ ID NO. 54), rs9513074 (SEQ ID NO. 55), rs9508015 (SEQ ID NO. 56), rs2011950 (SEQ ID NO. 57), rs9513110 (SEQ ID NO. 58), rs9513112 (SEQ ID NO. 59), rs9513113 (SEQ ID NO. 60), rs9551471 (SEQ ID NO. 61), rs2296285 (SEQ ID NO. 62), rs9513116 (SEQ ID NO. 63), rs9551473 (SEQ ID NO. 64), rs7330109 (SEQ ID NO. 65), rs9508037 (SEQ ID NO. 66), rs1924981 (SEQ ID NO. 67), rs34140996 (SEQ ID NO. 68), rs7985584 (SEQ ID NO. 69), rs7992940 (SEQ ID NO. 70) and rs718273 (SEQ ID NO. 71).

Accordingly, in an another embodiment of the present invention the one or more variant alleles of the VEGFR-1 gene include variations in the VEGFR-1 gene corresponding to linked SNPs as well as other linked SNPs upstream or downstream from VEGFR-1 gene corresponding to variations in the PAN3 gene and in intergenic DNAselected from the group consisting of:

(a) a sequence corresponding to rs17619037 (SEQ ID NO. 5) or having at least 80%, at least 85%, or more preferably at least 90%, or even more preferably, at least 95%, at least 96%, at least 97%, at least 98%, or at least 99% homology to rs17619037 (SEQ ID NO. 5) having a substitution, deletion or addition of at least one nucleotide corresponding to position 51 of rs17619037 (SEQ ID NO. 5);
(b) a sequence corresponding to rs9579177 (SEQ ID NO. 6) or having at least 80%, at least 85%, or more preferably at least 90%, or even more preferably, at least 95%, at least 96%, at least 97%, at least 98%, or at least 99% homology to rs9579177 (SEQ ID NO. 6) having a substitution, deletion or addition of at least one nucleotide corresponding to position 51 of rs9579177 (SEQ ID NO. 6);
(c) a sequence corresponding to rs9579176 (SEQ ID NO. 7) or having at least 80%, at least 85%, or more preferably at least 90%, or even more preferably, at least 95%, at least 96%, at least 97%, at least 98%, or at least 99% homology to rs9579176 (SEQ ID NO. 7) having a substitution, deletion or addition of at least one nucleotide corresponding to position 51 of rs9579176 (SEQ ID NO. 7);
(d) a sequence corresponding to rs9554319 (SEQ ID NO. 8) or having at least 80%, at least 85%, or more preferably at least 90%, or even more preferably, at least 95%, at least 96%, at least 97%, at least 98%, or at least 99% homology to rs9554319 (SEQ ID NO. 8) having a substitution, deletion or addition of at least one nucleotide corresponding to position 51 of rs9554319 (SEQ ID NO. 8);
(e) a sequence corresponding to rs7996030 (SEQ ID NO. 9) or having at least 80%, at least 85%, or more preferably at least 90%, or even more preferably, at least 95%, at least 96%, at least 97%, at least 98%, or at least 99% homology to rs7996030 (SEQ ID NO. 9) having a substitution, deletion or addition of at least one nucleotide corresponding to position 51 of rs7996030 (SEQ ID NO. 9);
(f) a sequence corresponding to rs9513075 (SEQ ID NO. 10) or having at least 80%, at least 85%, or more preferably at least 90%, or even more preferably, at least 95%, at least 96%, at least 97%, at least 98%, or at least 99% homology to rs9513075 (SEQ ID NO. 10) having a substitution, deletion or addition of at least one nucleotide corresponding to position 51 of rs9513075 (SEQ ID NO. 10);
(g) a sequence corresponding to rs7993418 (SEQ ID NO. 11) or having at least 80%, at least 85%, or more preferably at least 90%, or even more preferably, at least 95%, at least 96%, at least 97%, at least 98%, or at least 99% homology to rs7993418 (SEQ ID NO. 11) having a substitution, deletion or addition of at least one nucleotide corresponding to position 51 of rs7993418 (SEQ ID NO. 11);
(h) a sequence corresponding to rs9513071 (SEQ ID NO. 12) or having at least 80%, at least 85%, or more preferably at least 90%, or even more preferably, at least 95%, at least 96%, at least 97%, at least 98%, or at least 99% homology to rs9513071 (SEQ ID NO. 12) having a substitution, deletion or addition of at least one nucleotide corresponding to position 51 of rs9513071 (SEQ ID NO. 12);
(i) a sequence corresponding to rs11620238 (SEQ ID NO. 13) or having at least 80%, at least 85%, or more preferably at least 90%, or even more preferably, at least 95%, at least 96%, at least 97%, at least 98%, or at least 99% homology to rs11620238 (SEQ ID NO. 13) having a substitution, deletion or addition of at least one nucleotide corresponding to position 51 of rs11620238 (SEQ ID NO. 13);
(j) a sequence corresponding to rs17618631 (SEQ ID NO. 14) or having at least 80%, at least 85%, or more preferably at least 90%, or even more preferably, at least 95%, at least 96%, at least 97%, at least 98%, or at least 99% homology to rs17618631 (SEQ ID NO. 14) having a substitution, deletion or addition of at least one nucleotide corresponding to position 51 of rs17618631 (SEQ ID NO. 14);
(k) a sequence corresponding to rs12429309 (SEQ ID NO. 15) or having at least 80%, at least 85%, or more preferably at least 90%, or even more preferably, at least 95%, at least 96%, at least 97%, at least 98%, or at least 99% homology to rs12429309 (SEQ ID NO. 15) having a substitution, deletion or addition of at least one nucleotide corresponding to position 51 of rs12429309 (SEQ ID NO. 15);
(1) a sequence corresponding to rs7982251 (SEQ ID NO 16) or having at least 80%, at least 85%, or more preferably at least 90%, or even more preferably, at least 95%, at least 96%, at least 97%, at least 98%, or at least 99% homology to rs7982251 (SEQ ID NO 16) having a substitution, deletion or addition of at least one nucleotide corresponding to position 51 of rs7982251 (SEQ ID NO 16);
(m) a sequence corresponding to rs9508016 (SEQ ID NO. 17) or having at least 80%, at least 85%, or more preferably at least 90%, or even more preferably, at least 95%, at least 96%, at least 97%, at least 98%, or at least 99% homology to rs9508016 (SEQ ID NO. 17) having a substitution, deletion or addition of at least one nucleotide corresponding to position 51 of rs9508016 (SEQ ID NO. 17);

(n) a sequence corresponding to rs7982957 (SEQ ID NO. 18) or having at least 80%, at least 85%, or more preferably at least 90%, or even more preferably, at least 95%, at least 96%, at least 97%, at least 98%, or at least 99% homology to rs7982957 (SEQ ID NO. 18) having a substitution, deletion or addition of at least one nucleotide corresponding to position 51 of rs7982957 (SEQ ID NO. 18);
(o) a sequence corresponding to rs3794400 (SEQ ID NO. 19) or having at least 80%, at least 85%, or more preferably at least 90%, or even more preferably, at least 95%, at least 96%, at least 97%, at least 98%, or at least 99% homology to rs3794400 (SEQ ID NO. 19) having a substitution, deletion or addition of at least one nucleotide corresponding to position 51 of rs3794400 (SEQ ID NO. 19);
(p) a sequence corresponding to rs45455097 (SEQ ID NO. 40) or having at least 80%, at least 85%, or more preferably at least 90%, or even more preferably, at least 95%, at least 96%, at least 97%, at least 98%, or at least 99% homology to rs45455097 (SEQ ID NO. 40) having a substitution, deletion or addition of at least one nucleotide corresponding to position 51 of rs45455097 (SEQ ID NO. 40);
(q) a sequence corresponding to rs1886233 (SEQ ID NO. 41) or having at least 80%, at least 85%, or more preferably at least 90%, or even more preferably, at least 95%, at least 96%, at least 97%, at least 98%, or at least 99% homology to rs1886233 (SEQ ID NO. 41) having a substitution, deletion or addition of at least one nucleotide corresponding to position 51 of rs1886233 (SEQ ID NO. 41);
(r) a sequence corresponding to rs9554309 (SEQ ID NO. 42) or having at least 80%, at least 85%, or more preferably at least 90%, or even more preferably, at least 95%, at least 96%, at least 97%, at least 98%, or at least 99% homology to rs9554309 (SEQ ID NO. 42) having a substitution, deletion or addition of at least one nucleotide corresponding to position 51 of rs9554309 (SEQ ID NO. 42);
(s) a sequence corresponding to rs11619230 (SEQ ID NO. 43) or having at least 80%, at least 85%, or more preferably at least 90%, or even more preferably, at least 95%, at least 96%, at least 97%, at least 98%, or at least 99% homology to rs11619230 (SEQ ID NO. 43) having a substitution, deletion or addition of at least one nucleotide corresponding to position 51 of rs11619230 (SEQ ID NO. 43);
(t) a sequence corresponding to rs9554311 (SEQ ID NO. 44) or having at least 80%, at least 85%, or more preferably at least 90%, or even more preferably, at least 95%, at least 96%, at least 97%, at least 98%, or at least 99% homology to rs9554311 (SEQ ID NO. 44) having a substitution, deletion or addition of at least one nucleotide corresponding to position 51 of rs9554311 (SEQ ID NO. 44);
(u) a sequence corresponding to rs4771233 (SEQ ID NO. 45) or having at least 80%, at least 85%, or more preferably at least 90%, or even more preferably, at least 95%, at least 96%, at least 97%, at least 98%, or at least 99% homology to rs4771233 (SEQ ID NO. 45) having a substitution, deletion or addition of at least one nucleotide corresponding to position 51 of rs4771233 (SEQ ID NO. 45);
(v) a sequence corresponding to rs6491274 (SEQ ID NO. 46) or having at least 80%, at least 85%, or more preferably at least 90%, or even more preferably, at least 95%, at least 96%, at least 97%, at least 98%, or at least 99% homology to rs6491274 (SEQ ID NO. 46) having a substitution, deletion or addition of at least one nucleotide corresponding to position 51 of rs6491274 (SEQ ID NO. 46);
(w) a sequence corresponding to rs7982639 (SEQ ID NO. 47) or having at least 80%, at least 85%, or more preferably at least 90%, or even more preferably, at least 95%, at least 96%, at least 97%, at least 98%, or at least 99% homology to rs7982639 (SEQ ID NO. 47) having a substitution, deletion or addition of at least one nucleotide corresponding to position 51 of rs7982639 (SEQ ID NO. 47);
(x) a sequence corresponding to rs12877718 (SEQ ID NO. 48) or having at least 80%, at least 85%, or more preferably at least 90%, or even more preferably, at least 95%, at least 96%, at least 97%, at least 98%, or at least 99% homology to rs12877718 (SEQ ID NO. 48) having a substitution, deletion or addition of at least one nucleotide corresponding to position 51 of rs12877718 (SEQ ID NO. 48);
(y) a sequence corresponding to rs10507382 (SEQ ID NO. 49) or having at least 80%, at least 85%, or more preferably at least 90%, or even more preferably, at least 95%, at least 96%, at least 97%, at least 98%, or at least 99% homology to rs10507382 (SEQ ID NO. 49) having a substitution, deletion or addition of at least one nucleotide corresponding to position 51 of rs10507382 (SEQ ID NO. 49);
(z) a sequence corresponding to rs57354941 (SEQ ID NO. 50) or having at least 80%, at least 85%, or more preferably at least 90%, or even more preferably, at least 95%, at least 96%, at least 97%, at least 98%, or at least 99% homology to rs57354941 (SEQ ID NO. 50) having a substitution, deletion or addition of at least one nucleotide corresponding to position 51 of rs57354941 (SEQ ID NO. 50);
(aa) a sequence corresponding to rs17086497 (SEQ ID NO. 51) or having at least 80%, at least 85%, or more preferably at least 90%, or even more preferably, at least 95%, at least 96%, at least 97%, at least 98%, or at least 99% homology to rs17086497 (SEQ ID NO. 51) having a substitution, deletion or addition of at least one nucleotide corresponding to position 51 of rs17086497 (SEQ ID NO. 51);
(bb) a sequence corresponding to rs3794395 (SEQ ID NO. 52) or having at least 80%, at least 85%, or more preferably at least 90%, or even more preferably, at least 95%, at least 96%, at least 97%, at least 98%, or at least 99% homology to rs3794395 (SEQ ID NO. 52) having a substitution, deletion or addition of at least one nucleotide corresponding to position 51 of rs3794395 (SEQ ID NO. 52);

(cc) a sequence corresponding to rs9554317 (SEQ ID NO. 53) or having at least 80%, at least 85%, or more preferably at least 90%, or even more preferably, at least 95%, at least 96%, at least 97%, at least 98%, or at least 99% homology to rs9554317 (SEQ ID NO. 53) having a substitution, deletion or addition of at least one nucleotide corresponding to position 51 of rs9554317 (SEQ ID NO. 53);

(dd) a sequence corresponding to rs9513073 (SEQ ID NO. 54) or having at least 80%, at least 85%, or more preferably at least 90%, or even more preferably, at least 95%, at least 96%, at least 97%, at least 98%, or at least 99% homology to rs9513073 (SEQ ID NO. 54) having a substitution, deletion or addition of at least one nucleotide corresponding to position 51 of rs9513073 (SEQ ID NO. 54);

(ee) a sequence corresponding to rs9513074 (SEQ ID NO. 55) or having at least 80%, at least 85%, or more preferably at least 90%, or even more preferably, at least 95%, at least 96%, at least 97%, at least 98%, or at least 99% homology to rs9513074 (SEQ ID NO. 55) having a substitution, deletion or addition of at least one nucleotide corresponding to position 51 of rs9513074 (SEQ ID NO. 55);

(ff) a sequence corresponding to rs9508015 (SEQ ID NO. 56) or having at least 80%, at least 85%, or more preferably at least 90%, or even more preferably, at least 95%, at least 96%, at least 97%, at least 98%, or at least 99% homology to rs9508015 (SEQ ID NO. 56) having a substitution, deletion or addition of at least one nucleotide corresponding to position 51 of rs9508015 (SEQ ID NO. 56);

(gg) a sequence corresponding to rs2011950 (SEQ ID NO. 57) or having at least 80%, at least 85%, or more preferably at least 90%, or even more preferably, at least 95%, at least 96%, at least 97%, at least 98%, or at least 99% homology to rs2011950 (SEQ ID NO. 57) having a substitution, deletion or addition of at least one nucleotide corresponding to position 51 of rs2011950 (SEQ ID NO. 57);

(hh) a sequence corresponding to rs9513110 (SEQ ID NO. 58) or having at least 80%, at least 85%, or more preferably at least 90%, or even more preferably, at least 95%, at least 96%, at least 97%, at least 98%, or at least 99% homology to rs9513110 (SEQ ID NO. 58) having a substitution, deletion or addition of at least one nucleotide corresponding to position 51 of rs9513110 (SEQ ID NO. 58);

(ii) a sequence corresponding to rs9513112 (SEQ ID NO. 59) or having at least 80%, at least 85%, or more preferably at least 90%, or even more preferably, at least 95%, at least 96%, at least 97%, at least 98%, or at least 99% homology to rs9513112 (SEQ ID NO. 59) having a substitution, deletion or addition of at least one nucleotide corresponding to position 51 of rs9513112 (SEQ ID NO. 59);

(jj) a sequence corresponding to rs9513113 (SEQ ID NO. 60) or having at least 80%, at least 85%, or more preferably at least 90%, or even more preferably, at least 95%, at least 96%, at least 97%, at least 98%, or at least 99% homology to rs9513113 (SEQ ID NO. 60) having a substitution, deletion or addition of at least one nucleotide corresponding to position 51 of rs9513113 (SEQ ID NO. 60);

(kk) a sequence corresponding to rs9551471 (SEQ ID NO. 61) or having at least 80%, at least 85%, or more preferably at least 90%, or even more preferably, at least 95%, at least 96%, at least 97%, at least 98%, or at least 99% homology to rs9551471 (SEQ ID NO. 61) having a substitution, deletion or addition of at least one nucleotide corresponding to position 51 of rs9551471 (SEQ ID NO. 61);

(11) a sequence corresponding to rs2296285 (SEQ ID NO. 62) or having at least 80%, at least 85%, or more preferably at least 90%, or even more preferably, at least 95%, at least 96%, at least 97%, at least 98%, or at least 99% homology to rs2296285 (SEQ ID NO. 62) having a substitution, deletion or addition of at least one nucleotide corresponding to position 51 of rs2296285 (SEQ ID NO. 62);

(mm) a sequence corresponding to rs9513116 (SEQ ID NO. 63) or having at least 80%, at least 85%, or more preferably at least 90%, or even more preferably, at least 95%, at least 96%, at least 97%, at least 98%, or at least 99% homology rs9513116 (SEQ ID NO. 63) having a substitution, deletion or addition of at least one nucleotide corresponding to position 51 of rs9513116 (SEQ ID NO. 63);

(nn) a sequence corresponding to rs9551473 (SEQ ID NO. 64) or having at least 80%, at least 85%, or more preferably at least 90%, or even more preferably, at least 95%, at least 96%, at least 97%, at least 98%, or at least 99% homology to rs9551473 (SEQ ID NO. 64) having a substitution, deletion or addition of at least one nucleotide corresponding to position 51 of rs9551473 (SEQ ID NO. 64);

(oo) a sequence corresponding to rs7330109 (SEQ ID NO. 65) or having at least 80%, at least 85%, or more preferably at least 90%, or even more preferably, at least 95%, at least 96%, at least 97%, at least 98%, or at least 99% homology to rs7330109 (SEQ ID NO. 65) having a substitution, deletion or addition of at least one nucleotide corresponding to position 51 of rs7330109 (SEQ ID NO. 65);

(pp) a sequence corresponding to rs9508037 (SEQ ID NO. 66) or having at least 80%, at least 85%, or more preferably at least 90%, or even more preferably, at least 95%, at least 96%, at least 97%, at least 98%, or at least 99% homology to rs9508037 (SEQ ID NO. 66) having a substitution, deletion or addition of at least one nucleotide corresponding to position 51 of rs9508037 (SEQ ID NO. 66);

(qq) a sequence corresponding to rs1924981 (SEQ ID NO. 67) or having at least 80%, at least 85%, or more preferably at least 90%, or even more preferably, at least 95%, at least 96%, at least 97%, at least 98%, or at least 99% homology to rs1924981 (SEQ ID NO. 67) having a substitution, deletion or addition of at least one nucleotide corresponding to position 51 of rs1924981 (SEQ ID NO. 67);
(rr) a sequence corresponding to rs34140996 (SEQ ID NO. 68) or having at least 80%, at least 85%, or more preferably at least 90%, or even more preferably, at least 95%, at least 96%, at least 97%, at least 98%, or at least 99% homology to rs3794400 rs34140996 (SEQ ID NO. 68) having a substitution, deletion or addition of at least one nucleotide corresponding to position 51 of rs34140996 (SEQ ID NO. 68);
(ss) a sequence corresponding to rs7985584 (SEQ ID NO. 69) or having at least 80%, at least 85%, or more preferably at least 90%, or even more preferably, at least 95%, at least 96%, at least 97%, at least 98%, or at least 99% homology to rs7985584 (SEQ ID NO. 69) having a substitution, deletion or addition of at least one nucleotide corresponding to position 51 of rs7985584 (SEQ ID NO. 69);
(tt) a sequence corresponding to rs7992940 (SEQ ID NO. 70) or having at least 80%, at least 85%, or more preferably at least 90%, or even more preferably, at least 95%, at least 96%, at least 97%, at least 98%, or at least 99% homology to rs7992940 (SEQ ID NO. 70) having a substitution, deletion or addition of at least one nucleotide corresponding to position 51 of rs7992940 (SEQ ID NO. 70); and
(uu) a sequence corresponding to rs718273 (SEQ ID NO. 71) or having at least 80%, at least 85%, or more preferably at least 90%, or even more preferably, at least 95%, at least 96%, at least 97%, at least 98%, or at least 99% homology to rs718273 (SEQ ID NO. 71) having a substitution, deletion or addition of at least one nucleotide corresponding to position 51 of rs718273 (SEQ ID NO. 71).

In the context of the herein described invention, the above described homology, with regard to the identified SNPs, applies to each embodiment herein described wherein specific SNPs are recited.

Further, the variations can include a substitution(s), deletion(s) or addition(s) at any position from position 1 to 50 and 52 to 101 of rs17619037 (SEQ ID NO. 5), rs9579177 (SEQ ID NO. 6), rs9579176 (SEQ ID NO. 7), rs9554319 (SEQ ID NO. 8), rs7996030 (SEQ ID NO. 9), rs9513075 (SEQ ID NO. 10), rs7993418 (SEQ ID NO. 11), rs9513071 (SEQ ID NO. 12), rs11620238 (SEQ ID NO. 13), rs17618631 (SEQ ID NO. 14), rs12429309 (SEQ ID NO. 15), rs7982251 (SEQ ID NO 16), rs9508016 (SEQ ID NO. 17), rs7982957 (SEQ ID NO. 18), rs3794400 (SEQ ID NO. 19), rs45455097 (SEQ ID NO. 40), rs1886233 (SEQ ID NO. 41), rs9554309 (SEQ ID NO. 42), rs11619230 (SEQ ID NO. 43), rs9554311 (SEQ ID NO. 44), rs4771233 (SEQ ID NO. 45), rs6491274 (SEQ ID NO. 46), rs7982639 (SEQ ID NO. 47), rs12877718 (SEQ ID NO. 48), rs10507382 (SEQ ID NO. 49), rs57354941 (SEQ ID NO. 50), rs17086497 (SEQ ID NO. 51), rs3794395 (SEQ ID NO. 52), rs9554317 (SEQ ID NO. 53), rs9513073 (SEQ ID NO. 54), rs9513074 (SEQ ID NO. 55), rs9508015 (SEQ ID NO. 56), rs2011950 (SEQ ID NO. 57), rs9513110 (SEQ ID NO. 58), rs9513112 (SEQ ID NO. 59), rs9513113 (SEQ ID NO. 60), rs9551471 (SEQ ID NO. 61), rs2296285 (SEQ ID NO. 62), rs9513116 (SEQ ID NO. 63), rs9551473 (SEQ ID NO. 64), rs7330109 (SEQ ID NO. 65), rs9508037 (SEQ ID NO. 66), rs1924981 (SEQ ID NO. 67), rs34140996 (SEQ ID NO. 68), rs7985584 (SEQ ID NO. 69), rs7992940 (SEQ ID NO. 70) and rs718273 (SEQ ID NO. 71).

Accordingly, in another embodiment of the present invention the one or more variant alleles of the VEGFR-1 gene include variations in the VEGFR-1 gene corresponding to linked SNPs as well as other linked SNPs upstream or downstream from VEGFR-1 gene corresponding to variations in the PAN3 gene and in intergenic DNA selected from the group consisting of:

(a) a sequence corresponding to rs17619037 (SEQ ID NO. 5) or having at least 80%, at least 85%, or more preferably at least 90%, or even more preferably, at least 95%, at least 96%, at least 97%, at least 98%, or at least 99% homology to rs17619037 (SEQ ID NO. 5), wherein position 51 is an A or G and having a substitution, deletion or addition of at least one nucleotide at any position from position 1 to 50 and 52 to 101;
(b) a sequence corresponding to rs9579177 (SEQ ID NO. 6) or having at least 80%, at least 85%, or more preferably at least 90%, or even more preferably, at least 95%, at least 96%, at least 97%, at least 98%, or at least 99% to rs9579177 (SEQ ID NO. 6), wherein position 51 is an A or G and having a substitution, deletion or addition of at least one nucleotide at any position from position 1 to 50 and 52 to 101;
(c) a sequence corresponding to rs9579176 (SEQ ID NO. 7) or having at least 80%, at least 85%, or more preferably at least 90%, or even more preferably, at least 95%, at least 96%, at least 97%, at least 98%, or at least 99% homology to rs9579176 (SEQ ID NO. 7), wherein position 51 is an A or G and having a substitution, deletion or addition of at least one nucleotide at any position from position 1 to 50 and 52 to 101;
(d) a sequence corresponding to rs9554319 (SEQ ID NO. 8) or having at least 80%, at least 85%, or more preferably at least 90%, or even more preferably, at least 95%, at least 96%, at least 97%, at least 98%, or at least 99% homology to rs9554319 (SEQ ID NO. 8), wherein position 51 is a C or T and having a substitution, deletion or addition of at least one nucleotide at any position from position 1 to 50 and 52 to 101;
(e) a sequence corresponding to rs7996030 (SEQ ID NO. 9) or having at least 80%, at least 85%, or more preferably at least 90%, or even more preferably, at least 95%, at least 96%, at least 97%, at least 98%, or at least 99% homology to rs7996030 (SEQ ID NO. 9), wherein position 51 is an A or G and having a substitution, deletion or addition of at least one nucleotide at any position from position 1 to 50 and 52 to 101;

(f) a sequence corresponding to rs9513075 (SEQ ID NO. 10) or having at least 80%, at least 85%, or more preferably at least 90%, or even more preferably, at least 95%, at least 96%, at least 97%, at least 98%, or at least 99% homology to rs9513075 (SEQ ID NO. 10), wherein position 51 is an A or G and having a substitution, deletion or addition of at least one nucleotide at any position from position 1 to 50 and 52 to 101;

(g) a sequence corresponding to rs7993418 (SEQ ID NO. 11) or having at least 80%, at least 85%, or more preferably at least 90%, or even more preferably, at least 95%, at least 96%, at least 97%, at least 98%, or at least 99% homology to rs7993418 (SEQ ID NO. 11), wherein position 51 is an A or G and having a substitution, deletion or addition of at least one nucleotide at any position from position 1 to 50 and 52 to 101;

(h) a sequence corresponding to rs9513071 (SEQ ID NO. 12) or having at least 80%, at least 85%, or more preferably at least 90%, or even more preferably, at least 95%, at least 96%, at least 97%, at least 98%, or at least 99% homology to rs9513071 (SEQ ID NO. 12), wherein position 51 is a T or G and having a substitution, deletion or addition of at least one nucleotide at any position from position 1 to 50 and 52 to 101;

(i) a sequence corresponding to rs11620238 (SEQ ID NO. 13) or having at least 80%, at least 85%, or more preferably at least 90%, or even more preferably, at least 95%, at least 96%, at least 97%, at least 98%, or at least 99% homology to rs11620238 (SEQ ID NO. 13), wherein position 51 is a T or G and having a substitution, deletion or addition of at least one nucleotide at any position from position 1 to 50 and 52 to 101;

(j) a sequence corresponding to rs17618631 (SEQ ID NO. 14) or having at least 80%, at least 85%, or more preferably at least 90%, or even more preferably, at least 95%, at least 96%, at least 97%, at least 98%, or at least 99% homology to rs17618631 (SEQ ID NO. 14), wherein position 51 is a C or G and having a substitution, deletion or addition of at least one nucleotide at any position from position 1 to 50 and 52 to 101;

(k) a sequence corresponding to rs12429309 (SEQ ID NO. 15) or having at least 80%, at least 85%, or more preferably at least 90%, or even more preferably, at least 95%, at least 96%, at least 97%, at least 98%, or at least 99% homology to rs12429309 (SEQ ID NO. 15), wherein position 51 is a T or C and having a substitution, deletion or addition of at least one nucleotide at any position from position 1 to 50 and 52 to 101;

(1) a sequence corresponding to rs7982251 (SEQ ID NO 16) or having at least 80%, at least 85%, or more preferably at least 90%, or even more preferably, at least 95%, at least 96%, at least 97%, at least 98%, or at least 99% homology to rs7982251 (SEQ ID NO 16), wherein position 51 is a T or C and having a substitution, deletion or addition of at least one nucleotide at any position from position 1 to 50 and 52 to 101;

(m) a sequence corresponding to rs9508016 (SEQ ID NO. 17) or having at least 80%, at least 85%, or more preferably at least 90%, or even more preferably, at least 95%, at least 96%, at least 97%, at least 98%, or at least 99% homology to rs9508016 (SEQ ID NO. 17), wherein position 51 is an A or G and having a substitution, deletion or addition of at least one nucleotide at any position from position 1 to 50 and 52 to 101;

(n) a sequence corresponding to rs7982957 (SEQ ID NO. 18) or having at least 80%, at least 85%, or more preferably at least 90%, or even more preferably, at least 95%, at least 96%, at least 97%, at least 98%, or at least 99% homology to rs7982957 (SEQ ID NO. 18), wherein position 51 is an A or T and having a substitution, deletion or addition of at least one nucleotide at any position from position 1 to 50 and 52 to 101;

(o) a sequence corresponding to rs3794400 (SEQ ID NO. 19) or having at least 80%, at least 85%, or more preferably at least 90%, or even more preferably, at least 95%, at least 96%, at least 97%, at least 98%, or at least 99% homology to rs3794400 (SEQ ID NO. 19), wherein position 51 is a T or C and having a substitution, deletion or addition of at least one nucleotide at any position from position 1 to 50 and 52 to 101;

(p) a sequence corresponding to rs45455097 (SEQ ID NO. 40) or having at least 80%, at least 85%, or more preferably at least 90%, or even more preferably, at least 95%, at least 96%, at least 97%, at least 98%, or at least 99% homology to rs45455097 (SEQ ID NO. 40), wherein position 51 is a T or C and having a substitution, deletion or addition of at least one nucleotide at any position from position 1 to 50 and 52 to 101;

(q) a sequence corresponding to rs1886233 (SEQ ID NO. 41) or having at least 80%, at least 85%, or more preferably at least 90%, or even more preferably, at least 95%, at least 96%, at least 97%, at least 98%, or at least 99% homology to rs1886233 (SEQ ID NO. 41), wherein position 51 is an A or G and having a substitution, deletion or addition of at least one nucleotide at any position from position 1 to 50 and 52 to 101;

(r) a sequence corresponding to rs9554309 (SEQ ID NO. 42) or having at least 80%, at least 85%, or more preferably at least 90%, or even more preferably, at least 95%, at least 96%, at least 97%, at least 98%, or at least 99% homology to rs9554309 (SEQ ID NO. 42), wherein position 51 is a T or C and having a substitution, deletion or addition of at least one nucleotide at any position from position 1 to 50 and 52 to 101;

(s) a sequence corresponding to rs11619230 (SEQ ID NO. 43) or having at least 80%, at least 85%, or more preferably at least 90%, or even more preferably, at least 95%, at least 96%, at least 97%, at least 98%, or at least 99% homology to rs11619230 (SEQ ID NO. 43), wherein position 51 is a T or C and having a substitution, deletion or addition of at least one nucleotide at any position from position 1 to 50 and 52 to 101;
(t) a sequence corresponding to rs9554311 (SEQ ID NO. 44) or having at least 80%, at least 85%, or more preferably at least 90%, or even more preferably, at least 95%, at least 96%, at least 97%, at least 98%, or at least 99% homology to rs9554311 (SEQ ID NO. 44), wherein position 51 is a T or C and having a substitution, deletion or addition of at least one nucleotide at any position from position 1 to 50 and 52 to 101;
(u) a sequence corresponding to rs4771233 (SEQ ID NO. 45) or having at least 80%, at least 85%, or more preferably at least 90%, or even more preferably, at least 95%, at least 96%, at least 97%, at least 98%, or at least 99% homology to rs4771233 (SEQ ID NO. 45), wherein position 51 is a T or C and having a substitution, deletion or addition of at least one nucleotide at any position from position 1 to 50 and 52 to 101;
(v) a sequence corresponding to rs6491274 (SEQ ID NO. 46) or having at least 80%, at least 85%, or more preferably at least 90%, or even more preferably, at least 95%, at least 96%, at least 97%, at least 98%, or at least 99% homology to rs6491274 (SEQ ID NO. 46), wherein position 51 is a T or C and having a substitution, deletion or addition of at least one nucleotide at any position from position 1 to 50 and 52 to 101;
(w) a sequence corresponding to rs7982639 (SEQ ID NO. 47) or having at least 80%, at least 85%, or more preferably at least 90%, or even more preferably, at least 95%, at least 96%, at least 97%, at least 98%, or at least 99% homology to rs7982639 (SEQ ID NO. 47), wherein position 51 is an A or G and having a substitution, deletion or addition of at least one nucleotide at any position from position 1 to 50 and 52 to 101;
(x) a sequence corresponding to rs12877718 (SEQ ID NO. 48) or having at least 80%, at least 85%, or more preferably at least 90%, or even more preferably, at least 95%, at least 96%, at least 97%, at least 98%, or at least 99% homology to rs12877718 (SEQ ID NO. 48), wherein position 51 is a T or C and having a substitution, deletion or addition of at least one nucleotide at any position from position 1 to 50 and 52 to 101;
(y) a sequence corresponding to rs10507382 (SEQ ID NO. 49) or having at least 80%, at least 85%, or more preferably at least 90%, or even more preferably, at least 95%, at least 96%, at least 97%, at least 98%, or at least 99% homology to rs10507382 (SEQ ID NO. 49), wherein position 51 is a T or C and having a substitution, deletion or addition of at least one nucleotide at any position from position 1 to 50 and 52 to 101;
(z) a sequence corresponding to rs57354941 (SEQ ID NO. 50) or having at least 80%, at least 85%, or more preferably at least 90%, or even more preferably, at least 95%, at least 96%, at least 97%, at least 98%, or at least 99% homology to rs57354941 (SEQ ID NO. 50), wherein position 51 is a T or C and having a substitution, deletion or addition of at least one nucleotide at any position from position 1 to 50 and 52 to 101;
(aa) a sequence corresponding to rs17086497 (SEQ ID NO. 51) or having at least 80%, at least 85%, or more preferably at least 90%, or even more preferably, at least 95%, at least 96%, at least 97%, at least 98%, or at least 99% homology to rs17086497 (SEQ ID NO. 51), wherein position 51 is a C or A and having a substitution, deletion or addition of at least one nucleotide at any position from position 1 to 50 and 52 to 101;
(bb) a sequence corresponding to rs3794395 (SEQ ID NO. 52) or having at least 80%, at least 85%, or more preferably at least 90%, or even more preferably, at least 95%, at least 96%, at least 97%, at least 98%, or at least 99% homology to rs3794395 (SEQ ID NO. 52), wherein position 51 is a T or C and having a substitution, deletion or addition of at least one nucleotide at any position from position 1 to 50 and 52 to 101;
(cc) a sequence corresponding to rs9554317 (SEQ ID NO. 53) or having at least 80%, at least 85%, or more preferably at least 90%, or even more preferably, at least 95%, at least 96%, at least 97%, at least 98%, or at least 99% homology to rs9554317 (SEQ ID NO. 53), wherein position 51 is a T or C and having a substitution, deletion or addition of at least one nucleotide at any position from position 1 to 50 and 52 to 101;
(dd) a sequence corresponding to rs9513073 (SEQ ID NO. 54) or having at least 80%, at least 85%, or more preferably at least 90%, or even more preferably, at least 95%, at least 96%, at least 97%, at least 98%, or at least 99% homology to rs9513073 (SEQ ID NO. 54), wherein position 51 is an A or G and having a substitution, deletion or addition of at least one nucleotide at any position from position 1 to 50 and 52 to 101;
(ee) a sequence corresponding to rs9513074 (SEQ ID NO. 55) or having at least 80%, at least 85%, or more preferably at least 90%, or even more preferably, at least 95%, at least 96%, at least 97%, at least 98%, or at least 99% homology to rs9513074 (SEQ ID NO. 55), wherein position 51 is a T or C and having a substitution, deletion or addition of at least one nucleotide at any position from position 1 to 50 and 52 to 101;
(ff) a sequence corresponding to rs9508015 (SEQ ID NO. 56) or having at least 80%, at least 85%, or more preferably at least 90%, or even more preferably, at least 95%, at least 96%, at least 97%, at least 98%, or at least 99% homology to rs9508015 (SEQ ID NO. 56), wherein position 51 is a T or C and having a substitution, deletion or addition of at least one nucleotide at any position from position 1 to 50 and 52 to 101;
(gg) a sequence corresponding to rs2011950 (SEQ ID NO. 57) or having at least 80%, at least 85%, or more preferably at least 90%, or even more preferably, at least 95%, at least 96%, at least 97%, at least 98%, or at least 99% homology to rs2011950 (SEQ ID NO. 57), wherein position 51 is a G or A and having a substitution, deletion or addition of at least one nucleotide at any position from position 1 to 50 and 52 to 101;
(hh) a sequence corresponding to rs9513110 (SEQ ID NO. 58) or having at least 80%, at least 85%, or more preferably at least 90%, or even more preferably, at least 95%, at least 96%, at least 97%, at least 98%, or at least 99% homology to rs9513110 (SEQ ID NO. 58), wherein position 51 is a T or G and having a substitution, deletion or addition of at least one nucleotide at any position from position 1 to 50 and 52 to 101;
(ii) a sequence corresponding to rs9513112 (SEQ ID NO. 59) or having at least 80%, at least 85%, or more preferably at least 90%, or even more preferably, at least 95%, at least 96%, at least 97%, at least 98%, or at least 99% homology to rs9513112 (SEQ ID NO. 59), wherein position 51 is a G or A and having a substitution, deletion or addition of at least one nucleotide at any position from position 1 to 50 and 52 to 101;
(jj) a sequence corresponding to rs9513113 (SEQ ID NO. 60) or having at least 80%, at least 85%, or more preferably at least 90%, or even more preferably, at least 95%, at least 96%, at least 97%, at least 98%, or at least 99% homology to rs9513113 (SEQ ID NO. 60), wherein position 51 is a T or C and having a substitution, deletion or addition of at least one nucleotide at any position from position 1 to 50 and 52 to 101;
(kk) a sequence corresponding to rs9551471 (SEQ ID NO. 61) or having at least 80%, at least 85%, or more preferably at least 90%, or even more preferably, at least 95%, at least 96%, at least 97%, at least 98%, or at least 99% homology to rs9551471 (SEQ ID NO. 61), wherein position 51 is a G or A and having a substitution, deletion or addition of at least one nucleotide at any position from position 1 to 50 and 52 to 101;
(11) a sequence corresponding to rs2296285 (SEQ ID NO. 62) or having at least 80%, at least 85%, or more preferably at least 90%, or even more preferably, at least 95%, at least 96%, at least 97%, at least 98%, or at least 99% homology to rs2296285 (SEQ ID NO. 62), wherein position 51 is a T or A and having a substitution, deletion or addition of at least one nucleotide at any position from position 1 to 50 and 52 to 101;
(mm) a sequence corresponding to rs9513116 (SEQ ID NO. 63) or having at least 80%, at least 85%, or more preferably at least 90%, or even more preferably, at least 95%, at least 96%, at least 97%, at least 98%, or at least 99% homology rs9513116 (SEQ ID NO. 63), wherein position 51 is a G or A and having a substitution, deletion or addition of at least one nucleotide at any position from position 1 to 50 and 52 to 101;
(nn) a sequence corresponding to rs9551473 (SEQ ID NO. 64) or having at least 80%, at least 85%, or more preferably at least 90%, or even more preferably, at least 95%, at least 96%, at least 97%, at least 98%, or at least 99% homology to rs9551473 (SEQ ID NO. 64), wherein position 51 is a T or G and having a substitution, deletion or addition of at least one nucleotide at any position from position 1 to 50 and 52 to 101;
(oo) a sequence corresponding to rs7330109 (SEQ ID NO. 65) or having at least 80%, at least 85%, or more preferably at least 90%, or even more preferably, at least 95%, at least 96%, at least 97%, at least 98%, or at least 99% homology to rs7330109 (SEQ ID NO. 65), wherein position 51 is a T or A and having a substitution, deletion or addition of at least one nucleotide at any position from position 1 to 50 and 52 to 101;
(pp) a sequence corresponding to rs9508037 (SEQ ID NO. 66) or having at least 80%, at least 85%, or more preferably at least 90%, or even more preferably, at least 95%, at least 96%, at least 97%, at least 98%, or at least 99% homology to rs9508037 (SEQ ID NO. 66), wherein position 51 is a G or A and having a substitution, deletion or addition of at least one nucleotide at any position from position 1 to 50 and 52 to 101 having a substitution, deletion or addition of at least one nucleotide corresponding to position 51 of rs9508037 (SEQ ID NO. 66);
(qq) a sequence corresponding to rs1924981 (SEQ ID NO. 67) or having at least 80%, at least 85%, or more preferably at least 90%, or even more preferably, at least 95%, at least 96%, at least 97%, at least 98%, or at least 99% homology to rs1924981 (SEQ ID NO. 67), wherein position 51 is a T or C and having a substitution, deletion or addition of at least one nucleotide at any position from position 1 to 50 and 52 to 101;
(rr) a sequence corresponding to rs34140996 (SEQ ID NO. 68) or having at least 80%, at least 85%, or more preferably at least 90%, or even more preferably, at least 95%, at least 96%, at least 97%, at least 98%, or at least 99% homology to rs34140996 (SEQ ID NO. 68), wherein position 51 is a G or A and having a substitution, deletion or addition of at least one nucleotide at any position from position 1 to 50 and 52 to 101;
(ss) a sequence corresponding to rs7985584 (SEQ ID NO. 69) or having at least 80%, at least 85%, or more preferably at least 90%, or even more preferably, at least 95%, at least 96%, at least 97%, at least 98%, or at least 99% homology to rs7985584 (SEQ ID NO. 69), wherein position 51 is a G or A and having a substitution, deletion or addition of at least one nucleotide at any position from position 1 to 50 and 52 to 101;
(tt) a sequence corresponding to rs7992940 (SEQ ID NO. 70) or having at least 80%, at least 85%, or more preferably at least 90%, or even more preferably, at least 95%, at least 96%, at least 97%, at least 98%, or at least 99% homology to rs7992940 (SEQ ID NO. 70), wherein position 51 is a T or G and having a substitution, deletion or addition of at least one nucleotide at any position from position 1 to 50 and 52 to 101; and
(uu) a sequence corresponding to rs718273 (SEQ ID NO. 71) or having at least 80%, at least 85%, or more preferably at least 90%, or even more preferably, at least 95%, at least 96%, at least 97%, at least 98%, or at least 99% homology to rs718273 (SEQ ID NO. 71), wherein position 51 is a T or C and having a substitution, deletion or addition of at least one nucleotide at any position from position 1 to 50 and 52 to 101.

In the context of the herein described invention, the above described homology, with regard to the identified SNPs, applies to each embodiment herein described wherein specific SNPs are recited.

In the context of the present invention, "homology" is understood to refer in the context of "variant alleles" to a sequence identity of at least 80%, particularly an identity of at least 85%, preferably at least 90% and still more preferably at least 95% over the full length of the sequence as defined by the SEQ ID NOs provided herein. In the context of this invention, a skilled person would understand that homology covers further variation(s) in the "variant alleles" in different ethnic groups.

As documented in the appended examples, it could surprisingly be shown that the biomarkers as provided herein and/or the assessment of the individual genetic setting of a given subject in the VEGF-R1 gene are not only associated with overall survival but also risk of progression in patients treated with angiogenesis inhibitors, like bevacizumab.

In accordance with the methods of the present invention, four SNPs in the VEGFR-1 gene correlated with overall survival in the bevacizumab-treated group in a study treating metastatic pancreatic cancer with gemcitabine-erlotinib plus bevacizumab or placebo. The four SNPs identified are derived for SNPs rs9582036 (SEQ ID NO. 2), rs9554316 (SEQ ID NO. 1), rs9513070 (SEQ ID NO. 3) and rs9554320 (SEQ ID NO.4). The most significant SNP, rs9582036 (SEQ ID NO. 2), sequence shown in Figure 1, had p-value<=3e-4 in an allelic risk effect model. Relative to AA carriers, the hazard ratio was 2.0 (CI=1.2-3.4; p=0.009) and 4.7 (CI=2.1-10.7; p=0.0002) for AC and CC carriers, respectively. Median overall survival was increased from 4.8 months in CC (n=9) carriers to 6.0 and 10.3 months in AC (n=28) and AA (n=40) carriers. More specifically this analysis indicates that for rs9582036 (SEQ ID NO. 2), patients with AA genotype have longer survival than patient with AC genotypes, and patients with AC genotypes have longer survival than patients with CC genotype. A similar association was observed with the progression free survival end-point. After adjustment for baseline prognostic factors (neutrophil counts, CRP and tumor location) the effect was attenuated but not suppressed. No effect was detected in the control group. A test for interaction between rs9582036 (SEQ ID NO. 2) genotype and treatment resulted in a p-value of 0.02. Likewise, the hazard ratio for TG and GG carriers in the rs9554316 (SEQ ID NO. 1) SNP was 2.07 (CI=1.25-3.43; p=0.0047) and 4.69 (CI=1.75-12.95; p=0.0021). Specifically this analysis indicates that for rs9554316 (SEQ ID NO. 1), patients with GG genotype have longer survival than patient with TG genotypes, and patients with TG genotypes have longer survival than patients with TT genotype. Intriguingly, two other SNPs in the VEGFR-1 gene, i.e. rs9513070 (SEQ ID NO. 3) and rs9554320 (SEQ ID NO. 4), also correlated with improved median overall survival. All 4 SNPs were located in the same chromosomal region encompassing exons 25 to 30 of the VEGFR-1 gene and coding for the essential receptor tyrosine-kinase (TK) domain.

In accordance with the methods of the present invention, two SNPs in the VEGFR-1 gene correlated with progression-free survival in the bevacizumab-treated group in a study treating renal cell cancer with interferon alpha plus bevacizumab or interferon alpha plus placebo. The two SNPs identified are derived for SNPs rs9554316 (SEQ ID NO. 1) and rs9513070 (SEQ ID NO. 3). AA carriers of rs9513070 (SEQ ID NO: 3) showed increased progression free survival in comparison to AG and GG carriers in the bevacizumab-treated group (Figure 13). GG carriers of rs9554316 (SEQ ID NO:1) showed increased progression free survival in comparison to GT and TT carriers in the bevacizumab-treated group (Figure 15).

Accordingly, the present invention provides an angiogenesis inhibitor for use in an improved chemotherapy regimen for a patient suffering from a malignant disease or a disease involving physiological and pathological angiogenesis wherein it is determined from a patient sample if the patient has one or more variant alleles of the VEGFR-1 gene and whereby the angiogenesis inhibitor is to be administered to a patient having one or more variant alleles of the VEGFR-1 gene. The angiogenesis inhibitor to be administered may be administered with other chemotherapeutic agents or chemotherapy regimens. The malignant disease or a disease involving physiological and pathological angiogenesis may be pancreatic cancer or renal cell cancer. The angiogenesis inhibitor may be bevacizumab.

The present invention relates to bevacizumab for use in an improved chemotherapy regimen for a patient suffering from pancreatic cancer wherein the it is determined from a patient sample if the patient has one or more variant alleles of the VEGFR-1 gene and whereby bevacizumab is to be administered to a patient having one or more variant alleles of the VEGFR-1 gene. The bevacizumab to be administered may be administered with other chemotherapeutic agents or chemotherapy regimens, such as gemcitabine-erlotinib therapy. The present invention relates to bevacizumab for use in an improved chemotherapy regimen for a patient suffering from renal cell cancer wherein the it is determined from a patient sample if the patient has one or more variant alleles of the VEGFR-1 gene and whereby bevacizumab is to be administered to a patient having one or more variant alleles of the VEGFR-1 gene. The bevacizumab to be administered may be administered with other chemotherapeutic agents or chemotherapy regimens, such as interferon alpha therapy.

The following similar uses may be applied mutatis mutandis.

The present invention provides the use of an angiogenesis inhibitor for improving overall survival of a patient suffering from a malignant disease or a disease involving physiological and pathological angiogenesis comprising the following steps:

(a) determining from a patient sample if the patient suffering from a malignant disease or a disease involving physiological and pathological angiogenesis has one or more variant alleles of the VEGFR-1 gene; and
(b) administering the angiogenesis inhibitor to the patient having one or more variant alleles of the VEGFR-1 gene.

The malignant disease or a disease involving physiological and pathological angiogenesis may be pancreatic cancer. The angiogenesis inhibitor may be bevacizumab.

Accordingly, the present invention relates to the use of an angiogenesis inhibitor for improving overall survival of a patient suffering from a malignant disease or a disease involving physiological and pathological angiogenesis comprising the following steps:

(a) obtaining a sample from a patient suffering from a malignant disease or a disease involving physiological and pathological angiogenesis;
(b) determining if the patient has one or more variant alleles of the VEGFR-1 gene; and
(c) administering the angiogenesis inhibitor to the patient having one or more variant alleles of the VEGFR-1 gene.

The malignant disease or a disease involving physiological and pathological angiogenesis may be pancreatic cancer. The angiogenesis inhibitor may be bevacizumab.

The present invention relates to an angiogenesis inhibitor for use in improving overall survival of a patient suffering from a malignant disease or a disease involving physiological and pathological angiogenesis wherein it is determined from a patient sample if the patient suffering from a malignant disease or a disease involving physiological and pathological angiogenesis has one or more variant alleles of the VEGFR-1 gene and whereby the angiogenesis inhibitor is to be administered to the patient having one or more variant alleles of the VEGFR-1 gene. The malignant disease or a disease involving physiological and pathological angiogenesis may be pancreatic cancer. The angiogenesis inhibitor may be bevacizumab.

The present invention provides the use of an angiogenesis inhibitor for improving progression-free survival of a patient suffering from a malignant disease or a disease involving physiological and pathological angiogenesis comprising the following steps:

(a) determining from a patient sample if the patient suffering from a malignant disease or a disease involving physiological and pathological angiogenesis has one or more variant alleles of the VEGFR-1 gene; and
(b) administering the angiogenesis inhibitor to the patient having one or more variant alleles of the VEGFR-1 gene.

The malignant disease or a disease involving physiological and pathological angiogenesis may be pancreatic cancer or renal cell cancer. The angiogenesis inhibitor may be bevacizumab.

Accordingly, the present invention relates to the use of an angiogenesis inhibitor for improving progression-free survival of a patient suffering from a malignant disease or a disease involving physiological and pathological angiogenesis comprising the following steps:

(a) obtaining a sample from a patient suffering from a malignant disease or a disease involving physiological and pathological angiogenesis;
(b) determining if the patient has one or more variant alleles of the VEGFR-1 gene; and
(c) administering the angiogenesis inhibitor to the patient having one or more variant alleles of the VEGFR-1 gene.

The malignant disease or a disease involving physiological and pathological angiogenesis may be pancreatic cancer or renal cell cancer. The angiogenesis inhibitor may be bevacizumab.

The present invention relates to an angiogenesis inhibitor for use in improving progression-free survival of a patient suffering from a malignant disease or a disease involving physiological and pathological angiogenesis wherein it is determined from a patient sample if the patient suffering from a malignant disease or a disease involving physiological and pathological angiogenesis has one or more variant alleles of the VEGFR-1 gene and whereby the angiogenesis inhibitor is to be administered to said patient having one or more variant alleles of the VEGFR-1 gene. The malignant disease or a disease involving physiological and pathological angiogenesis may be pancreatic cancer or renal cell cancer. The angiogenesis inhibitor may be bevacizumab.

Accordingly, the invention provides the use of an angiogenesis inhibitor for improving overall survival of a patient suffering from a metastatic pancreatic cancer comprising the following steps:

(a) determining from a patient sample if the patient suffering from metastatic pancreatic cancer has one or more variant alleles of the VEGFR-1 gene wherein the one or more variant alleles are selected from the group consisting of SNPs rs9554316 (SEQ ID NO. 1), rs9582036 (SEQ ID NO. 2), rs9513070 (SEQ ID NO. 3) and rs9554320 (SEQ ID NO.4); and
(b) administering the angiogenesis inhibitor to the patient having one or more variant alleles of the VEGFR-1 gene.

The angiogenesis inhibitor may be bevacizumab. The angiogenesis inhibitor may be administered with other chemotherapeutic agents or chemotherapy regimens, such as gemcitabine-erlotinib.

The invention relates to the use of an angiogenesis inhibitor for improving overall survival of a patient suffering from a metastatic pancreatic cancer comprising the following steps:

(a) obtaining a sample from a patient suffering from metastatic pancreatic cancer;
(b) determining from a patient sample if the patient has one or more variant alleles of the VEGFR-1 gene wherein the one or more variant alleles are selected from the group consisting of SNPs rs9554316 (SEQ ID NO. 1), rs9582036 (SEQ ID NO. 2), rs9513070 (SEQ ID NO. 3) and rs9554320 (SEQ ID NO.4); and
(c) administering the angiogenesis inhibitor to the patient having one or more variant alleles of the VEGFR-1 gene.

The angiogenesis inhibitor may be bevacizumab. The angiogenesis inhibitor may be administered with other chemotherapeutic agents or chemotherapy regimens, such as gemcitabine-erlotinib.

The present invention, therefore, relates to an angiogenesis inhibitor for use in improving overall survival of a patient suffering from metastatic pancreatic cancer wherein it is determined from a patient sample if the patient suffering from metastatic pancreatic cancer has one or more variant alleles of the VEGFR-1 gene and whereby the angiogenesis inhibitor it to be administered to the patient having one or more variant alleles of the VEGFR-1 gene. The angiogenesis inhibitor may be bevacizumab. The angiogenesis inhibitor may be administered with other chemotherapeutic agents or chemotherapy regimens, such as gemcitabine-erlotinib therapy.

Accordingly, the invention provides the use of an angiogenesis inhibitor for improving progression-free survival of a patient suffering from a metastatic pancreatic cancer comprising the following steps:

(a) determining from a patient sample if the patient suffering from metastatic pancreatic cancer has one or more variant alleles of the VEGFR-1 gene wherein the one or more variant alleles are selected from the group consisting of SNPs rs9554316 (SEQ ID NO. 1), rs9582036 (SEQ ID NO. 2), rs9513070 (SEQ ID NO. 3) and rs9554320 (SEQ ID NO.4); and
(b) administering the angiogenesis inhibitor to the patient having one or more variant alleles of the VEGFR-1 gene.

The angiogenesis inhibitor may be bevacizumab. The angiogenesis inhibitor may be administered with other chemotherapeutic agents or chemotherapy regimens, such as gemcitabine-erlotinib therapy.

Accordingly, the invention relates to the use of an angiogenesis inhibitor for improving progression-free survival of a patient suffering from a metastatic pancreatic cancer comprising the following steps:

(a) obtaining a sample from a patient suffering from metastatic pancreatic cancer;
(b) determining from a patient sample if the patient has one or more variant alleles of the VEGFR-1 gene wherein the one or more variant alleles are selected from the group consisting of SNPs rs9554316 (SEQ ID NO. 1), rs9582036 (SEQ ID NO. 2), rs9513070 (SEQ ID NO. 3) and rs9554320 (SEQ ID NO.4); and
(c) administering the angiogenesis inhibitor to the patient having one or more variant alleles of the VEGFR-1 gene.

The angiogenesis inhibitor may be bevacizumab. The angiogenesis inhibitor may be administered with other chemotherapeutic agents or chemotherapy regimens, such as gemcitabine-erlotinib therapy.

The present invention, therefore, relates to an angiogenesis inhibitor for use in improving progression-free survival of a patient suffering from metastatic pancreatic cancer wherein it is determined from a patient sample if the patient suffering from metastatic pancreatic cancer has one or more variant alleles of the VEGFR-1 gene and whereby the angiogenesis inhibitor is to be administered to the patient having one or more variant alleles of the VEGFR-1 gene. The angiogenesis inhibitor may be bevacizumab. The angiogenesis inhibitor may be administered with other chemotherapeutic agents or chemotherapy regimens, such as gemcitabine-erlotinib therapy.

Accordingly, the invention provides the use of an angiogenesis inhibitor for improving progression-free survival of a patient suffering from a renal cell cancer comprising the following steps:

(a) determining from a patient sample if the patient suffering from renal cell cancer has one or more variant alleles of the VEGFR-1 gene wherein the one or more variant alleles are selected from the group consisting of rs9554316 (SEQ ID NO. 1) and rs9513070 (SEQ ID NO. 3); and
(b) administering the angiogenesis inhibitor to the patient having one or more variant alleles of the VEGFR-1 gene.

The angiogenesis inhibitor may be bevacizumab. The angiogenesis inhibitor may be administered with other chemotherapeutic agents or chemotherapy regimens, such as interferon alpha therapy.

Accordingly, the invention relates to the use of an angiogenesis inhibitor for improving progression-free survival of a patient suffering from a renal cell cancer comprising the following steps:

(a) obtaining a sample from a patient suffering from renal cell cancer;
(b) determining if the patient has one or more variant alleles of the VEGFR-1 gene wherein the one or more variant alleles are selected from the group consisting of rs9554316 (SEQ ID NO. 1) and rs9513070 (SEQ ID NO. 3); and
(c) administering the angiogenesis inhibitor to the patient having one or more variant alleles of the VEGFR-1 gene.

The angiogenesis inhibitor may be bevacizumab. The angiogenesis inhibitor may be administered with other chemotherapeutic agents or chemotherapy regimens, such as interferon alpha therapy.

The present invention, therefore, relates to an angiogenesis inhibitor for use in improving progression-free survival of a patient suffering from renal cell cancer wherein it is determined from a patient sample if the patient suffering from renal cell cancer has one or more variant alleles of the VEGFR-1 gene and whereby the angiogenesis inhibitor is to be administered to the patient having one or more variant alleles of the VEGFR-1 gene is administered an angiogenesis inhibitor. The angiogenesis inhibitor may be bevacizumab. The angiogenesis inhibitor may be administered with other chemotherapeutic agents or chemotherapy regimens, such as interferon alpha therapy.

In accordance with the methods of the present invention, the herein identified SNPs are predictive of patients suffering from a malignant disease or a disease involving physiological and pathological angiogenesis, in particular metastatic pancreatic cancer or renal cell cancer, that is responsive or sensitive to treatment with an angiogenesis inhibitor, in particular bevacizumab. Accordingly, the present invention relates to a method of predicting the response to or sensitivity to an angiogenesis inhibitor of a patient suffering from a malignant disease or a disease involving physiological and pathological angiogenesis comprising determining from a patient sample if a patient suffering from a malignant disease or a disease involving physiological and pathological angiogenesis has one or more variant alleles of the VEGFR-1 gene. The malignant disease or a disease involving physiological and pathological angiogenesis may be pancreatic cancer or renal cell cancer. The angiogenesis inhibitor may be bevacizumab.

The present invention provides a method of predicting the response to or sensitivity to angiogenesis inhibitor therapy of a patient suffering from metastatic pancreatic cancer comprising determining from a patient sample if a patient suffering from metastatic pancreatic cancer has one or more variant alleles of the VEGFR-1 gene. The one or more variant alleles of the VEGFR-1 gene may be selected from the group consisting of SNPs rs9554316 (SEQ ID NO. 1), rs9582036 (SEQ ID NO. 2), rs9513070 (SEQ ID NO. 3) and rs9554320 (SEQ ID NO.4), whereby a G at position 51 of rs9554316 (SEQ ID NO. 1), an A at position 51 of rs9582036 (SEQ ID NO. 2), an A at position 51 of rs9513070 (SEQ ID NO. 3) and/or a C at position 51 of rs9554320 (SEQ ID NO.4) is indicative of a patient response or sensitive to angiogenesis inhibitor therapy. The angiogenesis inhibitor may be bevacizumab. The invention, therefore, relates to the use of specific primers and/or probes for the preparation of a composition for predicting the response to or sensitivity to angiogenesis inhibitor therapy of a patient suffering from metastatic pancreatic cancer wherein the primers and/or probes are capable of detecting at least one of the variant alleles selected from the group consisting of SNPs rs9554316 (SEQ ID NO. 1), rs9582036 (SEQ ID NO. 2), rs9513070 (SEQ ID NO. 3) and rs9554320 (SEQ ID NO.4), whereby a G at position 51 of rs9554316 (SEQ ID NO. 1), an A at position 51 of SNPs rs9582036 (SEQ ID NO. 2), an A at position 51 of rs9513070 (SEQ ID NO. 3) and/or a C at position 51 of rs9554320 (SEQ ID NO.4) is indicative of a patient response or sensitive to angiogenesis inhibitor therapy. The angiogenesis inhibitor may be bevacizumab.

The present invention also provides a method of predicting the response to or sensitivity to angiogenesis inhibitor therapy of a patient suffering from renal cell cancer comprising determining from a patient sample if the patient suffering from renal cell cancer has one or more variant alleles of the VEGFR-1 gene from a patient sample wherein the one or more variant alleles are selected from the group consisting of SNPs rs9554316 (SEQ ID NO. 1) and rs9513070 (SEQ ID NO. 3) whereby a G at position 51 of rs9554316 (SEQ ID NO. 1) and/or an A at position 51 of rs9513070 (SEQ ID NO. 3) is indicative of a patient response or sensitive to angiogenesis inhibitor therapy. The angiogenesis inhibitor may be bevacizumab. The invention, therefore, relates to the use of specific primers and/or probes for the preparation of a composition for predicting the response to or sensitivity to bevacizumab therapy of a patient suffering from renal cell cancer wherein the primers and/or probes are capable of detecting at least one of the variant alleles are selected from the group consisting of at least one of the variant alleles selected from the group consisting of SNPs rs9554316 (SEQ ID NO. 1) and rs9513070 (SEQ ID NO. 3) whereby a G at position 51 of rs9554316 (SEQ ID NO. 1) and/or an A at position 51 of rs9513070 (SEQ ID NO. 3) is indicative of a patient response or sensitive to angiogenesis inhibitor therapy. The angiogenesis inhibitor may be bevacizumab.

The sample to be used in the methods provided herein is a biological sample and may be a blood and/or tissue sample. Preferably, the sample is a blood sample and more preferably a peripheral blood sample. Even more preferably, the sample is a DNA sample. The DNA sample may be germline DNA or somatic DNA. Preferably, the DNA is germline DNA.

The sample is preferably obtained from a patient suffering from a malignant disease or a disease involving physiological and pathological angiogenesis. Examples of a malignant disease include, but are not limited to, pancreatic cancer, renal cell cancer, lung cancer, breast cancer, colorectal cancer, prostate cancer, lymphoma, bone cancer, ovarian cancer, melanoma, prostate haematological malignancy and/or metastatic forms of the above identified cancers. Preferably the sample is obtained from a patient suffering from pancreatic cancer, renal cell cancer, lung cancer, breast cancer and/or colorectal cancer. Even more preferably the sample is obtained from a patient suffering from metastatic pancreatic cancer, renal cell cancer, metastatic colorectal cancer, metastatic breast cancer and/or non-squamous non-small cell lung cancer. Most preferably, the patient is suffering from metastatic pancreatic cancer or renal cell cancer. Examples of disease involving physiological and pathological angiogenesis include, but are not limited to, high grade glioma, glioblastoma, M. Rendu-Osler, von-Hippel-Lindau diseases, hemangiomas, psoriasis, Kaposi's sarcoma, ocular neovascularisation, rheumatoid arthritis, endometriosis, atherosclerosis, myochardial ischemia, peripheral ischemia, cerebral ischemia and wound healing.

The terms "angiogenesis inhibitor" in the context of the present invention refers to all agents that alter angiogenesis (e.g. the process of forming blood vessels) and includes agents that inhibit the angiogenesis, including, but not limited to, tumor angiogenesis. In this context, inhibition can refer to blocking the formation of blood vessels and halting or slowing down the growth of blood vessels. Examples of angiogenesis inhibitors include bevacizumab (also known as Avastin®), pegaptanib, sunitinib, sorafenib and vatalanib. A preferred angiogenesis inhibitors is bevacizumab (Avastin®), which is a recombinant humanized monoclonal IgG1 antibody that binds to and inhibits the biological activity of human VEGFA in *in vitro* and *in vivo* assay system. The term "bevacizumab" encompass all corresponding anti-VEGF antibodies that fulfill the requirements necessary for obtaining a marketing authorization as an identical or biosimilar product in a country or territory selected from the group of countries consisting of the USA, Europe and Japan.

In the context of the present invention, "administration" or "administering" means the administration of a pharmaceutical composition, such as an angiogenesis inhibitor, to the patient. For example, 2.5 mg/kg of body weight to 15 mg/kg of body weight bevacizumab (Avastin®) can be administered every week, every 2 weeks or every 3 weeks, depending on the type of cancer being treated. Preferred dosages include 5 mg/kg, 7.5 mg/kg, 10 mg/kg and 15 mg/kg. Even more preferred dosages are 5 mg/kg every 2 weeks, 10 mg/kg every 2 weeks and 15 mg/kg every 3 weeks. With respect to bevacizumab (Avastin®) for the treatment of metastatic pancreatic cancer, dosages include 5 mg/kg or 10 mg/kg of body weight given once every 2 weeks, or 7.5 mg/kg or 15 mg/kg of body weight given once every 3 weeks. For the treatment of renal cell cancer, dosages of bevacizumab (Avastin®) include 10 mg/kg of body weight given once every 2 weeks.

Dosages of with bevacizumab (Avastin®) for treatments of specific cancers, according to the EMEA, are as follows (for details see http://www.emea.europa.eu/humandocs/PDFs/EPAR/avastin/emea-combined-h582en.pdf). For metastatic carcinoma of the colon or rectum (mCRC) preferred dosages are 5 mg/kg or 10 mg/kg of body weight given once every 2 weeks or 7.5 mg/kg or 15 mg/kg of body weight given once every 3 weeks, for metastatic breast cancer (mBC) preferred dosages are 10 mg/kg of body weight given once every 2 weeks or 15 mg/kg of body weight given once every 3 weeks as an intravenous infusion, and for non-small cell lung cancer (NSCLC) preferred dosages are 7.5 mg/kg or 15 mg/kg of body weight given once every 3 weeks as an intravenous infusion. Clinical benefit in NSCLC patients has been demonstrated with both 7.5 mg/kg and 15 mg/kg doses. For details refer to section 5.1 Pharmacodynamic Properties, Non-small cell lung cancer (NSCLC). For advanced and/or metastatic Renal Cell Cancer (mRCC) preferred dosages are 10 mg/kg of body weight given once every 2 weeks as an intravenous infusion(in addition to platinum-based chemotherapy for up to 6 cycles of treatment followed by bevacizumab (Avastin®) as a single agent until disease progression). For gliablastoma a preferred dosage is 10 mg/kg every 2 weeks.

In the context of the present invention, the angiogenesis inhibitor may be administered in addition to or as a co-therapy or a co-treatment with one or more chemotherapeutic agents administered as part of standard chemotherapy regimen as known in the art. Examples of agents included in such standard chemotherapy regimens include 5-fluorouracil, leucovorin, irinotecan, gemcitabine, erlotinib, capecitabine, taxanes, such as docetaxel and paclitaxel, interferon alpha, vinorelbine, and platinum-based chemotherapeutic agents, such as paclitaxel, carboplatin, cisplatin and oxaliplatin. Examples of co-treatments for metastatic pancreatic cancer include gemcitabine-erlotinib plus bevacizumab at a dosage of 5mg/kg or 10 mg/kg of body weight given once every two weeks or 7.5 mg/kg or 15 mg/kg of body weight given once every three weeks. Examples of co-treatments for renal cell cancer include interferon alpha plus bevacizumab at a dosage of or 10 mg/kg of body weight given once every two weeks. Further, a patient may be co-treated with a combination of irinotecan, 5-fluorouracil, leucovorin, also referred to as IFL, as, for example, a bolus-IFL, with a combination of oxaliplatin, leucovorin, and 5-fluorouracil, also referred to a FOLFOX4 regimen, or with a combination of capecitabine and oxaliplatin, also referred to as XELOX. Accordingly, in a further embodiment of the invention, the patient suffering from a malignant disease or a disease involving physiological and pathological angiogenesis is being treated with one or more chemotherapeutic agents such as 5-fluorouracil, leucovorin, irinotecan, gemcitabine-erlotinib, capecitabine and/or platinum-based chemotherapeutic agents, such as paclitaxel, carboplatin and oxaliplatin. Examples of co-therapy or co-treatment include 5 mg/kg bevacizumab (Avastin®) every two week with bolus-IFL or 10 mg/kg bevacizumab (Avastin®) every 2 weeks with FOLFOX4 for metastatic colorectal cancer, 15 mg/kg bevacizumab (Avastin®) every 3 weeks with caboplatis/paclitaxel for non-squamous non-small cell lung cancer, and 10 mg/kg bevacizumab (Avastin®) every 2 weeks with paclitaxel for metastatic breast cancer. Further, the angiogenesis inhibitor to be administered may be administered as a co-therapy or a co-treatment with radiotherapy.

As used herein "chemotherapeutic agent" or "chemotherapy regimen" includes any active agent that can provide an anticancer therapeutic effect and may be a chemical agent or a biological agent, in particular, that are capable of interfering with cancer or tumor cells. Preferred active agents are those that act as anti-neoplastic (chemotoxic or chemostatic) agents which inhibit or prevent the development, maturation or proliferation of malignant cells. Nonlimiting examples of chemotherapeutic agents include alkylating agents such as nitrogen mustards (e.g., mechlorethamine, cyclophosphamide, ifosfamide, melphalan and chlorambucil), nitrosoureas (e.g., carmustine (BCNU), lomustine (CCNU), and semustine (methyl-CCNU)), ethylenimines/ methylmelamines (e.g., thriethylenemelamine (TEM), triethylene, thiophosphoramide (thiotepa), hexamethylmelamine (HMM, altretamine)), alkyl sulfonates (e.g., busulfan), and triazines (e.g., dacarbazine (DTIC)); antimetabolites such as folic acid analogs (e.g., methotrexate, trimetrexate), pyrimidine analogs (e.g., 5-fluorouracil, capecitabine, fluorodeoxyuridine, gemcitabine, cytosine arabinoside (AraC, cytarabine), 5-azacytidine, 2,2'-difluorodeoxycytidine), and purine analogs (e.g., 6-mercaptopurine, 6-thioguanine, azathioprine, 2'-deoxycoformycin (pentostatin), erythrohydroxynonyladenine (EHNA), fludarabine phosphate, and 2-chlorodeoxyadenosine (cladribine, 2-CdA)); antimitotic drugs developed from natural products (e.g., paclitaxel, vinca alkaloids (e.g., vinblastine (VLB), vincristine, and vinorelbine), docetaxel, estramustine, and estramustine phosphate), epipodophylotoxins (.e.g., etoposide, teniposide), antibiotics (.e.g, actimomycin D, daunomycin (rubidomycin), daunorubicon, doxorubicin, epirubicin, mitoxantrone, idarubicin, bleomycins, plicamycin (mithramycin), mitomycinC, actinomycin), enzymes (e.g., L-asparaginase), and biological response modifiers (e.g., interferon-alpha, IL-2, G-CSF, GM-CSF); miscellaneous agents including platinum coordination complexes (e.g., cisplatin, carboplatin, oxaliplatin), anthracenediones (e.g., mitoxantrone), substituted urea (i.e., hydroxyurea), methylhydrazine derivatives (e.g., N-methylhydrazine (MIH), procarbazine), adrenocortical suppressants (e.g., mitotane (o,p'-DDD), aminoglutethimide); hormones and antagonists including adrenocorticosteroid antagonists (.e.g, prednisone and equivalents, dexamethasone, aminoglutethimide), progestins (e.g., hydroxyprogesterone caproate, medroxyprogesterone acetate, megestrol acetate), estrogens (e.g., diethylstilbestrol, ethinyl estradiol and equivalents thereof); antiestrogens (e.g., tamoxifen), androgens (e.g., testosterone propionate, fluoxymesterone and equivalents thereof), antiandrogens (e.g., flutamide, gonadotropin-releasing hormone analogs, leuprolide), non-steroidal antiandrogens (e.g., flutamide), epidermal growth factor inhibitors (e.g., erlotinib, lapatinib, gefitinib) antibodies (e.g., trastuzumab), irinotecan and other agents such as leucovorin. For the treatment of metastatic pancreatic cancer, chemotherapeutic agents for administration with bevacizumab include gemcitabine and erlotinib and combinations thereof (see also the examples herein provided). For the treatment of renal cell cancer, chemotherapeutic agents for administration with bevacizumab include interferon alpha (see also the examples herein provided).

The terms "responder for an angiogenesis inhibitor" means in the context of the present invention that a subject/patient suffering from a malignant disease or a disease involving physiological and pathological angiogenesis shows a response to a treatment with the angiogenesis inhibitor. A skilled person will readily be in a position to determine whether a person treated with the angiogenesis inhibitor shows a response. For example, a response to an angiogenesis inhibitor may be reflected by decreased suffering from a malignant disease or a disease involving physiological and pathological angiogenesis, such as a diminished and/or halted growth of a tumor and/or a reduction of the size of a tumor, the prevention of the formation of metastases or a reduction of number or size of metastases.

The terms "patient sensitive to an angiogenesis inhibitor" in the context of the present invention refers to a patient which shows in some way a positive reaction when treated with an angiogenesis inhibitors. The reaction of the patient may be less pronounced when compared to a responder as described hereinabove. For example, the patient may experience less suffering from a malignant disease, such as cancer, though no reduction in tumor growth may be measured. The reaction of the patient to the angiogenesis inhibitor may also be only of a transient nature, i.e. growth of (a) tumor and/or (a) metastasis(es) may only be temporarily reduced or halted.

The terms "a patient suffering from" in the context of the present invention refers to a patient showing clinical signs in respect to a certain malignant disease, such as cancer, a disease involving physiological and pathological angiogenesis and/or tumorous disease.

The terms "for improving overall survival" in the context of the present invention refer to the average survival of the patient within a patient group. As the skilled person will appreciate, a patient's overall survival is improved or enhanced, if the patient belongs to a subgroup of patients that has a statistically significant longer mean survival time as compared to another subgroup of patients.

The terms "for improving progression-free survival" in the context of the present invention refer, with respect to a patient within a patient group, to the average length of time during and after treatment in which a patient's disease does not get worse. As the skilled person will appreciate, a patient's progression-free survival is improved or enhanced, if the patient belongs to a subgroup of patients that has a significantly longer mean length of time during which the disease does not get worse compared to another subgroup of patients.

The terms "oligonucleotide" and "polynucleotide" are used interchangeably and in the context of the present invention refer to a molecule comprised of two or more deoxyribonucleotides or ribonucleotides, preferably more than three. Its exact size will depend on many factors, which in turn depend on the ultimate function or use of the oligonucleotide. An oligonucleotide can be derived synthetically or by cloning. Chimeras of deoxyribonucleotides and ribonucleotides may also be in the scope of the present invention.

Sequence amplification is a method for generating large amounts of a target sequence. In general, one or more amplification primers are annealed to a nucleic acid sequence. Using appropriate enzymes, sequences found adjacent to or in between the primers are amplified.

Amplification primer is an oligonucleotide that is capable of annealing to a target sequence and servings as an initiation point for DNA synthesis when placed under conditions in which the synthesis of primer extension product that is complementary to a nucleic acid is initiated.

Primers according to the present invention can be designed as commonly known in the art based on the sequences of the SNPs provided herein. Primers are preferably 10 to 45 nucleotides long and more preferably 15 to 30 nucleotides long.

According to the invention herein described, a primer designed to bind upstream of a SNP is used in combination with a second primer designed to bind downstream of the same SNP. Examples of nucleic acid primers derived for SNPs rs9582036 (SEQ ID NO. 2), rs9554316 (SEQ ID NO. 1), rs9513070 (SEQ ID NO. 3) and rs9554320 (SEQ ID NO. 4) are shown in Figure 2. More specifically, for rs9554316 (SEQ ID NO. 1), ACGTTGGATGATCGTAAAGACATCATTCG (SEQ ID NO. 25) and ACGTTGGATGTGCTGGAGACCATGACCAAG (SEQ ID NO. 26) can be used. For SNPs rs9582036 (SEQ ID NO. 2), ACGTTGGATGACTGTGCCCAGCAACAATAG (SEQ ID NO. 20) and ACGTTGGATGGCATAATAGCACTTTACTCC (SEQ ID NO. 21) can be used. For rs9513070 (SEQ ID NO. 3), ACGTTGGATGGCAAGCTTGCCCAACTTGTG (SEQ ID NO. 35) and ACGTTGGATGGTTTGTGTTGGGCTGCACTC (SEQ ID NO. 36) can be used. For rs9554320 (SEQ ID NO.4), ACGTTGGATGGTGGGAGGCCAGTTTGTAAC (SEQ ID NO. 30) and ACGTTGGATGAGCAAGGTTCCTGTGTGTAG (SEQ ID NO. 31) can be used.

As will be understood by the person of ordinary skill, a multitude of probes can be designed for each SNP identified herein. For example, probes that are extendable primers, which may be extended in a termination extension reaction, are shown in Figure 2 with there extension products. For rs9554316 (SEQ ID NO. 1), AAGACATCATTCGATTTTTTTTCT (SEQ ID NO. 27) was used. For SNPs rs9582036 (SEQ ID NO. 2), AGCAACAATAGCCTTCTT (SEQ ID NO. 22) was used. For rs9513070 (SEQ ID NO. 3), CGTGTGGCCCACGGGCT (SEQ ID NO. 37) was used. For rs9554320 (SEQ ID NO.4), ACAGCGGCTTTGCAGTGC (SEQ ID NO. 32) was used. Probes that are extendable primers are preferably 15 to 30 nucleotides long and more preferably 15 to 25 nucleotides long.

The present invention provides oligonucleotides or polynucleotides useful for determining the presence of at least one or more variant alleles of the VEGF-R1 gene. The oligonucleotides or polynucleotides may comprise primers and/or probes. With respect to primers, the primers may be selected from the group consisting of SEQ ID NO. 20, SEQ ID NO. 25, SEQ ID NO. 30, SEQ ID NO. 35, SEQ ID NO. 21, SEQ ID NO. 26, SEQ ID NO. 31 and SEQ ID NO. 36. With respect to probes, that the probes may be selected form the group consisting of SEQ ID NO. 22, SEQ ID NO. 27, SEQ ID NO. 32 and SEQ ID NO. 37.

A skilled person will also have the ability to use probes, such as hybridization probes, that can be labelled by standard labelling techniques such as with a radiolabel, enzyme label, fluorescent label, biotin-avidin label, chemiluminescence, and the like. After hybridization, the probes can be visualized using known techniques.

The present invention also relates to a diagnostic composition or kit comprising any of the mentioned oligonucleotides and optionally suitable means for detection.

The kit of the invention may advantageously be used for carrying out a method of the invention and could be, inter alia, employed in a variety of applications, e.g., in the diagnostic field or as a research tool. The parts of the kit of the invention can be packages individually in vials or in combination in containers or multicontainer units. Manufacture of the kit follows preferably standard procedures which are known to the person skilled in the art. The kit or diagnostic compositions may be used for detection of the one or more variant alleles of the VEGFR-1 gene in accordance with the herein-described methods of the invention, employing, for example, amplification techniques as described herein.

Accordingly, in a further embodiment of the present invention provides a kit useful for carrying out the methods herein described, comprising oligonucleotides or polynucleotides capable of determining the presence of at least one or more variant alleles of the VEGF-R1 gene. The oligonucleotides or polynucleotides may comprise primers and/or probes. With respect to primers, the primers may be selected from the group consisting of SEQ ID NO. 20, SEQ ID NO. 25, SEQ ID NO. 30, SEQ ID NO. 35, SEQ ID NO. 21, SEQ ID NO. 26, SEQ ID NO. 31 and SEQ ID NO. 36. With respect to probes, the probes may be selected form the group consisting of SEQ ID NO. 22, SEQ ID NO. 27, SEQ ID NO. 32 and SEQ ID NO. 37.

The present invention is further described by reference to the following non-limited figures and examples.

**Figure 1:** Sequence of SNPs. The diagnostic allele for each SNP is identified. Figure 1A are SNPs genotyped in AVITA study and associated with bevacizumab outcome. Figure 1B are linked SNPs belonging to the rs9554316 (SEQ ID NO. 1) and rs9582036 (SEQ ID NO. 2) haplotype block (within the locus). Figure 1C are linked SNPs 5' up or downstream of the haplotype block (outside the locus) and in LD (D'>0.8) with rs9554316 (SEQ ID NO. 1) and rs9582036 (SEQ ID NO. 2). Figure 1D are linked SNPs with $r^2$ equal or larger than 0.12 to rs9554316 (SEQ ID NO. 1), rs9582036 (SEQ ID NO. 2), rs9513070 (SEQ ID NO. 3) or rs9554320 (SEQ ID NO.4).

**Figure 2:** Primers for SNPs rs9554316 (SEQ ID NO. 1), rs9582036 (SEQ ID NO. 2), rs9513070 (SEQ ID NO. 3) and rs9554320 (SEQ ID NO. 4). The unextended primers (probes), extended primers (probes) and their masses are also provided.

**Figure 3:** Kaplan Meier curve: Overall Survival, probability vs. time on study (days), by rs9582036 (SEQ ID NO. 2) genotype in bevacizumab treated sub-group of AVITA trial (patients with metastatic pancreatic cancer treated with bevacizumab plus gemcitabine-erlotinib (GE) therapy). In the legend 0, 1 and 2 refer to the genotypes: 0 is AA (n=40, median=309), 1 is AC (n=28, median=171) and 2 is CC (n=9, median=144). 1 vs 0 (HR=2.00 [3.36, 1.19], Wald-test p=0.0091) and 2 vs 0: (HR=4.72 [10.68, 2.08], Wald-test p=0.0002). In the figure, the solid line is for genotype AA, the dashed line is for genotype AC, and the dotted line is for genotype CC.

**Figure 4:** Kaplan Meier curve: Overall Survival, probability vs. time on study (days), by rs9582036 (SEQ ID NO. 2) genotype in placebo treated sub-group of AVITA trial (patients with metastatic pancreatic cancer treated with placebo plus gemcitabine-erlotinib (GE) therapy). In the legend 0, 1 and 2 refer to the genotypes: 0 is AA (n=38, median=226), 1 is AC (n=29, median=177) and 2 is CC (n=10, median=149). 1 vs 0 (HR=1.62 [2.68, 0.97], Wald-test p=0.063) and 2 vs 0: (HR=1.53 [3.12, 0.75], Wald-test p=0.240). In the figure, the solid line is for genotype AA, the dashed line is for genotype AC, and the dotted line is for genotype CC.

**Figure 5:** Kaplan Meier curve: Progression Free Survival, probability vs. time on study (days), by rs9582036 (SEQ ID NO. 2) genotype in bevacizumab treated sub-group of AVITA trial (patients with metastatic pancreatic cancer treated with bevacizumab plus gemcitabine-erlotinib (GE) therapy). In the legend 0, 1 and 2 refer to the genotypes: 0 is AA (n=40, median=218), 1 is AC (n=28, median=133) and 2 is CC (n=9, median=102). 1 vs 0 (HR=1.86 [3.10, 1.11], Wald-test p=0.0180) and 2 vs 0: (HR=3.63 [8.05, 1.64], Wald-test p=0.0015). In the figure, the solid line is for genotype AA, the dashed line is for genotype AC, and the dotted line is for genotype CC.

**Figure 6:** Kaplan Meier curve: Progression Free Survival, probability vs. time on study (days), by rs9582036 (SEQ ID NO. 2) genotype in placebo treated sub-group of AVITA trial (patients with metastatic pancreatic cancer treated with placebo plus gemcitabine-erlotinib (GE) therapy). In the legend 0, 1 and 2 refer to the genotypes: 0 is AA (n=38, median=147), 1 is AC (n=29, median=120) and 2 is CC (n=10, median=134). 1 vs 0 (HR=1.36 [2.22, 0.83], Wald-test p=0.23) and 2 vs 0: (HR=1.04 [2.11, 0.51], Wald-test p=0.91). In the figure, the solid line is for genotype AA, the dashed line is for genotype AC, and the dotted line is for genotype CC.

**Figure 7:** Kaplan Meier curve: Overall Survival, probability vs. time on study (days), by rs9554316 (SEQ ID NO. 1) genotype in bevacizumab treated sub-group of AVITA trial (patients with metastatic pancreatic cancer treated with bevacizumab plus gemcitabine-erlotinib (GE) therapy). In the legend 0, 1 and 2 refer to the genotypes: 0 is GG (n=42, median=309), 1 is TG (n=30, median=148) and 2 is TT (n=5, median=132). 1 vs 0 (HR=2.07 [3.43, 1.25], Wald-test p=0.0047) and 2 vs 0: (HR=4.69 [12.59, 1.75], Wald-test p=0.0021). In the figure, the solid line is for genotype GG, the dashed line is for genotype TG, and the dotted line is for genotype TT.

**Figure 8:** Kaplan Meier curve: Overall Survival, probability vs. time on study (days), by rs9554316 (SEQ ID NO. 1) genotype in placebo treated sub-group of AVITA trial (patients with metastatic pancreatic cancer treated with placebo plus gemcitabine-erlotinib (GE) therapy). In the legend 0, 1 and 2 refer to the genotypes: 0 is GG (n=43, median=222), 1 is TG (n=26, median=177) and 2 is TT (n=7, median=149). 1 vs 0 (HR=1.34 [2.22, 0.81], Wald-test p=0.25) and 2 vs 0: (HR=1.36 [3.04, 0.60], Wald-test p=0.46). In the figure, the solid line is for genotype GG, the dashed line is for genotype TG, and the dotted line is for genotype TT.

**Figure 9:** Kaplan Meier curve: Progression Free Survival, probability vs. time on study (days), by rs9554316 (SEQ ID NO. 1) genotype in bevacizumab treated sub-group of AVITA trial (patients with metastatic pancreatic cancer treated with bevacizumab plus gemcitabine-erlotinib (GE) therapy). In the legend 0, 1 and 2 refer to the genotypes: 0 is GG (n=42, median=213), 1 is TG (n=30, median=121) and 2 is TT (n=5, median=65). 1 vs 0 (HR=1.86 [3.05, 1.13], Wald-test p=0.0150) and 2 vs 0: (HR=4.60 [12.42, 1.70], Wald-test p=0.0026). In the figure, the solid line is for genotype GG, the dashed line is for genotype TG, and the dotted line is for genotype TT.

**Figure 10:** Kaplan Meier curve: Progression Free Survival, probability vs. time on study (days), by rs9554316 (SEQ ID NO. 1) genotype in placebo treated sub-group of AVITA trial (patients with metastatic pancreatic cancer treated with placebo plus gemcitabine-erlotinib (GE) therapy). In the legend 0, 1 and 2 refer to the genotypes: 0 is GG (n=43, median=122), 1 is TG (n=26, median=121) and 2 is TT (n=7, median=134). 1 vs 0 (HR=1.26 [2.06, 0.77], Wald-test p=0.3700) and 2 vs 0: (HR=1.00 [2.25, 0.45], Wald-test p=0.9900). In the figure, the solid line is for genotype GG, the dashed line is for genotype TG, and the dotted line is for genotype TT.

**Figure 11:** Kaplan Meier curve: Progression Free Survival, probability vs. study month, for overall population of AVOREN trial (patients with renal cell cancer treated with bevacizumab plus interferon alpha (IFN) therapy or placebo plus interferon alpha (IFN) therapy). In the figure, the dashed line is for patients treated with placebo plus interferon alpha and the solid line is for patients treated with bevacizumab plus interferon alpha.

**Figure 12:** Kaplan Meier curve: Progression Free Survival, probability vs. study month, for genotype population of AVOREN trial (patients with renal cell cancer treated with bevacizumab plus interferon alpha (IFN) therapy or placebo plus interferon alpha (IFN) therapy). In the figure, the dashed line is for patients treated with placebo plus interferon alpha and the solid line is for patients treated with bevacizumab plus interferon alpha.

**Figure 13:** Kaplan Meier curve: Progression Free Survival, probability vs. time on study (month), by rs9513070 (SEQ ID NO. 3) genotype in bevacizumab treated sub-group of AVOREN trial (patients with renal cell cancer treated with bevacizumab plus interferon alpha (IFN) therapy). The genotypes are AA (n=19/17, med=18.53), AG (n=28/28, med=12.78) and GG (n=9/9, med=13.6). AG vs AA (HR=1.89 [3.50, 1.02], Wald-test p=0.044) and GG vs AA: (HR=2.69 [6.29, 1.15], Wald-test p=0.022). In the figure, the solid line is for genotype AA, the dashed line is for genotype AG, and the dotted line is for genotype GG.

**Figure 14:** Kaplan Meier curve: Progression Free Survival, probability vs. time on study (month), by rs9513070 (SEQ ID NO. 3) genotype in placebo treated sub-group of AVOREN trial (patients with renal cell cancer treated with placebo plus interferon alpha (IFN) therapy). The genotypes are AA (n=11/10, med=5.52), AG (n=26/19, med=14.23) and GG (n=8/8, med=3.71). AG vs AA (HR=0.44 [0.97, 0.21], Wald-test p=0.04) and GG vs AA: (HR=1.54 [3.95, 0.60], Wald-test p=0.37). In the figure, the solid line is for genotype AA, the dashed line is for genotype AG, and the dotted line is for genotype GG.

**Figure 15:** Kaplan Meier curve: Progression Free Survival, probability vs. time on study (month), by rs9554316 (SEQ ID NO. 1) genotype in bevacizumab treated sub-group of AVOREN trial (patients with renal cell cancer treated with bevacizumab plus interferon alpha (IFN) therapy). The genotypes are GG (n=36/34, med=16.66), GT (n=18/18, med=10.15) and TT (n=1/1, med=14.52). GT vs GG (HR=1.96 [3.56, 1.08], Wald-test p=0.026) and TT vs GG: (HR=2.15 [16.17, 0.29], Wald-test p=0.457). In the figure, the solid line is for genotype GG, the dashed line is for genotype GT, and the dotted line is for genotype TT.

**Figure 16:** Kaplan Meier curve: Progression Free Survival, probability vs. time on study (month), by rs9554316 (SEQ ID NO. 1) genotype in placebo treated sub-group of AVOREN trial (patients with renal cell cancer treated with placebo plus interferon alpha (IFN) therapy). The genotypes are GG (n=26/21, med=7.95), GT (n=17/15, med=13.37) and TT (n=2/1, med=8.11). GT vs GG (HR=0.944 [1.839, 0.485], Wald-test p=0.866) and TT vs GG: (HR=0.413 [3.082, 0.055], Wald-test p=0.389). In the figure, the solid line is for genotype GG, the dashed line is for genotype GT, and the dotted line is for genotype TT.

As documented in the appended illustrative examples, the means and methods provided herein relate to analysis and evaluation of biological samples from Caucasians.

**Example 1:**

Genetic determination can influence sensitivity of the endothelium to VEGF. In accordance with this and in context of this invention, we explored genetic variability in underlying signaling pathways in order to discover predictive patterns for anti angiogenic treatment. In this analysis, the correlation of genetic variability in the VEGF signaling pathway with clinical outcome of patients with metastatic pancreatic cancer treated with gemcitabine-erlotinib (GE) plus bevacizumab or placebo in a phase III trial (AVITA) was evaluated.

Germline DNA was available from 154 out of 607 patients, of which 77 received GE plus bevacizumab and 77 GE alone (median overall survival = 7.5 and 6.8 months); see AVITA study (BO17706) accessible at http://www.roche-trials.com/patient/trials/trial11.html. Common single nucleotide polymorphisms (SNPs) located in the hypoxia-inducible factor-la and -2$\alpha$, VEGF, its receptors (VEGFR-1 and -2) and other relevant genes were selected using a SNP tagging approach (f$\geq$0.1 and $r^2 \leq 0.8$). 157 SNPs were successfully genotyped using MALDI-TOF mass spectrometry. Risk and survival estimates were calculated using Cox regression analyses.

Four SNPs in the VEGFR-1 gene correlated with overall survival and progression-free survival in the bevacizumab -treated group. The most significant SNP, rs9582036, had p-value<=3e-4 in an allelic risk effect model. Relative to AA carriers, the hazard ratio was 2.0 (CI=1.2-3.4; p=0.009) and 4.7 (CI=2.1-10.7; p=0.0002) for AC and CC carriers, respectively. Median overall survival was increased from 4.8 months in CC (n=9) carriers to 6.0 and 10.3 months in AC (n=28) and AA (n=40) carriers. A similar association was observed with the PFS end-point. After adjustment for baseline prognostic factors (neutrophil counts, CRP and tumor location) the effect was attenuated but not suppressed. No effect was detected in the control group. A test for interaction between rs9582036 (SEQ ID NO. 2) genotype and treatment resulted in a p value of 0.02. All 4 SNPs were located in the same chromosomal region encompassing exons 25 to 30 of the VEGFR-1 gene and coding for the essential receptor tyrosine-kinase (TK) domain. It was, furthermore, surprisingly found that no association with the previously reported VEGF -2578C/A polymorphism could be detected in this data set.

These subgroup data provide for a genetic locus in the VEGFR-1 gene, in particular, in the TK domain of VEGFR-1 that correlates with clinical outcome in cancer patients, for example in pancreatic cancer patients treated with an angiogenesis inhibitor, like bevacizumab. Accordingly, predictive values using the herein described parameters can be obtained. These parameters, i.e. the herein described variations of the VEGFR-1 gene can be used for the assessment of the beneficial effect of anti-angiogenic treatment in subjects suffering from cancer. Accordingly, the invention as provided herein relates to the provision of novel and inventive biomarkers for predictive as well as prognostic assessments.

**PATIENTS AND METHODS**

**Samples**

In the AVITA trial, there were 607 eligible patients. The trial protocol was approved by the institutional review board at each site and was conducted in accordance with the Declaration of Helsinki, current US Food and Drug Administration Good Clinical Practices, and local ethical and legal requirements. Frozen whole-blood samples were available from 160 out of 607 patients (26.4%), 154 of these had a disease progression event and for 150 patients the disease was fatal. Of these 160 patients, 6 were of Asian ethnicity, while all remaining 154 patients were of Caucasian origin. As patients of Asian origin are genetically distinct from Caucasian patients and SNP frequencies may differ between both ethnic groups, these 6 patients were omitted from further analysis. The remaining 154 DNA specimens were provided to the investigators in a de-identified fashion. All corresponding patients provided separate written informed consent for genetic biomarker testing.

**Assessments**

Patients were assessed as described previously (Van Cutsem et al., J. Clinc. Oncol. 27, 2231-7 (2009)). Briefly, tumor assessments were made according to Response Evaluation Criteria in Solid Tumors guidelines at baseline, weeks 8, 16, 24, 32, 40, and every 12 weeks thereafter until disease progression. Patients were followed for survival and subsequent disease treatment until death, loss to follow-up, or trial termination. Assessments of Karnofsky performance status (KPS) and body weight were collected weekly. Adverse events were recorded at every visit using the National Cancer Institute Common Terminology Criteria for Adverse Events version 3.0 (NCICTCAE v3). Blood samples for genetic biomarker analyses were collected before trial treatment began.

**Single Nucleotide Polymorphism Selection**

The following genes involved in the VEGF signalling cascade were selected: the VEGF ligand, the VEGF homologues (placental growth factor or PlGF, VEGF-B and -C, as well as VEGF-D or FIGF), the VEGF receptor-2 (KDR or VEGFR-2) and VEGF receptor-1 (FLT1 or VEGFR-1). Genomic sequences 5 kb upstream of the translation start site up to 3' poly-A adenylation site of each gene, were used to select SNPs from the HapMap database (HapMap Data Rel 24/phaseII Nov08, on NCBI B36 assembly, dbSNP b126). Tagging SNPs were selected using the Tagger (Pe'er, I., et al., Nat. Genet. 38, 663-7 (2006)) as provided in the HAPLOVIEW software package (Barrett, J.C., et al., Bioinformatics. 21, 263-5 (2005)). Only SNPs occurring commonly, i.e., with a minor allele frequency f$\geq$0.1 and a minimum $r^2$ threshold $\geq$0.8 were considered. In total, 167 tagging SNPs were selected following these criteria. Additionally, 11 SNPs located in exonic sequences and inducing non-synonymous amino-acid changes at a frequency f$\geq$0.1 were selected from the dbSNP database, as well as 4 SNPs in VEGF (rs699947, rs833061, rs2010963 and rs3025039), 1 SNP in VEGFR-1 (rsTP53_R-1) and 1 SNP in VEGFR-2 (rs2071559), which previously have been reported to affect function or expression of these genes.

The chromosomal location and position of each SNP within the VEGFR-1 gene for SNPs rs9582036 (SEQ ID NO. 2), rs9554316 (SEQ ID NO. 1), rs9513070 (SEQ ID NO. 3) and rs9554320 (SEQ ID NO.4) is shown, together with the allelic variation and the reference allele.

| Variation ID | Position (bp) | Position to Transcript | Alleles | Reference Allele | Genotyping Assay | Frequency (N=154) |
|---|---|---|---|---|---|---|
| rs9554320 (SEQ ID NO. 4) | 27784927 | Intron 25 | A/C | A | 6 | 0.43 |
| rs9582036 (SEQ ID NO. 2) | 27783408 | Intron 27 | C/A | C | 3 | 0.7 |
| rs9554316 (SEQ ID NO. 1) | 27779335 | Intron 28 | T/G | T | 7 | 0.25 |
| rs9513070 (SEQ ID NO. 3) | 27777839 | Intron 29 | G/A | A | 5 | 0.60 |

## Genotyping

Peripheral blood was sampled in K2EDTA plastic Vacutainer tubes. After centrifugation, germ-line DNA was extracted from the precipitated leucocyte cell fraction according to standard procedures. Genotyping for the selected SNPs was carried out at the Vesalius Research Center using the Sequenom® iPLEX platform, as described previously by the manufacturer. All the SNPs that failed to provide robust genotypes in a first genotyping round were re-designed using a different set of PCR primers and tested again. In case of repetitive failure in the second design these SNPs were considered as failed. Of the 184 selected SNPs, 27 SNPs (14.7%) failed and 157 (85.3%) were successfully genotyped. The overall genotype success rate was 98.5%. At the individual sample level, all samples were genotyped with a high success rate (i.e., <5% of the samples had a success rate <95%, whereas >70% of the samples had a success rate >99%). No samples were excluded from the analysis and no significant deviations from Hardy-Weinberg were observed.

The amplification primers designed for SNPs rs9582036 (SEQ ID NO. 2), rs9554316 (SEQ ID NO. 1), rs9513070 (SEQ ID NO. 3) and rs9554320 (SEQ ID NO.4) are shown in Figure 2. For rs9554316 (SEQ ID NO. 1), ACGTTGGATGATCGTAAAGACATCATTCG (SEQ ID NO. 25) and ACGTTGGATGTGCTGGAGACCATGACCAAG (SEQ ID NO. 26) were used. For SNP rs9582036 (SEQ ID NO. 2), ACGTTGGATGACTGTGCCCAGCAACAATAG (SEQ ID NO. 20) and ACGTTGGATGGCATAATAGCACTTTACTCC (SEQ ID NO. 21) were used. For rs9513070 (SEQ ID NO. 3), ACGTTGGATGGCAAGCTTGCCCAACTTGTG (SEQ ID NO. 35) and ACGTTGGATGGTTTGTGTTGGGCTGCACTC (SEQ ID NO. 36) were used. For rs9554320 (SEQ ID NO.4), ACGTTGGATGGTGGGAGGCCAGTTTGTAAC (SEQ ID NO. 30) and ACGTTGGATGAGCAAGGTTCCTGTGTGTAG (SEQ ID NO. 31) were used.

The unextended primers (probes) designed for SNPs rs9582036 (SEQ ID NO. 2), rs9554316 (SEQ ID NO. 1), rs9513070 (SEQ ID NO. 3) and rs9554320 (SEQ ID NO.4) are also shown in Figure 2. For rs9554316 (SEQ ID NO. 1), AAGACATCATTCGATTTTTTTTCT (SEQ ID NO. 28) was used. For SNPs rs9582036 (SEQ ID NO. 2), AGCAACAATAGCCTTCTT (SEQ ID NO. 22) was used. For rs9513070 (SEQ ID NO. 3), CGTGTGGCCCACGGGCT (SEQ ID NO. 37) was used. For rs9554320 (SEQ ID NO.4), ACAGCGGCTTTGCAGTGC (SEQ ID NO. 32) was used.

## Statistics

When the allelic model was used, patients homozygous with the minor allele were coded as 2, heterozygous patients were coded as 1, and patients homozygous for the major allele were coded as 0. An independent, separate model was modelled for each SNP.

The Cox proportional hazard regression model was used to evaluate the effect of different genotypes on Overall Survival and Progression Free Survival.

In the Cox model, the time from entry in the study until death or censoring was used as the Overall Survival end-point. Time from entry in the study until disease progression or death was used and the Progression Survival end-point.

For overall survival, the null hypothesis stated that the genotype has no effect on overall survival. The likelihood ratio test was used to calculate a P value corresponding to this hypothesis for each tested SNP.

For progression free survival, the null hypothesis stated that the genotype has no effect on risk of progression or survival. The likelihood ratio test was used to calculate a P value corresponding to this hypothesis for each tested SNP.

The primary analysis were conducted in the sub-group of patients in the bevacizumab arm of the trial. Additional analyses were performed in the sub-group of patients in the placebo arm of the trial, and treatment by genotype interaction was evaluated in model including both.

In the primary analysis, only the genotypes were included as predictors in the model. In order to further characterize and confirm the primary results, some complimentary analyses were conducted with adjustment for ancillary clinical prognostic factors. This was achieved by running additional regression models taking these prognostic factor as covariates in addition to the effect of genotype.

To account for multiple testing, p-value was considered significant if below 0.0005 (5.e-4).

Survival curves and median survival time were calculated using standard Kaplan-Meier estimates.

**Clinical Characteristics of genetic patient population**

Since germ-line DNA was obtained from only 26.4% of the study participants (160 out of 607 patients), it was first assessed whether this subgroup exhibited similar disease characteristics as the full patient cohort. All baseline demographics characteristics of the genetics subpopulation were similar to the overall study population. There were 77 patients in the placebo arm and 77 patients in the bevacizumab arm. Overall, the subgroup available for genetic analysis behaved very similar, exhibiting a comparable age and gender distribution, smoking status, KPS, visual analogue scale for pain, progression free survival and overall survival. The median overall survival in the sub-group was 7.2 and 6.1 months in the bevacizumab and placebo arm, median progression free survival was 4.6 and 3.6 months respectively.

Baseline demographics were balanced between treatment arms.

| | First trial medication | | | | All | |
|---|---|---|---|---|---|---|
| | Bevacizumab | | Placebo | | | |
| | N | % | N | % | N | % |
| **Sex** | | | | | | |
| **FEMALE** | 26 | 37.14 | 25 | 37.31 | 51 | 37.23 |
| **MALE** | 44 | 62.86 | 42 | 62.69 | 86 | 62.77 |
| **Age Category (years)** | | | | | | |
| **<65** | 41 | 58.57 | 44 | 65.67 | 85 | 62.04 |
| **>=65** | 29 | 41.43 | 23 | 34.33 | 52 | 37.96 |
| **KPS (%) Category at Baseline** | | | | | | |
| **<80%** | 8 | 11.43 | 6 | 8.96 | 14 | 10.22 |
| **>=80%** | 62 | 88.57 | 61 | 91.04 | 123 | 89.78 |
| **VAS Category at Baseline** | | | | | | |
| **<20** | 47 | 67.14 | 50 | 74.63 | 97 | 70.80 |
| **>=20** | 23 | 32.86 | 17 | 25.37 | 40 | 29.20 |
| **KPS : karnofsky performance status**<br>**VAS : Visual Analogue Scale of Pain at Baseline** | | | | | | |

SNPs in VEGFR-1 correlated with overall survival in the treatment arm

**Overall Survival Analysis**

**Analysis in the sub-group of patients treated with Bevacizumab**

Overall Survival by Genotype - No covariates - Subset : Bev Arm

| | snp | gene | HR | CI_95 | pvalue | star | adj.pvalue | N/Nevts |
|---|---|---|---|---|---|---|---|---|
| 152 | rs9582036 | VEGFR1 | 2.113 | (1.45,3.07) | 1.4e-04 | *** | 2.2e-02 | 77/68 |
| 150 | rs9554316 | VEGFR1 | 2.119 | (1.42,3.17) | 4.2e-04 | *** | 6.6e-02 | 77/68 |
| 80 | rs3025035 | VEGF | 0.327 | (0.14,0.77) | 1.7e-03 | ** | 2.6e-01 | 74/65 |
| 146 | rs9513070 | VEGFR1 | 1.67 | (1.14,2.44) | 8.1e-03 | ** | 1.0e+00 | 74/65 |
| 151 | rs9554320 | VEGFR1 | 1.543 | (1.11,2.14) | 9.7e-03 | ** | 1.0e+00 | 75/67 |

**Analysis in the sub-group of patients treated with Placebo**

Overall Survival by Genotype - No covariates - Subset : Placebo Arm

| | snp | gene | HR | CI_95 | pvalue | star |
|---|---|---|---|---|---|---|
| 151 | rs9554320 | VEGFR1 | 1.377 | (1.02, 1.85) | 3.6e-02 | * |
| 149 | rs9554311 | VEGFR1 | 1.541 | (1.01, 2.34) | 4.3e-02 | * |
| 134 | rs7982639 | VEGFR1 | 1.554 | ( 1, 2.42) | 5.5e-02 | |
| 128 | rs7673274 | VEGFR2 | 0.721 | (0.51, 1.02) | 5.8e-02 | |
| 29 | rs1485766 | VEGF-C | 0.707 | (0.49, 1.02) | 6.0e-02 | |

No genotypes were detected to be associated with overall survival in the placebo sub-group.

**Analysis of treatment by genotype interaction**

Overall Survival by Genotype - No covariates - Interaction Test - Subset:NULL

| | snp | HR_SNP | HR_treat | HR_inter | pval_inter | N/Nevts star | gene |
|---|---|---|---|---|---|---|---|
| 150 | rs9554316 | 1.189 | 0.587 | 1.814 | 2.3e-02 | 153/143 * | VEGFR1 |
| 152 | rs9582036 | 1.302 | 0.635 | 1.632 | 4.1e-02 | 154/143 * | VEGFR1 |
| 19 | rs12505758 | 0.73 | 0.69 | 1.845 | 9.1e-02 | 154/143 | VEGFR2 |
| 93 | rs3936415 | 1.301 | 1.016 | 0.668 | 1.3e-01 | 154/143 | VEGFR1 |

A Cox regression analysis was performed for each of the SNPs to assess an effect on overall survival in the treatment arm. To correct for multiple testing, the significance level was set at p<0.0005. Two SNPs predicted a favourable median overall survival (p=0.00014 and p=0.00042 for rs9582036 (SEQ ID NO. 2) and rs9554316 (SEQ ID NO. 1), respectively). Both SNPs were located in VEGFR-1 and demonstrated a superior overall survival with an additive risk effect: relative to rs9582036 (SEQ ID NO. 2) AA carriers, the hazard ratio (HR) was 2.0 (CI=1.2-3.4; p=0.009) and 4.7 (CI=2.1-10.7; p=0.0002) for AC and CC carriers, respectively. Median overall survival was increased from 4.8 months in CC carriers to 6.0 and 10.3 months in AC and AA carriers. Likewise, the hazard ratio for TG and GG carriers in the rs9554316 (SEQ ID NO. 1) SNP was 2.07 (CI=1.25-3.43; p=0.0047) and 4.69 (CI=1.75-12.95; p=0.0021). Intriguingly, two other SNPs in the VEGFR-1 gene, i.e. rs9513070 (SEQ ID NO. 3) and rs9554320 (SEQ ID NO. 4), also correlated with improved median overall survival. These 2 SNPs had very low p-values (p=0.0081 and p=0.0097, respectively), but did not reach the multiplicity adjusted p-value threshold.

A Cox regression for rs9582036 (SEQ ID NO. 2) and rs9554316 (SEQ ID NO.1) in the placebo arm failed to reveal a significant correlation with overall survival. Indeed, relative to rs9582036 (SEQ ID NO 2) AA carriers, the hazard ratio was 1.62 (CI=0.97-2.68; p=0.063) and 1.53 (CI=0.75-3.12; p=0.24) for rs9582036 (SEQ ID NO. 2) AC and CC carriers, respectively. Likewise, the hazard ratio for rs9554316 (SEQ ID NO: 1) TG and GG carriers was 1.34 (CI=0.81-2.22; p=0.25) and 1.36 (CI=0.60-3.04; p=0.46) relative to rs9554316 TT carriers. Consequently, a regression analysis assessing the interaction between genotypes and treatment was significant (p=0.023 for rs9582036 (SEQ ID NO. 2) and p=0.041 for rs9554316 (SEQ ID NO. 1)), indicating that rs9582036 (SEQ ID NO. 2) and rs9554316 (SEQ ID NO. 1) were predictive, rather than prognostic markers.

**Analysis in the sub-group of patients treated with bevacizumab, adjusting for ancillary prognostic factors: SNPs in VEGFR-1 are independent predictive markers for treatment**

Overall Survival by Genotype - With covariates - Subset : Bev Arm

| | snp | gene | HR | CI_95 | pvalue | star | adj.pvalue | N/Nevts |
|---|---|---|---|---|---|---|---|---|
| 152 | rs9582036 | VEGFR1 | 1.921 | (1.27, 2.9) | 2.0e-03 | ** | 3.0e-01 | 71/63 |
| 150 | rs9554316 | VEGFR1 | 1.826 | (1.17,2.86) | 8.9e-03 | ** | 1.0e+00 | 71/63 |
| 44 | rs1830792 | VEGFR1 | 1.723 | (1.14,2.61) | 1.6e-02 | * | 1.0e+00 | 69/61 |
| 80 | rs3025035 | VEGF | 0.392 | (0.16,0.95) | 1.7e-02 | * | 1.0e+00 | 68/60 |
| 123 | rs7324547 | VEGFR1 | 1.711 | (1.11,2.64) | 2.1e-02 | * | 1.0e+00 | 71/63 |
| 136 | rs7995976 | VEGFR1 | 1.958 | (1.13, 3.4) | 2.2e-02 | * | 1.0e+00 | 69/61 |

To assess whether rs9582036 and rs9554316 correlated with overall survival independently from other covariates known to affect treatment response, a Cox regression model was performed while adjusting for neutrophil counts, CRP levels and tumor location. Only patients with normal albumin levels were considered in this analysis.

Although the correlation with overall survival was attenuated, it was not suppressed and remained significant for both SNPs (p=0.002 and p=0.0089).

rs9582036 (SEQ ID NO. 2) has the lowest p-value and a p-value well below 0.05. rs9554316 (SEQ ID NO. 1) also has a p-value below 0.05. This further suggests that rs9582036 (SEQ ID NO. 2) and rs9554316 (SEQ ID NO. 1) are associated with overall survival in bevacizumab treated patients independently from other prognostic factors.

**Progression Free Survival**

**Analysis in the sub-group of patients treated with Bevacizumab**

Progression Free Survival by Genotype - No covariates - Subset : Bev Arm

| | snp | gene | HR | CI_95 | pvalue | star | adj.pvalue | N/Nevts |
|---|---|---|---|---|---|---|---|---|
| 152 | rs9582036 | VEGFR1 | 1.889 | (1.32,2.71) | 8.1e-04 | *** | 1.3e-01 | 77/72 |
| 150 | rs9554316 | VEGFR1 | 1.989 | (1.33,2.98) | 1.2e-03 | ** | 1.9e-01 | 77/72 |
| 151 | rs9554320 | VEGFR1 | 1.442 | (1.05,1.99) | 2.7e-02 | * | 1.0e+00 | 75/71 |
| 146 | rs9513070 | VEGFR1 | 1.503 | (1.05,2.16) | 2.8e-02 | * | 1.0e+00 | 74/69 |
| 44 | rs1830792 | VEGFR1 | 1.537 | (1.05,2.25) | 3.6e-02 | * | 1.0e+00 | 75/70 |
| 81 | rs3025039 | VEGF | 1.797 | (1.06,3.05) | 3.9e-02 | * | 1.0e+00 | 77/72 |

We also analyzed whether rs9582036 (SEQ ID NO. 2) and rs9554316 (SEQ ID NO. 1) correlated with progression free survival. rs9582036 (SEQ ID NO.2) and rs9554316 (SEQ ID NO. 1) both have a very low p-value when looking at association with progression free survival in the bevacizumab treated group. This provides evidence that and are not only associated with overall survival but also risk of progression in patients treated with bevacizumab. In the bevacizumab arm, there was a clear and significant correlation with progression free survival. Relative to AA carriers, the hazard ratio for rs9582036 (SEQ ID NO. 2) AC and CC carriers was 1.86 (CI=1.11-3.10; p=0.018) and 3.63 (CI=1.64-8.05; p=0.0015). Such effect was not observed in the placebo arm. A similar situation was observed for the rs9554316 (SEQ ID NO. 1) SNP, showing a significant correlation with PFS in the bevacizumab arm, but not the placebo arm.

**VEGFR-1 SNPs define a locus in the VEGFR-1 tyrosine-kinase domain**

Since two SNPs in VEGFR-1 were significantly associated with bevacizumab-related outcome, and two other SNPs in VEGFR-1 also tended to be associated with outcome, the exact location of these SNPs in the VEGFR-1 gene as well as the haplotypes they represent as tagging SNPs, were studied in more detail. Intriguingly, all 4 SNPs were located close to each other (i.e., in introns 25, 27, 28 and 29) respectively for the rs9554320 (SEQ ID NO. 4), rs9582036 (SEQ ID NO: 2), rs9554316 (SEQ ID NO. 1) and rs9513070 (SEQ ID NO. 3) SNPs and represented 4 consecutive haplotypes in the VEGFR-1 gene. As a consequence of their proximate location, these 4 SNPs were also in significant linkage disequilibrium and did not occur independently from each other.

When considering the p-value of every SNP as a measure of its association with overall survival in the bevacizumab arm, and plotting these values in function of their location in the VEGFR-1 gene, a remarkable association signal encompassing exons 25 to 30 was noticed. Such association signal was not observed in the placebo group. Exon 25 to 30 of the VEGFR-1 gene code for amino acid residue 1029 to 1338, which are part of the receptor tyrosine-kinase (TK) domain in VEGFR-1 that contain two major auto-phosphorylation sites in the VEGFR-1 gene.

**Example 2**

In the context of the invention, genetic variability in underlying signalling pathways was explored in order to discover predictive patterns for anti angiogenic treatment. In this analysis, the correlation of genetic variability in the VEGF signaling pathway with clinical outcomes of patients with metastatic renal cell cancer (mRCC) treated with interferon alpha (IFN) plus bevacizumab or placebo in a phase III trial (AVOREN) was evaluated; see study number BO17705E; Escudier et al., Lancet 370: 2103-2111 (2007). Patients were randomized to receive bevacizumab (Avastin®) 10 mg/kg as an IV infusion every 2 weeks plus interferon alpha (IFN) starting at 9 MIU three times weekly (n=327) or placebo plus IFN (n=322). Patients were treated with bevacizumab (Avastin®) or placebo until disease progression or unacceptable toxicity. IFN was discontinued after a maximum of 52 weeks but bevacizumab (Avastin®) or placebo was continued until after disease progression or unacceptable toxicity.

**Samples and Single Nucleotide Polymorphism Selection**

Germline DNA was available from 110 out of the 649 patients, of which 51 received interferon alpha plus placebo and 59 of which received interferon alpha plus bevacitumab; study number BO17705E; Escudier et al., Lancet 370: 2103-2111 (2007). As in Example 1, the sample from 1 patient of Asian ethnicity was not analysed. The four SNPs identified in Example 1 plus one additional VEGFA SNP (rs699947) were initially assessed.

**Genotyping**

As in Example 1, peripheral blood was sampled in K2EDTA plastic Vacutainer tubes. After centrifugation, germline DNA was extracted from the precipitated leucocyte cell fraction according to standard procedures. Genotyping for the selected SNPs was carried out at the Vesalius Research Center using the Sequenom® iPLEX platform, as described previously by the manufacturer. All the SNPs that failed to provide robust genotypes in a first genotyping round were re-designed using a different set of PCR primers and tested again. In case of repetitive failure in the second design these SNPs were considered as failed. Of the 184 selected SNPs, 27 SNPs (14.7%) failed and 157 (85.3%) were successfully genotyped. The overall genotype success rate was 98.5%. At the individual sample level, all samples were genotyped with a high success rate (i.e., <5% of the samples had a success rate <95%, whereas >70% of the samples had a success rate >99%). No samples were excluded from the analysis and no significant deviations from Hardy-Weinberg were observed.

The amplification primers designed for SNPs rs9582036 (SEQ ID NO. 2), rs9554316 (SEQ ID NO. 1), rs9513070 (SEQ ID NO. 3) and rs9554320 (SEQ ID NO.4) are shown in Figure 2. For rs9554316 (SEQ ID NO. 1), ACGTTGGATGATCGTAAAGACATCATTCG (SEQ ID NO. 25) and ACGTTGGATGTGCTGGAGACCATGACCAAG (SEQ ID NO. 26) were used. For SNP rs9582036 (SEQ ID NO. 2), ACGTTGGATGACTGTGCCCAGCAACAATAG (SEQ ID NO. 20) and ACGTTGGATGGCATAATAGCACTTTACTCC (SEQ ID NO. 21) were used. For rs9513070 (SEQ ID NO. 3), ACGTTGGATGGCAAGCTTGCCCAACTTGTG (SEQ ID NO. 35) and ACGTTGGATGGTTTGTGTTGGGCTGCACTC (SEQ ID NO. 36) were used. For rs9554320 (SEQ ID NO.4), ACGTTGGATGGTGGGAGGCCAGTTTGTAAC (SEQ ID NO. 30) and ACGTTGGATGAGCAAGGTTCCTGTGTGTAG (SEQ ID NO. 31) were used.

The unextended primers (probes) designed for SNPs rs9582036 (SEQ ID NO. 2), rs9554316 (SEQ ID NO. 1), rs9513070 (SEQ ID NO. 3) and rs9554320 (SEQ ID NO.4) are also shown in Figure 2. For rs9554316 (SEQ ID NO. 1), AAGACATCATTCGATTTTTTTTCT (SEQ ID NO. 28) was used. For SNPs rs9582036 (SEQ ID NO. 2), AGCAACAATAGCCTTCTT (SEQ ID NO. 22) was used. For rs9513070 (SEQ ID NO. 3), CGTGTGGCCCACGGGCT (SEQ ID NO. 37) was used. For rs9554320 (SEQ ID NO.4), ACAGCGGCTTTGCAGTGC (SEQ ID NO. 32) was used.

**Statistics**

When the allelic model was used, patients homozygous with the minor allele were coded as 2, heterozygous patients were coded as 1, and patients homozygous for the major allele were coded as 0. An independent, separate model was modelled for each SNP.

The Cox proportional hazard regression model was used to evaluate the effect of different genotypes Progression Free Survival.

In the Cox model, the time from entry in the study until disease progression or death was used as the Progression-Free Survival end-point.

For progression free survival, the null hypothesis stated that the genotype has no effect on risk of progression or survival. The likelihood ratio test was used to calculate a P value corresponding to this hypothesis for each tested SNP.

The primary analysis were conducted in the sub-group of patients in the bevacizumab arm of the trial. Additional analyses were performed in the sub-group of patients in the placebo arm of the trial, and treatment by genotype interaction was evaluated in model including both.

P-value below 0.05 were considered statistically significant. No correction for multiple testing was necessary in a confirmatory analysis of the findings from example 1.

Survival curves and median survival time were calculated using standard Kaplan-Meier estimates.

**Clinical Characteristics Of Genetic Patient Population**

All baseline demographics of the genetics subpopulation were similar to the overall study population.

| | Placebo + IFN N=322 | Placebo + IFN (Genotype population) N=51 | Bevacizumab + IFN N=327 | Bevacizumab + IFN (Genotype population) N=59 |
|---|---|---|---|---|
| Sex | | | | |
| Female | 87 (27%) | 13 (25%) | 105(32%) | 17 (29%) |
| Male | 235(73%) | 38(75%) | 222 (68%) | 42(71%) |
| n | 322 | 51 | 327 | 59 |
| | | | | |
| Age in years | | | | |
| Mean | 59.4 | 60.1 | 60.1 | 59.4 |
| SD | 10.89 | 10.74 | 10.12 | 10.32 |
| SEM | 0.61 | 1.50 | 0.56 | 1.34 |
| Median | 60.0 | 61.0 | 61.0 | 59.0 |
| Min-Max | 18-81 | 38-80 | 30-82 | 36-82 |
| n | 322 | 51 | 327 | 59 |
| | | | | |
| Weight in kg | | | | |
| Mean | 77.52 | 76.86 | 75.99 | 76.36 |
| SD | 14.797 | 13.523 | 15.033 | 13.954 |
| SEM | 0.828 | 1.894 | 0.831 | 1.817 |
| Median | 76.00 | 76.90 | 75.00 | 78.00 |
| Min-Max | 42.0-121.0 | 46.5-118.0 | 40.0-115.0 | 54.0-109.0 |
| n | 319 | 51 | 327 | 59 |
| | | | | |
| Height in cm | | | | |
| Mean | 169.9 | 170.2 | 169.8 | 171.5 |
| SD | 8.84 | 8.82 | 9.07 | 9.24 |
| SEM | 0.50 | 1.25 | 0.50 | 1.20 |
| Median | 170.5 | 171.0 | 170.0 | 173.0 |

(continued)

| | | | | |
|---|---|---|---|---|
| Height in cm | | | | |
| Min-Max | 147-198 | 152-191 | 146-194 | 178-190 |
| n | 314 | 50 | 325 | 59 |
| | | | | |
| Body Surface Area ($m^2$) | | | | |
| Mean | 1.89 | 1.88 | 1.87 | 1.92 |
| SD | 0.199 | 0.196 | 0.207 | 0.198 |
| SEM | 0.011 | 0.028 | 0.011 | 0.026 |
| Median | 1.87 | 1.88 | 1.86 | 1.91 |
| Min-Max | 1.4-2.5 | 1.4-2.5 | 1.3-2.4 | 1.5-2.3 |
| n | 312 | 50 | 325 | 59 |
| | Placebo + IFN N=322 | Placebo + IFN (Genotype population) N=51 | Bevacizumab + IFN N=327 | Bevacizumab + IFN (Genotype population) N=59 |
| Age Category (years) | | | | |
| < 65 | 204 (63%) | 30 (59%) | 206 (63%) | 37 (63%) |
| > = 65 | 118 (37%) | 21 (41 %) | 121 (37%) | 22 (37%) |
| n | 322 | 51 | 327 | 59 |
| | | | | |
| Race | | | | |
| Black | 1 (<1%) | - | 2 (<1%) | - |
| Other | 1 (<1%) | - | 2 (<1%) | - |
| Caucasian/White | 312 (97%) | 51 (100%) | 312 (95%) | 58 (98%) |
| Asian | 8 (2%) | - | 11 (3%) | 1 (2%) |
| n | 322 | 51 | 327 | 59 |
| | | | | |
| Smoking Status at Screening | | | | |
| Never smoked | 148 (46%) | 22 (43%) | 154 (47%) | 30(51%) |
| Past smoker | 129 (40%) | 20 (39%) | 126 (39%) | 23 (39%) |
| Current smoker | 43 (13%) | 9 (18%) | 45 (14%) | 6 (10%) |
| n | 320 | 51 | 325 | 59 |

**Summary of progression free survival**

The progression-free survival Kaplan Meier curves cross at the same time the overall population and the genotype population (Figures 11 and 12). But the genotype population had overall a better clinical outcome than the overall population (see table below, time to event).

| | Placebo + IFN N=322 | Bevacizumab + IFN N=327 | Placebo + IFN (Genotype population) N=51 | Bevacizumab + IFN (Genotype population) N=59 |
|---|---|---|---|---|
| Patientswith events | 298 (92.5%) | 301 (92.0%) | 42 (82.4%) | 56 (94.9%) |
| Patients without events | 24 (7.5%) | 26 (8.0%) | 9 (17.6%) | 3 (5.1%) |
| | | | | |
| Time to event (months) | | | | |
| Median # | 5.5 | 10.2 | 8.7 | 15.5 |
| 95% CI for Median # | [4.2; 5.7] | [7.7; 11.1] | [7.2 ; 14.2] | [13.5 ; 18.4] |
| 25% and 75%-ile | 2.0 ; 10.4 | 3.7 ; 16.6 | 4.0 ; 20.1 | 9.4 ; 21.7 |
| Range | 0.5 to 45.8 | 0.4 to 44.1 | 1.8 to 41.1 | 1.4 to 41.3 |
| p-Value (Log-Rank Test) | 0.0004 | | 0.7345 | |
| p-Value (Wilcoxon Test) | < 0.0001 | | 0.0561 | |
| | | | | |
| Hazard Ratio | 0.75 | | 0.93 | |
| 95% CI | [0.64 ; 0.88] | | [0.62 ; 1.40] | |

SNPs in VEGFR-1 correlated with progression-free survival in the bevacizumab treatment arm.

*Analysis in placebo treated group (progression-free survival, without covariates, S_BEV=0*)

| | snp | gene | HR | CI_95 | pvalue | star | holm_pval | N/Nevts | geno.freq |
|---|---|---|---|---|---|---|---|---|---|
| 1 | rs699947 | VEGF | 0.632 | (0.4, 1) | 4.70e-02 | * | 2.35e-01 | 45/37 | 14/22/9 |
| 5 | rs9582036 | VEGFR1 | 0.768 | (0.5,1.19) | 2.25e-01 | | 8.99e-01 | 45/37 | 21/17/7 |
| 3 | rs9554316 | VEGFR1 | 0.826 | (0.47,1.44) | 4.93e-01 | | 1.00e+00 | 45/37 | 26/17/2 |
| 2 | rs9513070 | VEGFR1 | 1.109 | (0.59,2.08) | 7.48e-01 | | 1.00e+00 | 45/37 | 11/26/8 |
| 4 | rs9554320 | VEGFR1 | 0.977 | (0.64,1.49) | 9.14e-01 | | 1.00e+00 | 45/37 | 14/21/10 |

No genotypes were detected to be associated with progression-free survival in the patients treated with placebo.

*Analysis in bevacizumab treated group (progression-free survival, without covariates, S_BEV=1*)

| | snp | gene | HR | CI_95 | pvalue | star | holm_pval | N/Nevts | geno.freq |
|---|---|---|---|---|---|---|---|---|---|
| 2 | rs9513070 | VEGFR1 | 1.679 | (1.12,2.51) | 1.18e-02 | * | 5.92e-02 | 56/54 | 19/28/9 |

(continued)

| | snp | gene | HR | CI_95 | pvalue | star | holm_pval | N/Nevts | geno.freq |
|---|---|---|---|---|---|---|---|---|---|
| 3 | rs9554316 | VEGFR1 | 1.812 | (1.08,3.05) | 3.26e-02 | * | 1.30e-01 | 55/53 | 36/18/1 |
| 5 | rs9582036 | VEGF1 | 1.262 | (0.85,1.88) | 2.63e-01 | | 7.90e-01 | 56/54 | 30/20/6 |
| 1 | rs699947 | VEGF | 1.178 | (0.81,1.71) | 3.92e-01 | | 7.90-01 | 55/53 | 19/24/12 |
| 4 | rs9554320 | VEGFR1 | 1.115 | (0.76,1.63) | 5.76e-01 | | 7.90e-01 | 52/50 | 20/22/10 |

Two SNPs, rs9513070 (SEQ ID NO: 3) and rs9554316 (SEQ ID NO:1), in the VEGFR-1 gene correlated with progression-free survival in the bevacizumab-treated group only (see Figures 13 to 16). AA carriers of rs9513070 (SEQ ID NO: 3) showed increased progression free survival in comparison to AG and GG carriers in the bevacizumab-treated group (Figure 13). GG carriers of rs9554316 (SEQ ID NO:1) showed increased progression free survival in comparison to GT and TT carriers in the bevacizumab-treated group only (Figure 15).

The application comprises the following items:

1. A method for improving the overall survival of a patient suffering from a malignant disease or a disease involving physiological and pathological angiogenesis by treatment with an angiogenesis inhibitor, said method comprising the following steps:

(a) determining from a patient sample if the patient suffering from a malignant disease or a disease involving physiological and pathological angiogenesis has one or more variant alleles of the VEGFR-1 gene; and
(b) administering an angiogenesis inhibitor to the patient having one or more variant alleles of the VEGFR-1 gene.

2. A method for improving the progression-free survival of a patient suffering from a malignant disease or a disease involving physiological and pathological angiogenesis by treatment with an angiogenesis inhibitor, said method comprising the following steps:

(a) determining from a patient sample if the patient suffering from a malignant disease or a disease involving physiological and pathological angiogenesis has one or more variant alleles of the VEGFR-1 gene; and
(b) administering an angiogenesis inhibitor to the patient having one or more variant alleles of the VEGFR-1 gene.

3. An angiogenesis inhibitor for use in improving overall survival of a patient suffering from a malignant disease or a disease involving physiological and pathological angiogenesis wherein it is determined from a patient sample if the patient suffering from a malignant disease or a disease involving physiological and pathological angiogenesis has one or more variant alleles of the VEGFR-1 gene and whereby the angiogenesis inhibitor is to be administered to said patient having one or more variant alleles of the VEGFR-1 gene.

4. An angiogenesis inhibitor for use in improving progression-free survival of a patient suffering from a malignant disease or a disease involving physiological and pathological angiogenesis wherein it is determined from a patient sample if the patient suffering from a malignant disease or a disease involving physiological and pathological angiogenesis has one or more variant alleles of the VEGFR-1 gene and whereby the angiogenesis inhibitor is to be administered to said patient having one or more variant alleles of the VEGFR-1 gene.

5. An in vitro method for the identification of a responder for or a patient sensitive to an angiogenesis inhibitor, said method comprising determining from a patient sample if a patient suffering from a malignant disease or a disease involving physiological and pathological angiogenesis has one or more variant alleles of the VEGFR-1 gene, whereby the presence of the one or more variant alleles of the VEGFR-1 gene is indicative for a responding patient or is indicative for a sensitivity of the patient to the angiogenesis inhibitor.

6. An angiogenesis inhibitor for use in an improved chemotherapy regimen for a patient suffering from a malignant disease or a disease involving physiological wherein it is determined from a patient sample if a patient suffering from said disease has one or more variant alleles of the VEGFR-1 gene in a patient sample and whereby the angiogenesis inhibitor is to be administered to the patient having one or more variant alleles of the VEGFR-1 gene.

7. The method or angiogenesis inhibitor of any one of 1 to 6, wherein the patient sample is a blood sample.

8. The method or angiogenesis inhibitor of any one of items 1 to 7, wherein the patient sample is a peripheral blood sample.

9. The method or angiogenesis inhibitor of any one of items 1 to 8, wherein the angiogenesis inhibitor is bevacizumab.

10. The method or angiogenesis inhibitor of any one of items 1 to 9, wherein the angiogenesis inhibitor is administered as a co-treatment with a chemotherapeutic agent or chemotherapy regimen.

11. The method or angiogenesis inhibitor of any of items 1 to 10, wherein the patient is being co-treated with one or more agents selected from the group consisting of interferon alpha, 5-fluorouracil, leucovorin, irinotecan, gemcitabine-erlotinib and platinum-based chemotherapeutic agents.

12. The method or angiogenesis inhibitor of item 11, wherein the one or more platinum based chemotherapeutic agents is selected from the group consisting of paclitaxel, carboplatin and oxaliplatin.

13. The method or angiogenesis inhibitor of any of items 1 to 12, wherein the malignant disease is pancreatic cancer, renal cell cancer, colorectal cancer, breast cancer, or lung cancer.

14. The method or angiogenesis inhibitor of any one of items 1 to 12, wherein the malignant disease is pancreatic cancer.

15. The method or angiogenesis inhibitor of item 14, wherein the angiogenesis inhibitor is bevecizumab and the co-therapy is gemcitabine-erlotinib therapy.

16. The method or angiogenesis inhibitor of any one of items 2 and 4 to 11, wherein the malignant disease is renal cell cancer.

17. The method or angiogenesis inhibitor item 16, wherein the angiogenesis inhibitor is bevacizumab and the co-therapy is interferon alpha therapy.

18. The method or angiogenesis inhibitor of any of items 1 to 17, wherein the one or more variant alleles of the VEGFR-1 gene are in the region encompassing the tyrosine-kinase domain of the VEGFR-1 gene.

19. The method or angiogenesis inhibitor of any one of items 1 to 18, wherein the one or more variant alleles of the VEGFR-1 gene are in the region encompassing exons 25 to 30 of the VEGFR-1 gene.

20. The method or angiogenesis inhibitor of any one of items 1 to 19, wherein the one or more variant alleles of the VEGFR-1 gene are in an intron region of the VEGFR-1 gene.

21. The method or angiogenesis inhibitor of item 13, 14 or 15, wherein the one or more variant alleles of the VEGFR-1 gene are selected from the group consisting of rs9554316 (SEQ ID NO. 1), rs9582036 (SEQ ID NO. 2), rs9513070 (SEQ ID NO. 3) and rs9554320 (SEQ ID NO. 4).

22. The method or angiogenesis inhibitor of item 16 or 17, wherein the one or more variant alleles of the VEGFR-1 gene are selected from the group consisting of rs9554316 (SEQ ID NO. 1) and rs9513070 (SEQ ID NO. 3).

23. The method or angiogenesis inhibitor of item 13, 14 or 15, wherein the one or more variant alleles of the VEGFR-1 gene are selected from the group consisting of:

(a) a sequence corresponding to rs9554316 (SEQ ID NO. 1) or having at least 80% homology to rs9554316 (SEQ ID NO. 1) having a substitution, deletion or addition of at least one nucleotide corresponding to position 51 of rs9554316 (SEQ ID NO. 1);
(b) a sequence corresponding to rs9582036 (SEQ ID NO. 2) or having at least 80% homology to rs9582036 (SEQ ID NO. 2) having a substitution, deletion or addition of at least one nucleotide corresponding to position 51 of rs9582036 (SEQ ID NO. 2);
(c) a sequence corresponding to rs9513070 (SEQ ID NO. 3) or having at least 80% homology to rs9513070 (SEQ ID NO. 3) having a substitution, deletion or addition of at least one nucleotide corresponding to position 51 of rs9513070 (SEQ ID NO. 3); and
(d) a sequence corresponding to rs9554320 (SEQ ID NO. 4) or having at least 80% homology to rs9554320 (SEQ ID NO. 4) having a substitution, deletion or addition of at least one nucleotide corresponding to position 51 of rs9554320 (SEQ ID NO. 4).

24. The method or angiogenesis inhibitor of item 16 or 17, wherein the one or more variant alleles of the VEGFR-1 gene are selected from the group consisting of:

(a) a sequence corresponding to rs9554316 (SEQ ID NO. 1) or having at least 80% homology to rs9554316 (SEQ ID NO. 1) having a substitution, deletion or addition of at least one nucleotide corresponding to position 51 of rs9554316 (SEQ ID NO. 1); and
(b) a sequence corresponding to rs9513070 (SEQ ID NO. 3) or having at least 80% homology to rs9513070 (SEQ ID NO. 3) having a substitution, deletion or addition of at least one nucleotide corresponding to position 51 of rs9513070 (SEQ ID NO. 3).

25. The method or angiogenesis inhibitor of item 13, 14 or 15, wherein the one or more variant alleles of the VEGFR-1 gene are selected from the group consisting of:

(a) a sequence corresponding to rs9554316 (SEQ ID NO. 1) or having at least 80% homology to rs9554316 (SEQ ID NO. 1), wherein position 51 is a T or G and having a substitution, deletion or addition of at least one nucleotide at any position from position 1 to 50 and 52 to 101;
(b) a sequence corresponding to rs9582036 (SEQ ID NO. 2) or having at least 80% homology to rs9582036 (SEQ ID NO. 2) wherein position 51 is a C or A and having a substitution, deletion or addition of at least one nucleotide at any position from position 1 to 50 and 52 to 101;
(c) a sequence corresponding to rs9513070 (SEQ ID NO. 3) or having at least 80% homology to rs9513070 (SEQ ID NO. 3) wherein position 51 is a G or A and having a substitution, deletion or addition of at least one nucleotide at any position from position 1 to 50 and 52 to 101; and
(d) a sequence corresponding to rs9554320 (SEQ ID NO. 4) or having at least 80% homology to rs9554320 (SEQ ID NO. 4) wherein position 51 is a C or A and having a substitution, deletion or addition of at least one nucleotide at any position from position 1 to 50 and 52 to 101.

26. The method or angiogenesis inhibitor of item 16 or 17, wherein the one or more variant alleles of the VEGFR-1 gene are selected from the group consisting of:

(a) a sequence corresponding to rs9554316 (SEQ ID NO. 1) or having at least 80% homology to rs9554316 (SEQ ID NO. 1), wherein position 51 is a T or G and having a substitution, deletion or addition of at least one nucleotide at any position from position 1 to 50 and 52 to 101; and
(b) a sequence corresponding to rs9513070 (SEQ ID NO. 3) or having at least 80% homology to rs9513070 (SEQ ID NO. 3) wherein position 51 is a G or A and having a substitution, deletion or addition of at least one nucleotide at any position from position 1 to 50 and 52 to 101.

27. The method or angiogenesis inhibitor of item 13, 14 or 15, wherein the one or more variant alleles of the VEGFR-1 gene are selected from the group consisting of rs9554316 (SEQ ID NO. 1), wherein position 51 is a G, rs9582036 (SEQ ID NO. 2), wherein position 51 is an A, rs9513070 (SEQ ID NO. 3), wherein position 51 is an A, and rs9554320 (SEQ ID NO.4), wherein position 51 is a C.

28. The method or angiogenesis inhibitor of item 16 or 17, wherein the one or more variant alleles of the VEGFR-1 gene are selected from the group consisting of rs9554316 (SEQ ID NO. 1), wherein position 51 is a G, and rs9513070 (SEQ ID NO. 3), wherein position 51 is an A.

29. The method or angiogenesis inhibitor of item 13, 14 or 15, wherein the one or more variant alleles of the VEGFR-1 gene are selected from the group consisting of rs9554316 (SEQ ID NO. 1) and rs9582036 (SEQ ID NO. 2).

30. The method or angiogenesis inhibitor of item 29, wherein wherein the one or more variant alleles of the VEGFR-1 gene are selected from the group consisting of rs9554316 (SEQ ID NO. 1), wherein position 51 is a G, and rs9582036 (SEQ ID NO. 2), wherein position 51 is an A.

31. The method or angiogenesis inhibitor of any one of items 1 to 17, wherein the wherein the one or more variant alleles of the VEGFR-1 gene are selected from the group consisting of rs17619037 (SEQ ID NO. 5), rs9579177 (SEQ ID NO. 6), rs9579176 (SEQ ID NO. 7), rs9554319 (SEQ ID NO. 8), rs7996030 (SEQ ID NO. 9), rs9513075 (SEQ ID NO. 10), rs7993418 (SEQ ID NO. 11), rs9513071 (SEQ ID NO. 12), rs12429309 (SEQ ID NO. 15), rs7982251 (SEQ ID NO 16), rs9508016 (SEQ ID NO. 17), rs7982957 (SEQ ID NO. 18), rs3794400 (SEQ ID NO. 19), rs3794395 (SEQ ID NO. 52), rs9554317 (SEQ ID NO. 53), rs9513073 (SEQ ID NO. 54), rs9513074 (SEQ ID NO. 55), rs9508015 (SEQ ID NO. 56), rs2011950 (SEQ ID NO. 57), rs9513110 (SEQ ID NO. 58), rs9513112 (SEQ ID NO. 59), rs9513113 (SEQ ID NO. 60), rs9551471 (SEQ ID NO. 61), rs2296285 (SEQ ID NO. 62), rs9513116 (SEQ ID NO. 63), rs9551473 (SEQ ID NO. 64), rs7330109 (SEQ ID NO. 65), rs9508037 (SEQ ID NO. 66), rs1924981 (SEQ ID NO. 67), rs34140996 (SEQ ID NO. 68), rs7985584 (SEQ ID NO. 69), rs7992940 (SEQ ID NO. 70) and rs718273 (SEQ ID NO. 71).

32. The method or angiogenesis inhibitor of any one of item 1 to 20 and 31, further comprising determining if the patient has one or more variant alleles selected from the group consisting of rs45455097 (SEQ ID NO. 40), rs1886233 (SEQ ID NO. 41), rs9554309 (SEQ ID NO. 42), rs11619230 (SEQ ID NO. 43), rs9554311 (SEQ ID NO. 44), rs11620238 (SEQ ID NO. 13), rs17618631 (SEQ ID NO. 14), rs4771233 (SEQ ID NO. 45), rs6491274 (SEQ ID NO. 46), rs7982639 (SEQ ID NO. 47), rs12877718 (SEQ ID NO. 48), rs10507382 (SEQ ID NO. 49), rs57354941 (SEQ ID NO. 50) and rs17086497 (SEQ ID NO. 51).

33. The method or angiogenesis inhibitor of any of items 1 to 7, wherein the disease involving physiological and pathological angiogenesis is high grade glioma, glioblastoma, M. Rendu-Osler, von-Hippel-Lindau diseases, hemangiomas, psoriasis, Kaposi's sarcoma, ocular neovascularisation, rheumatoid arthritis, endometriosis, atherosclerosis, myochardial ischemia, peripheral ischemia, cerebral ischemia or wound healing

34. Use of specific primers and/or probes for the preparation of a composition for predicting the response to or sensitivity to bevacizumab therapy of a patient suffering or suspected to suffer from metastatic pancreatic cancer wherein the primers and/or probes are capable of detecting at least one of the variant alleles selected from the group consisting of rs9554316 (SEQ ID NO. 1), SNPs rs9582036 (SEQ ID NO. 2), rs9513070 (SEQ ID NO. 3) and rs9554320 (SEQ ID NO. 4), whereby a G at position 51 of rs9554316 (SEQ ID NO. 1), an A at position 51 of SNPs rs9582036 (SEQ ID NO. 2), an A at position 51 of rs9513070 (SEQ ID NO. 3) and/or a C at position 51 of rs9554320 (SEQ ID NO.4) is indicative of a patient response or sensitive to bevacizumab therapy.

35. Use of specific primers and/or probes for the preparation of a composition for predicting the response to or sensitivity to bevacizumab therapy of a patient suffering from or suspected to suffer from renal cell cancer wherein the primers and/or probes are capable of detecting at least one of the variant alleles are selected from the group consisting of at least one of the variant alleles selected from the group consisting of SNPs rs9554316 (SEQ ID NO. 1) and rs9513070 (SEQ ID NO. 3) whereby a G at position 51 of rs9554316 (SEQ ID NO. 1) and/or an A at position 51 of rs9513070 (SEQ ID NO. 3) is indicative of a patient response or sensitive to bevacizumab therapy.

36. A kit useful for carrying out the method, angiogenesis inhibitor or use of any of items 1 to 35, comprising oligonucleotides or polynucleotides capable of determining the presence of at least one or more variant alleles of the VEGF-R1 gene.

37. Use of an oligonucleotides or polynucleotides for the detection of the one or more variant alleles of VEGFR-1 of the method, angiogenesis inhibitor or use of any one of items 1 to 35.

38. The kit of item 36 or use of item 37, wherein said oligonucleotides or polynucleotides comprise primers and/or probes.

39. The kit of item 38 or the use of items 34, 35 or 38, wherein the primers are selected from the group consisting of SEQ ID NO. 20, SEQ ID NO. 25, SEQ ID NO. 30, SEQ ID NO. 35, SEQ ID NO. 21, SEQ ID NO. 26, SEQ ID NO. 31 and SEQ ID NO. 36.

40. The kit of item 38 or the use of item 34, 35 or 38, wherein the probes are selected form the group consisting of SEQ ID NO. 22, SEQ ID NO. 27, SEQ ID NO. 32 and SEQ ID NO. 37.

41. The kit of item 38 or the use of any one of items 34, 35 or 38, wherein the probes are selected from the group consisting of SEQ ID NO. 23, SEQ ID NO. 24, SEQ ID NO. 28, SEQ ID NO. 29, SEQ ID NO. 33, SEQ ID NO. 34, SEQ ID NO. 38 and SEQ ID NO. 39.

## Claims

1. Bevacizumab for use in improving overall survival of a patient suffering from cancer wherein it is determined from a patient sample if the patient suffering from said cancer has the variant allele of the VEGFR-1 gene corresponding to rs9513070 (SEQ ID NO. 3), wherein position 51 is A, and whereby bevacizumab is to be administered to said patient having said variant allele of the VEGFR-1 gene.

2. Bevacizumab for use in improving progression-free survival of a patient suffering from cancer wherein it is determined from a patient sample if the patient suffering from said cancer has the variant alleles of the VEGFR-1 gene corresponding to rs9513070 (SEQ ID NO. 3), wherein position 51 is A, and whereby bevacizumab is to be administered to said patient having said variant allele of the VEGFR-1 gene.

3. An in vitro method for the identification of a responder for or a patient sensitive to bevacizumab, said method comprising determining from a patient sample if a patient suffering cancer has the variant allele of the VEGFR-1 gene corresponding to rs9513070 (SEQ ID NO. 3), wherein position 51 is A, whereby the presence of said variant allele of the VEGFR-1 gene is indicative for a responding patient or is indicative for a sensitivity of the patient to bevacizumab.

4. Bevacizumab for use in a chemotherapy regimen for a patient suffering from cancer wherein it is determined from a patient sample if a patient suffering from said cancer has the variant allele of the VEGFR-1 gene corresponding to rs9513070 (SEQ ID NO. 3), wherein position 51 is A,whereby bevacizumab is to be administered to the patient having said variant allele of the VEGFR-1 gene.

5. Bevacizumab for the use according to any one of claims 1, 2, and 4 or the *in vitro* method according to claim 3, wherein the patient sample is a blood sample.

6. Bevacizumab for the use of according to any one of claims 1, 2, 4, and 5 or the *in vitro* method according to claim 3 or 5, wherein bevacizumab is administered as a co-treatment with a chemotherapeutic agent or chemotherapy regimen.

7. Bevacizumab for the use according to any one of claims 1, 2, and 4 to 6 or the *in vitro* method according to any one of claims 3, 5, and 6, wherein the patient is being co-treated with one or more agents selected from the group consisting of interferon alpha, 5-fluorouracil, leucovorin, irinotecan, gemcitabine-erlotinib and platinum-based chemotherapeutic agents.

8. Bevacizumab for the use according to any one of claims 1, 2, and 4 to 7 or the *in vitro* method according to any one of claims 3 and 5 to 7, wherein the cancer is pancreatic cancer.

9. Bevacizumab for the use according to claim 8 or the *in vitro* method according to claim 8, wherein the co-therapy is gemcitabine-erlotinib therapy.

10. Bevacizumab for the use according to any one of claims 2 and 4 to 7 or the *in vitro* method according to any one of claims 3 and 5 to 7, wherein the cancer is renal cell cancer.

11. Bevacizumab for the use according to claim 10 or the *in vitro* method according to claim 10, wherein the co-therapy is interferon alpha therapy.

12. Bevacizumab for use according to claim 8 or 9 or the *in vitro* method according to claim 8 or 9 further comprising determining if the patient has more variant alleles of the VEGFR-1 gene selected from the group consisting of:

(a) rs9554316 (SE ID NO. 1), wherein position 51 is G;
(b) rs9582036 (SEQ ID NO. 2), wherein position 51 is A; and
(c) rs9554320 (SEQ ID NO. 4), wherein position 51 is C.

13. Bevacizumab for use according to claim 10 or 11 or the *in vitro* method according to claim 10 or 11 further comprising determining if the patient has the variant allele of the VEGFR-1 corresponding to rs9554316 (SE ID NO. 1), wherein position 51 is G.

14. Use of primers and/or probes for predicting the response to or sensitivity to bevacizumab therapy of a patient suffering or suspected to suffer from metastatic pancreatic cancer or renal cell cancer wherein the primers and/or probes are capable of detecting the variant allele of rs9513070 (SEQ ID NO. 3), whereby an A at position 51 of rs9513070 (SEQ ID NO. 3) is indicative of a patient responsive to or sensitive to bevacizumab therapy.

15. Use of a kit for carrying out the *in vitro* method according to any one of claims 3 and 5 to 13, comprising primers or probes capable of determining the presence of the variant allele of the VEGFR-1 gene corresponding to rs9513070 (SEQ ID NO. 3).

16. The use of claim 15, wherein

(a) the primers are selected from the group consisting of SEQ ID NO. 35 and SEQ ID NO. 36; and
(b) the probes are selected from the group consisting of SEQ ID NO. 37, SEQ ID NO. 38, and SEQ ID NO. 39.

Figure 1A

**SNPs genotyped in AVITA and associated with bevacizumab outcome**

| | SNP_ID | Rare diagnostic allele | SEQUENCE |
|---|---|---|---|
| SEQ ID NO:1 | rs9554316 | T | ATAGACACAAATTCTAAGAAATCGTAAAGACATCATTCGATTTTTTTTCT**[T/G]**GAACTCAATG GAATTCTCAGCCCCCAGCCCTTGGTCATGGTCTCCAGCAA |
| SEQ ID NO:2 | rs9582036 | C | TGCTGCGATTACAGGCACGAGCCACTGTGCCCAGCAACAATAGCCTTCTT**[C/A]**CAAAATGT ATACTAAAGTATTTAGGAGTAAAGTGCTATTATGCCTTAAGC |
| SEQ ID NO:3 | rs9513070 | G | TTGCTTTTTATACTATAATGCAAGCTTGCCCAACTTGTGGCCCACGGGCT**[G/A]**CATGAATCC CAGGATGGCTTTGAGTGCAGCCCAACACAAACTCGTAAACT |
| SEQ ID NO:4 | rs9554320 | A | TTTCCGGGGCTGTGAGCAAGGTTCCTGTGTGTAGCTGATCATTCTCCCCC**[A/C]**GCACTGCA AAGCCGCTGTTACAAACTGGCCTCCCACCACTCAAGGCACAC |

Figure 1B

**SNPs belonging to the rs9554316 and rs9582036 haplotype block (within the locus) but not genotyped within AVITA**

| | SNP_ID | Rare diagnostic allele | SEQUENCE |
|---|---|---|---|
| SEQ ID NO:5 | rs17619037 | G | AGTGCAAAACATCCACAAAGCCCCAGGAAAAAAAGTGTCCAAGACAGGCA**[A/G]**GCAAGTG CAATTTGCACAGTGGAGAAAGCCTCACATGGACACCCGAAAAG |
| SEQ ID NO:6 | rs9579177 | G | ACATTACTGGCGTTGGTGTTTGGAAGGGATCTGAAGAAGGGGTCTATCGG**[G/A]**GTGCACT AGGTACCTTAACTTCACGACTTACATCAAACATGGAGGTGGCA |
| SEQ ID NO:7 | rs9579176 | G | CCCCTTAGCCCCTCAACCCCACATCACAGGGCTTTAGAGAGAAGAGCTGG**[G/A]**AGACCTCT TGTTTGCCAGGCTATACTGGTTAGCTTCTCCCATTGTGATTG |
| SEQ ID NO:8 | rs9554319 | C | CTTCTTCCAAAATGTATACTAAAGTATTTAGGAGTAAAGTGCTATTATGC**[C/T]**TTAAGCCTC CTTTTAAATGGTTTGGCAAAAAGAAAAATCCGTAACAAACA |
| SEQ ID NO:9 | rs7996030 | A | CTCCCAGAGGGACCTACTCTGGTCCGTTGTGAAATCAGAGAATGCCAGAC**[A/G]**TAGGAGA GACTGGAGGCATCATCTCGTCTGAGTGCCCAATGGCACAGGAG |
| SEQ ID NO:10 | rs9513075 | G | GAGGACACCACGCACCAGTGACAAGAACAGGTCCCCTCCCGCTTCCCTAC**[G/A]**CTCAGGC AGAGCTCCCCAGCCCTGCTGTTGGGCCTGAGTCTCCTGTCCAC |
| SEQ ID NO:11 | rs7993418 | G | TCTTCAAAGGTTTTGATTCTTTCCAGGCTCATGAACTTGAAAGCATTTAC**[G/A]**TATCTAATGA AGAAACAGAAAGAATTATCAAGACAGGAAAAGGCATCCAG |
| SEQ ID NO:12 | rs9513071 | G | TTACTTGTGCTGGGAGATGGCGGTTGCTGGGGAAAAAACCTGAGAAACCC**[T/G]**AGTTCAT ATCCTCCCTCTGACACTTCTTAGAGGTATGCCTTTAGGGGGCG |

Figure 1C

**SNPs 5' up or downstream of the haplotype block (outside the locus) and in LD (D'/LOD>80) with rs9554316 or rs9582036 but not typed in AVITA**

| | SNP_ID | Rare diagnostic allele | SEQUENCE |
|---|---|---|---|
| SEQ ID NO:13 | rs11620238 | G | AGGAAGTGCATGATCTTTTCTGACCTGGCCTCCAAGCCATGCAGTGTCCC**[T/G]**TCTTTC ACATTGCATTGATCACAAGTGAGTCCTAAAGCTAGCTCTGATTC |
| SEQ ID NO:14 | rs17618631 | G | CTTCAAGTCATTTGCCTGAATGAATGATTTTATTCTTATGTTATGAAACC**[C/G]**ATAATAC TTTTTAAAGGATTTCAGATTCTTGAAGACAAAATGCCTCTTGC |
| SEQ ID NO:15 | rs12429309 | C | GCAGTGTATGAGGGTTCCAATTTCTCCATATCCTCGCCACACTGACTTCA**[T/C]**CTAGCT ATCCTCATGGGCGTAAAGTGGTATCTTATGGTAATTTCTTTCCC |
| SEQ ID NO:16 | rs7982251 | C | GTGGCTGTCTGCAGGGACATGCTCTGCATTCAGAGAGAACACTGGGGAGA**[C/T]**GACAC GTTTCTCTTTTTGTGAAACATGCTCCCGTAGTGATTACTAAATGA |
| SEQ ID NO:17 | rs9508016 | A | AGGGAGGACATGGGAGGTCATCCACAAAAGTGAAAAATAACACTATGTCT**[A/G]**AAAAC AAGCAGGGAAGAGAAGCCAGTACGAGCCAGCGGGGAGATGCTAGG |
| SEQ ID NO:18 | rs7982957 | A | AGAACACTGGGGAGACGACACGTTTCTCTTTTTGTGAAACATGCTCCCGT**[A/T]**GTGATT ACTAAATGACATTCCTCCCAGAGCATGCAACACGGGCAGAGCTA |
| SEQ ID NO:19 | rs3794400 | T | AGAAGGACAGAAATACGTTTGAGGAGGGGAGAGGCAAAAGCCAGCCTCCC**[T/C]**GCCT CTTAACCAGACCCTTGCGAGTAGAGCGGCTTCTGGTTACTGTCACA |

Linkage disequilibrium was defined here as having a D'/LOD>80

Figure 1D

**SNPs linked with $r^2$ equal or larger than 0.12 to rs9554316 (SEQ ID NO. 1), rs9582036 (SEQ ID NO. 2), rs9513070 (SEQ ID NO. 3) or rs9554320 (SEQ ID NO.4)**

| | SNP_ID | Rare diagnostic allele | SEQUENCE |
|---|---|---|---|
| SEQ ID NO: 40 | rs45455097 | C | TGTGTTTTTTTAAAATTTTTTAAAAACCAATTTCTTAATACTTTACATTT[**T/C**]ATTATTAATATTT ATACTTCTAAGTATTTTTCCTAAGGAAAAAGTACTTC |
| SEQ ID NO: 41 | rs1886233 | A | TACTGGGAGAAAAATTATGGAGCATCAACTGGGTAAACTATAATACAAAC[**A/G**]AACGTTAAG CGTGAAGGGATAGAAACATAGGGCAGTGTTTGTGTAAAAAT |
| SEQ ID NO: 42 | rs9554309 | C | TAAATAATTTCATAGAAGTTCAGTATAGTACGTAATTCTTGTAAGAGTAT[**C/T**]GTATGCAAGC TCTCTGTATGCCACCCACTGTTAGTTCAAATTGAGATTTT |
| SEQ ID NO: 43 | rs11619230 | T | TGTATGCCACCCACTGTTAGTTCAAATTGAGATTTTTGTTTTGTTTTGTT[**C/T**]TTAAGAACATA CCAAGAAAATACCAGTGAATTGTTGGATATGAATTCCCT |
| SEQ ID NO: 44 | rs9554311 | C | AAATTGAGATTTTTGTTTTGTTTTGTTCTTAAGAACATACCAAGAAAATA[**C/T**]CAGTGAATTG TTGGATATGAATTCCCTGTTAGTTGATTTAAATCCTTTCT |
| SEQ ID NO: 45 | rs4771233 | T | GAGCCCAGAGTTTTGAGACAGGCCTGGGCAACATAGTGAGACCTTGTCTC[**T/C**]ACAAAAAG TAAAATCAGCCAGGCATGGTGTCACGCACCTGTAGCCCCAGC |
| SEQ ID NO: 46 | rs6491274 | C | GGAGCACCTTCACATGGCCTCCATGTGGCTTAGGTTTCTCCAAGTATGGC[**T/C**]GCTGGGTC CCTGGCGGGAACTGTTGAAGAGTGAGTGTACCAAGACTCAGG |
| SEQ ID NO: 47 | rs7982639 | G | TTCAAAAGTCAGGGAAAAGAGGTAAAACCAGCAAAAGAGACAGGTAGCCT[**A/G**]TGAGATGG GAAGAAGAAAGTATCATGTCCTGGAACCAAACTGAAGTTGTT |
| SEQ ID NO: 48 | rs12877718 | C | ATAAGTGTGTTCCTTTAAAGCACTTTAAATAGTTGCTGGCACATATTGGG[**T/C**]ACTCAAATGA TAGCAATGATAGCAATTACTAGCTATCACTGCTTCCCAAA |
| SEQ ID NO: 49 | rs10507382 | C | TGGGAAAAAGAAGGATAATATAGGTATGTTTCAAAACAAATATATTTTAG[**T/C**]CTGAGATTAA GCCCTTCCACCTTTTAAAATGGGTTTTCTCCTATGAAAAG |
| SEQ ID NO: 50 | rs57354941 | T | AACATTAGTAGCATCTGGTTGGATTTTGGGTTTTTTTTTTCCCCCTCCCT[**C/T**]AAGTGTTCTT AGCTCGCAGAATAGAAGAATGGCCATCCCTGTGTTGGGAT |
| SEQ ID NO: 51 | rs17086497 | A | AGTTTCCAGAGCCATGAGAACATTTCTAAACTGACGTGACATTGATTCCT[**C/A**]AAGCATTCTT CAAGTCATTTGCCTGAATGAATGATTTTATTCTTATGTTA |
| SEQ ID NO: 52 | rs3794395 | T | TGGCATGGCTACACGGCTAAATATTATTTAATGGCATAATCGAGTGCTAG[**C/T**]GCCAGGCTT GTATTTTGTGTGTGTGTGTGATTACAGATTGGGAGAATTGG |
| SEQ ID NO: 53 | rs9554317 | T | CCATCTCGGCCTCCCAGAGTGCTGGGATTACAGGCGTGAGCCACTGTGCC[**C/T**]GGCCGCAA GCAGTATTTTCTGACTGCTGCAGCTTCTTCCCATATCTTCCC |
| SEQ ID NO: 54 | rs9513073 | G | TATGTAAAGACTTGAAGGTTTGAGAAAGTCCAAAGCAGTTTTTGGTCCAA[**A/G**]GCTGATGTG ACTTTGTACCTCTGAGTTATTTGCGACCACAGCCTGGGTAT |

**SNPs linked with $r^2$ equal or larger than 0.12 to rs9554316 (SEQ ID NO. 1), rs9582036 (SEQ ID NO. 2), rs9513070 (SEQ ID NO. 3) or rs9554320 (SEQ ID NO.4)**

| | SNP_ID | Rare diagnostic allele | SEQUENCE |
|---|---|---|---|
| SEQ ID NO: 55 | rs9513074 | T | ATGCTTAAAATAGTGGCACTTATAAGCACCAAATAACTATTTAATCAGTA[**C/T**]TATTCAATACTGTTATAATAAAAACTAATTTCAGGCCAGGCATGGAGGCT |
| SEQ ID NO: 56 | rs9508015 | T | TAGTGGCACTTATAAGCACCAAATAACTATTTAATCAGTACTATTCAATA[**C/T**]TGTTATAATAAAAACTAATTTCAGGCCAGGCATGGAGGCTCACGCCTGTA |
| SEQ ID NO: 57 | rs2011950 | G | CTGCAATCTCCGCTTCATAGGCTCAAGTGATTCTCATGCCTCAGCCTCCC[**A/G**]AGTAGCTGCCACCAGGCCCGGCTAACTTTTGTATTTTTAGTAGAGACGGG |
| SEQ ID NO: 58 | rs9513110 | G | AACAGATCTTTGTTTTAGAAAGAAGAAAAGACAATCCAGAAACAACCAGG[**T/G**]GGACTCTAGACACCCATGGAGGAGGTGATCAGAGCTCATGAGAGTACCAG |
| SEQ ID NO: 59 | rs9513112 | A | TAGCTCTATAGTAGGCAGAAATATCCTTTAAGTTCATTAAAGGTTCTCAG[**G/A**]TCTTATTTAAAGAAATGTACCCCAATGCTAGCAATGATTACAACCTTGCA |
| SEQ ID NO: 60 | rs9513113 | T | CGTTACACGTTTTTGTTGCTAAGTTATTCCATTTCCATGGCGAGAAATGT[**C/T**]GATGAAAAACAACACTAGCTTGAGGCTGATGCATTAATCTTTCATACATA |
| SEQ ID NO: 61 | rs9551471 | G | ACAATCCCTTTTCTTCCACCTAGAATCATGCAATAGAAATACCAGTCTAC[**A/G**]CTCTTCAAAACAAAACCCCCAGACATCAAAAGACAGTATCTATGCATATC |
| SEQ ID NO: 62 | rs2296285 | A | CCAGTTGAAAAAGGGGGAAAAAAATCAAATTACCTTCCCCCAACAGATTA[**T/A**]AAAGGAAATACCAAACAGCCACAAAGAAGCTCGAAACCTTTTCTATCGCC |
| SEQ ID NO: 63 | rs9513116 | A | CTCTAAAAGCCAATACCAATTTCAGCACTGGGTCCACTGAAATTCTATCC[**G/A**]AAGATATCAGAACTTCAGTGATTTGTGTCCCTCCAAACACTATGCAAAAC |
| SEQ ID NO: 64 | rs9551473 | G | ACATATAAAAAGAATTATATACAATGCCCAAGTGGGATTTTTTTCAGGAA[**T/G**]GCAAGATTGGTTTAACATCCAAAAACCAATGTAATTACTATATCATGTCA |
| SEQ ID NO: 65 | rs7330109 | T | TAAAATGGCAGAAGAAGGATTTGACAAAAATCCAAGCTCCTTTCGTGATG[**A/T**]AAACACTCAGGAAAATGGGAATTGCACTGAATTTCCTCAACCTAATAAAT |
| SEQ ID NO: 66 | rs9508037 | G | AGAAATTAAAGAAGATCTACTAAGTGGGAAACATCTCATGTTCATGGATT[**A/G**]GAAGACTTAATATTGTTAAGATGTTAATGTCCCCCAAATTGATCTACAGA |
| SEQ ID NO: 67 | rs1924981 | T | ATACATGAAAGAACCTCAAAAATATTATGCTGAGTGAAAGAAATTAGTCA[**C/T**]AAAAGACTACAAATTATGTATTCTATTTATATAAAATATCCATGAGACAA |
| SEQ ID NO: 68 | rs34140996 | G | ATAAGGGGTGATAGCTAAAGGCTATGGAGTTTATTTTGAGACAATGACAT[**A/G**]TTCTAAAATTGACCGTGATGATGATGGTTGCATATATCTGTCAATATGCT |
| SEQ ID NO: 69 | rs7985584 | A | TGTCAATATGCTACAAACCACTGAATCATCATTTTAAATGGGTGTGGTAT[**G/A**]TAAATTATATCTCAATAAAACTTATTTTGAATTAAAACAGAGGTTTAGTC |
| SEQ ID NO: 70 | rs7992940 | G | AATAAGAAGTGCCTCTTTCTAAAAAAGAGCTCTTTGCCTACTACCTGCCT[**T/G**]TCCTCAGAAGAAAGACATTGCTTTTTGTTTTGTTTTGTTTTGTTTGTTTT |

**SNPs linked with $r^2$ equal or larger than 0.12 to rs9554316 (SEQ ID NO. 1), rs9582036 (SEQ ID NO. 2), rs9513070 (SEQ ID NO. 3) or rs9554320 (SEQ ID NO.4)**

| | SNP_ID | Rare diagnostic allele | SEQUENCE |
|---|---|---|---|
| SEQ ID NO: 71 | rs718273 | T | CCTTGAAGCTATTACTGACCAATCAGAATACTATCACTAAGGATCTCTCT[**C/T**]GCCTCCTTCCTCCCCTCATCCCTCAGTCTCTCTTCTCCTTCTCCCTCTTC |

Figure 2

| | SNP_ID | 2nd-PCR PRIMER SEQ | |
|---|---|---|---|
| SEQ ID NO:2 | rs9582036 | ACGTTGGATGACTGTGCCCAGCAACAATAG | SEQ ID NO:20 |
| SEQ ID NO:1 | rs9554316 | ACGTTGGATGATCGTAAAGACATCATTCG | SEQ ID NO:25 |
| SEQ ID NO:4 | rs9554320 | ACGTTGGATGGTGGGAGGCCAGTTTGTAAC | SEQ ID NO:30 |
| SEQ ID NO:3 | rs9513070 | ACGTTGGATGGCAAGCTTGCCCAACTTGTG | SEQ ID NO:35 |

| | SNP_ID | 1st-PCR PRIMER SEQ | |
|---|---|---|---|
| SEQ ID NO:2 | rs9582036 | ACGTTGGATGGCATAATAGCACTTTACTCC | SEQ ID NO:21 |
| SEQ ID NO:1 | rs9554316 | ACGTTGGATGTGCTGGAGACCATGACCAAG | SEQ ID NO:26 |
| SEQ ID NO:4 | rs9554320 | ACGTTGGATGAGCAAGGTTCCTGTGTGTAG | SEQ ID NO:31 |
| SEQ ID NO:3 | rs9513070 | ACGTTGGATGGTTTGTGTTGGGCTGCACTC | SEQ ID NO:36 |

| | SNP_ID | AL | MT | UEP_MASS | UEP_SEQ | |
|---|---|---|---|---|---|---|
| SEQ ID NO:2 | rs9582036 | 91 | 46,9 | 5442,6 | AGCAACAATAGCCTTCTT | SEQ ID NO:22 |
| SEQ ID NO:1 | rs9554316 | 100 | 48,2 | 7282,8 | AAGACATCATTCGATTTTTTTTCT | SEQ ID NO:27 |
| SEQ ID NO:4 | rs9554320 | 93 | 98,4 | 5515,6 | ACAGCGGCTTTGCAGTGC | SEQ ID NO:32 |
| SEQ ID NO:3 | rs9513070 | 93 | 97 | 5203,4 | CGTGTGGCCCACGGGCT | SEQ ID NO:37 |

**Figure 2 (continued)**

| | SNP_ID | EXT1_CALL | EXT1_MASS | EXT1_SEQ | |
|---|---|---|---|---|---|
| SEQ ID NO:2 | rs9582036 | C | 5689,8 | AGCAACAATAGCCTTCTTC | SEQ ID NO:23 |
| SEQ ID NO:1 | rs9554316 | G | 7570 | AAGACATCATTCGATTTTTTTTCTG | SEQ ID NO:28 |
| SEQ ID NO:4 | rs9554320 | C | 5802,8 | ACAGCGGCTTTGCAGTGCG | SEQ ID NO:33 |
| SEQ ID NO:3 | rs9513070 | A | 5474,6 | CGTGTGGCCCACGGGCTA | SEQ ID NO:38 |

| | SNP_ID | EXT2_CALL | EXT2_MASS | EXT2_SEQ | |
|---|---|---|---|---|---|
| SEQ ID NO:2 | rs9582036 | A | 5713,8 | AGCAACAATAGCCTTCTTA | SEQ ID NO:24 |
| SEQ ID NO:1 | rs9554316 | T | 7609,9 | AAGACATCATTCGATTTTTTTTCTT | SEQ ID NO:29 |
| SEQ ID NO:4 | rs9554320 | A | 5842,7 | ACAGCGGCTTTGCAGTGCT | SEQ ID NO:34 |
| SEQ ID NO:3 | rs9513070 | G | 5490,6 | CGTGTGGCCCACGGGCTG | SEQ ID NO:39 |

Abbreviations:

| | |
|---|---|
| SEQ | Sequence |
| AL | AMPLICON LENGTH |
| MT | Melting Temperature |
| UEP | Unextended primer mass |
| EXT1 | Extended product 1 |
| EXT2 | Extended product 2 |

Figure 3

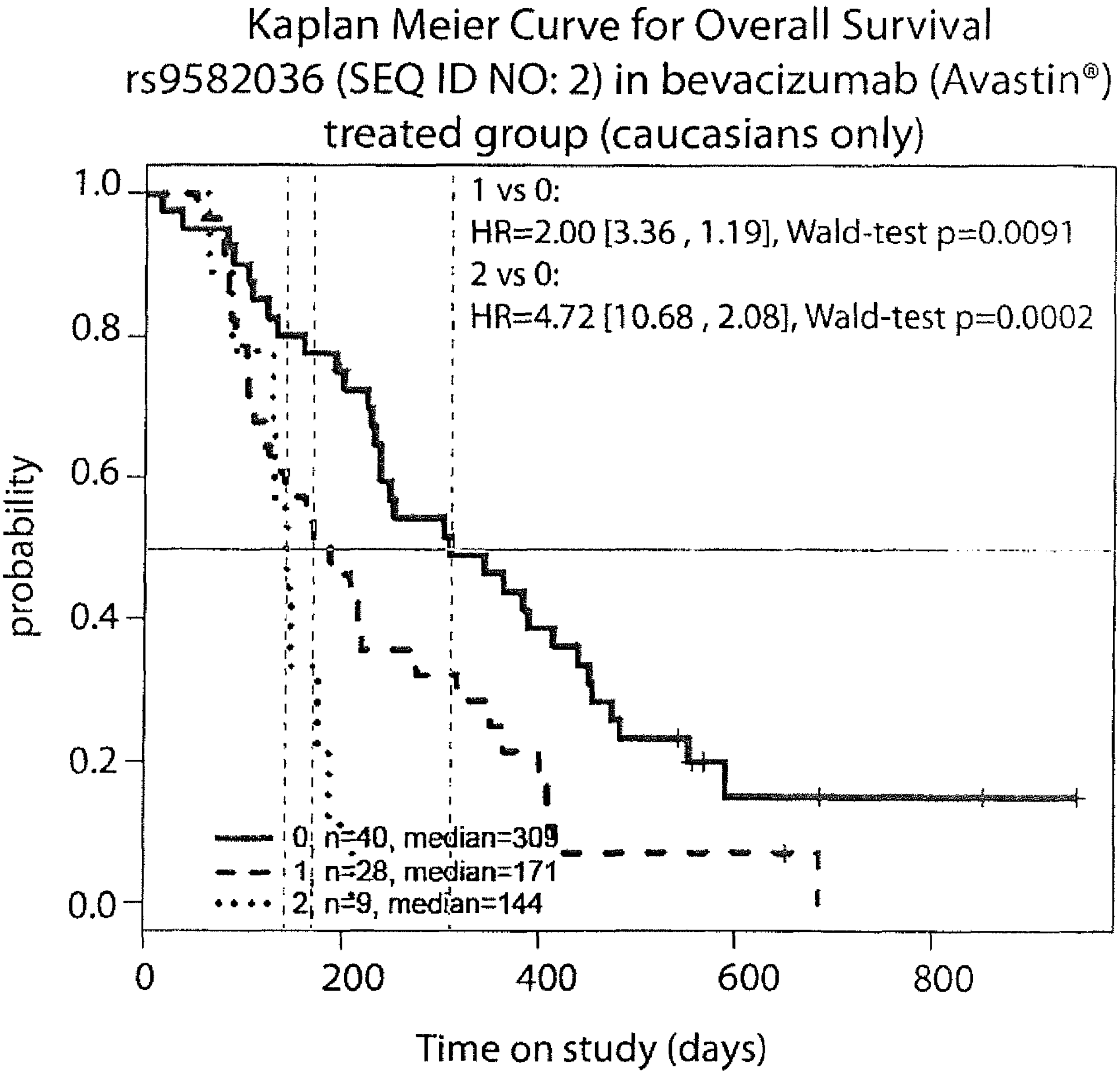


In the legend 0, 1 and 2 refer to genotypes:
0: AA (n=40, median=309)
1: AC (n=28, median=171)
2: CC (n=9, median=144)

Figure 4

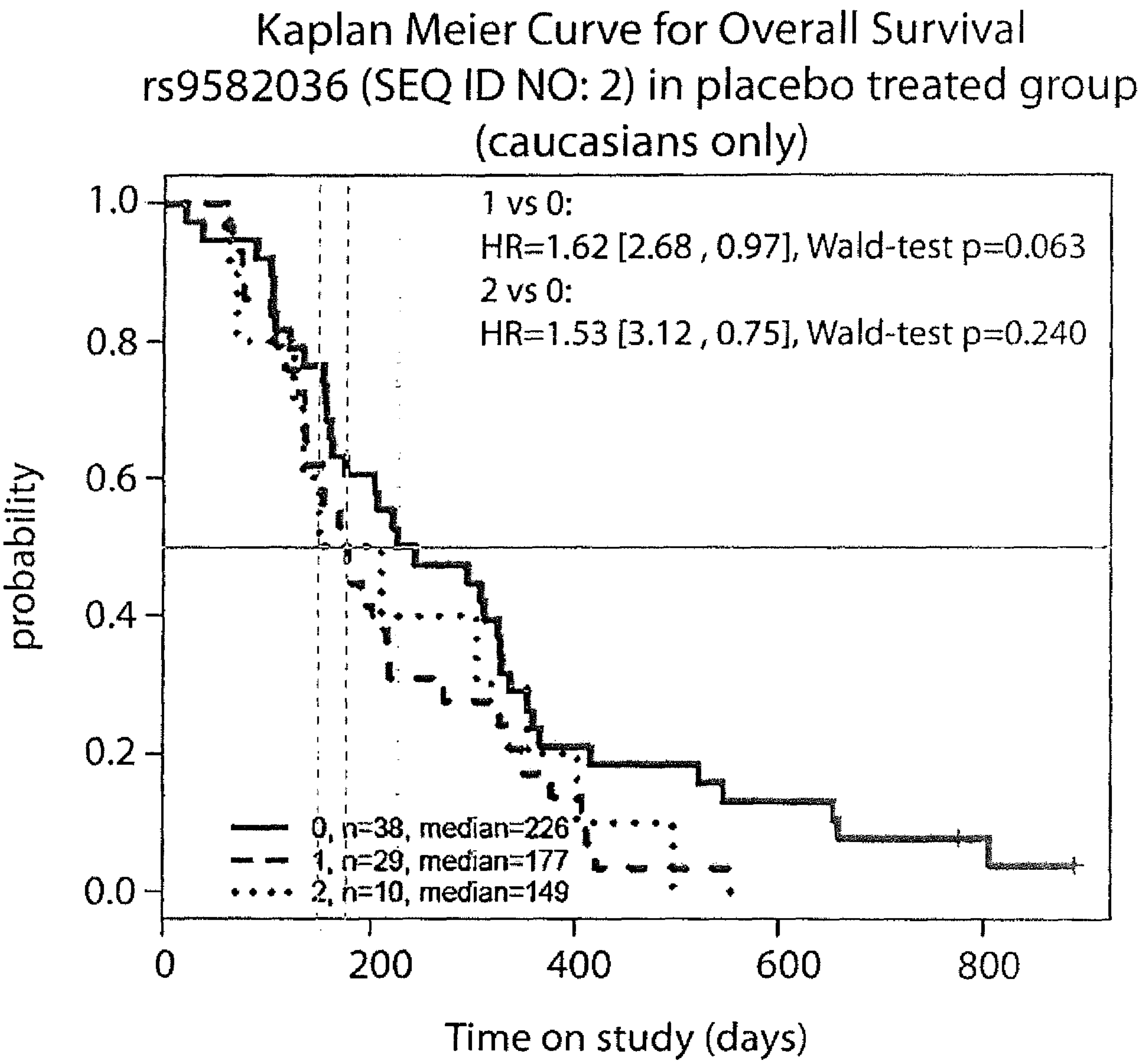


In the legend 0, 1 and 2 refer to genotypes:

0: AA (n=38, median=226)

1: AC (n=29, median=177)

2: CC (n=10, median=149)

Figure 5

Kaplan Meier Curve for Progression Free Survival rs9582036 (SEQ ID NO: 2) in bevacizumab (Avastin®) treated group (caucasians only)

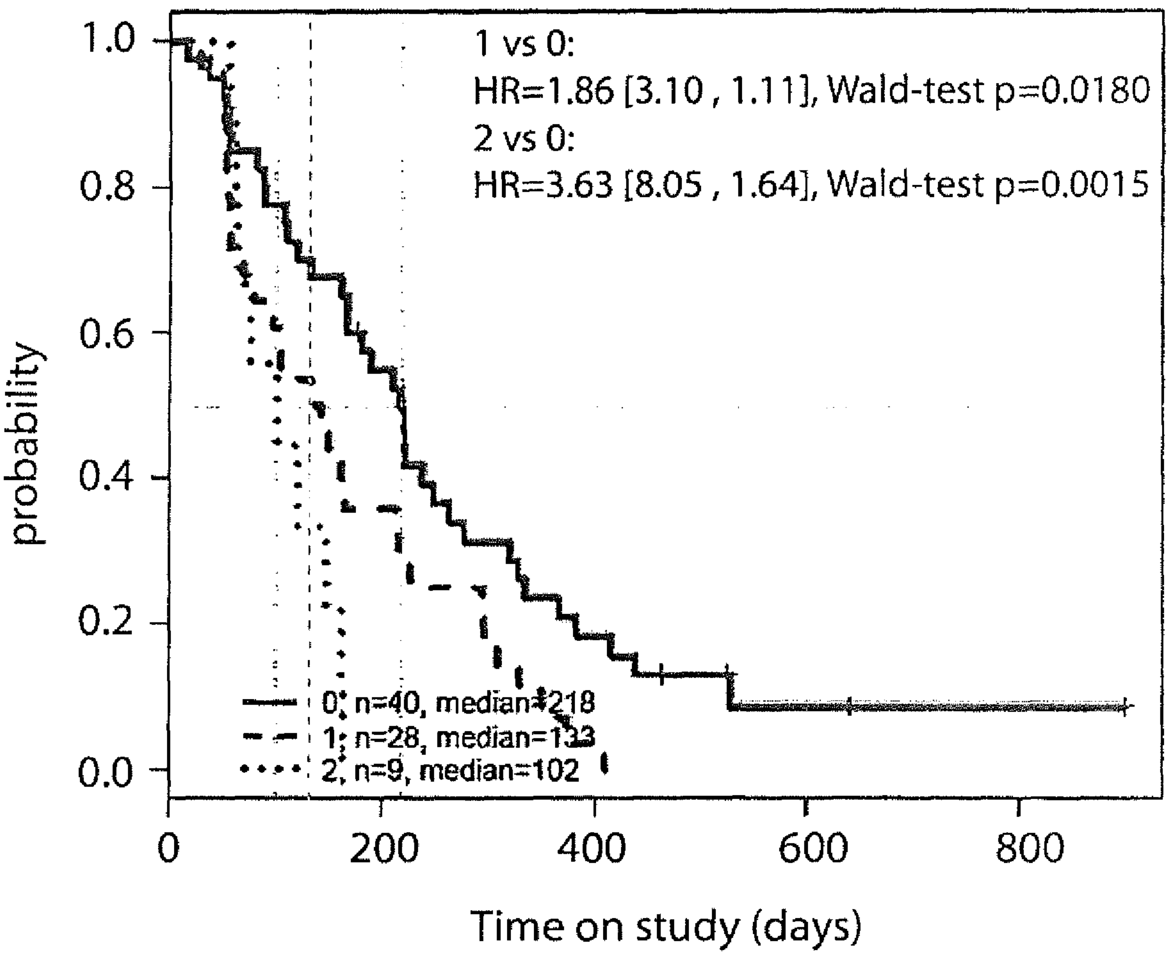


In the legend 0, 1 and 2 refer to genotypes:
0: AA (n=40, median=218)
1: AC (n=28, median=133)
2: CC (n=9, median=102)

Figure 6

Kaplan Meier Curve for Progression Free Survival rs9582036 (SEQ ID NO: 2) in placebo treated group (caucasians only)

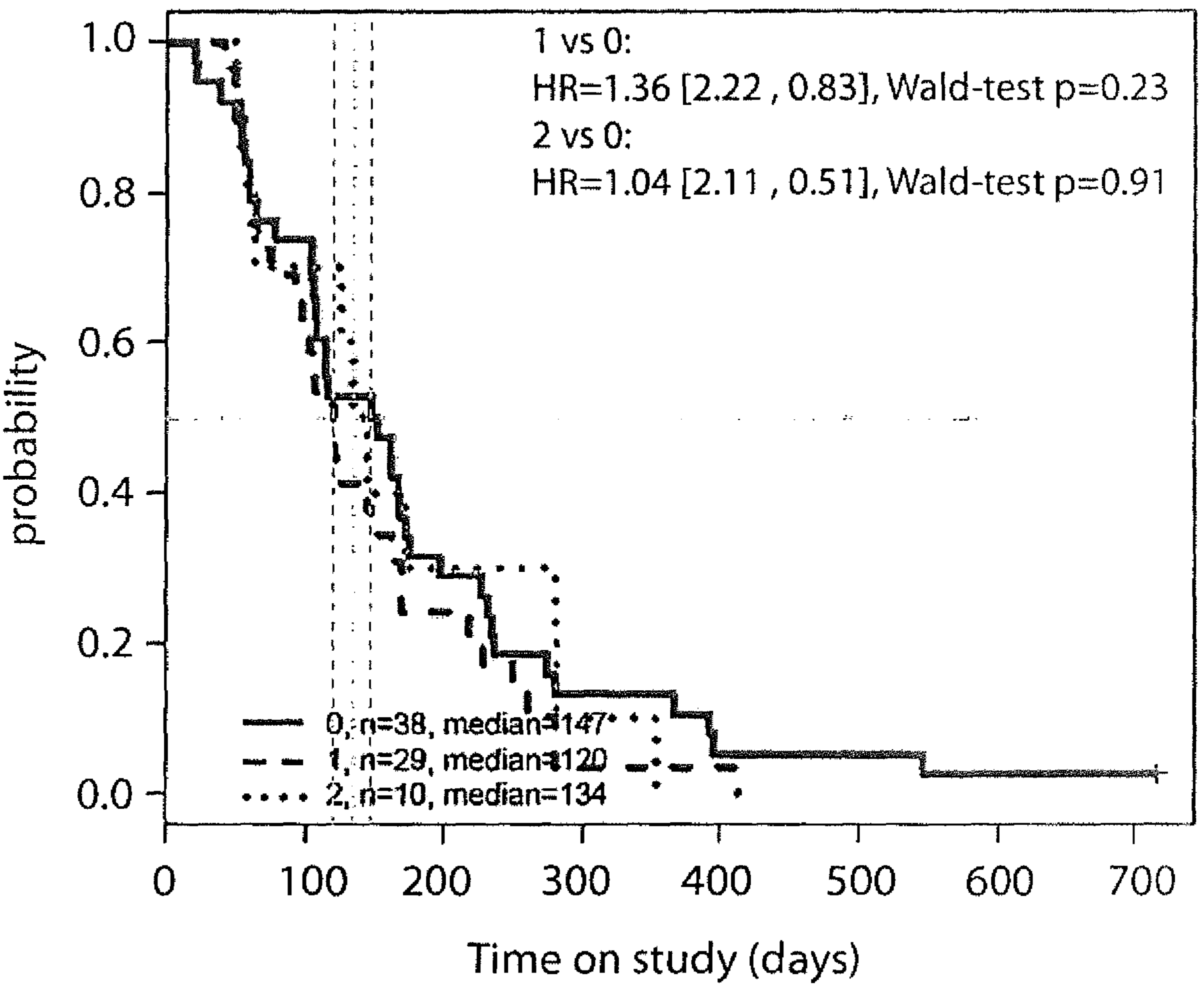


In the legend 0, 1 and 2 refer to genotypes:
0: AA (n=38, median=147)
1: AC (n=29, median=120)
2: CC (n=10, median=134)

Figure 7

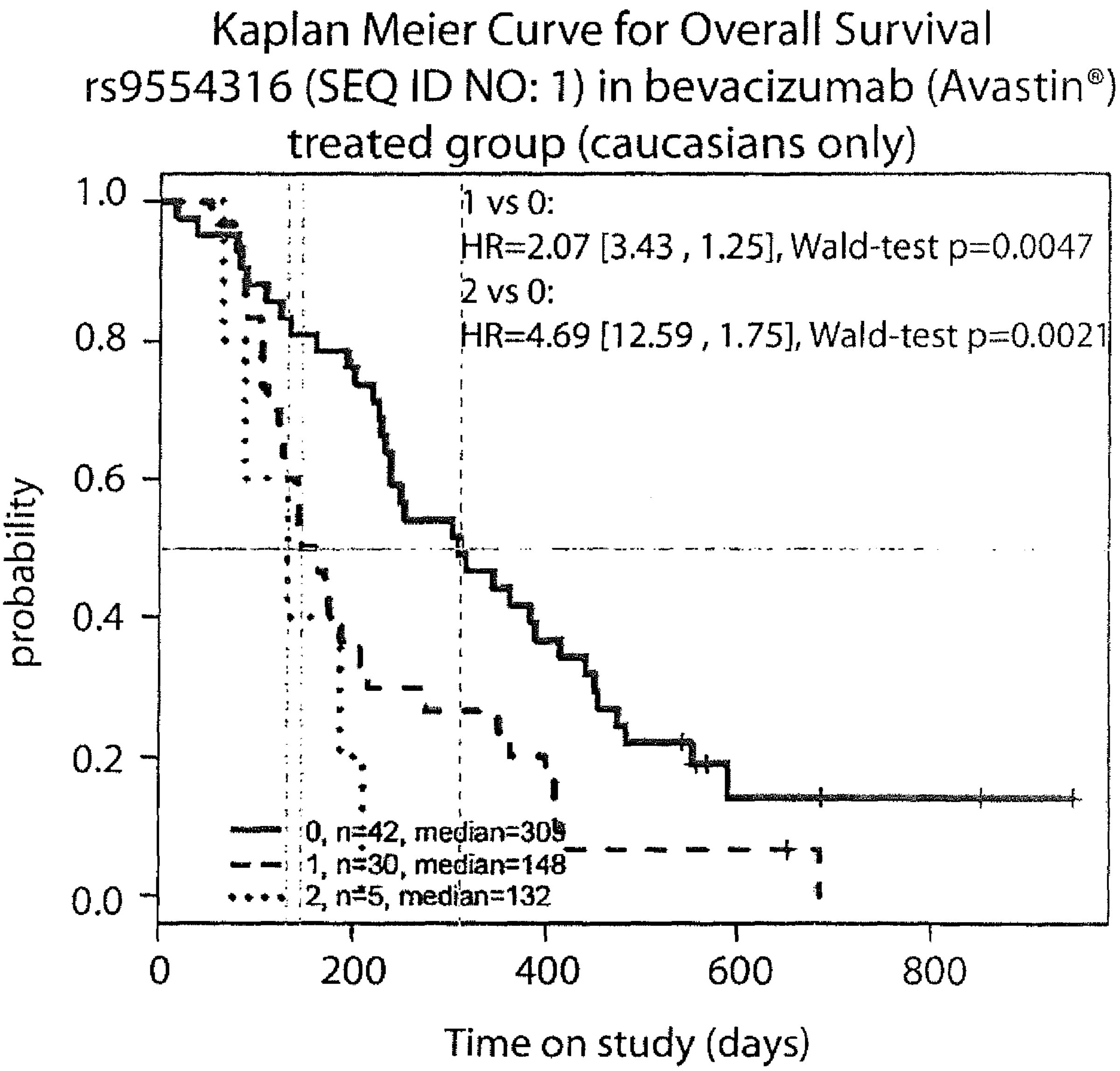


In the legend 0, 1 and 2 refer to genotypes:
0: GG (n=42, median=309)
1: TG (n=30, median=148)
2: TT (n=5, median=132)

Figure 8

Kaplan Meier Curve for Overall Survival
rs9554316 (SEQ ID NO: 1) in placebo treated group
(caucasians only)

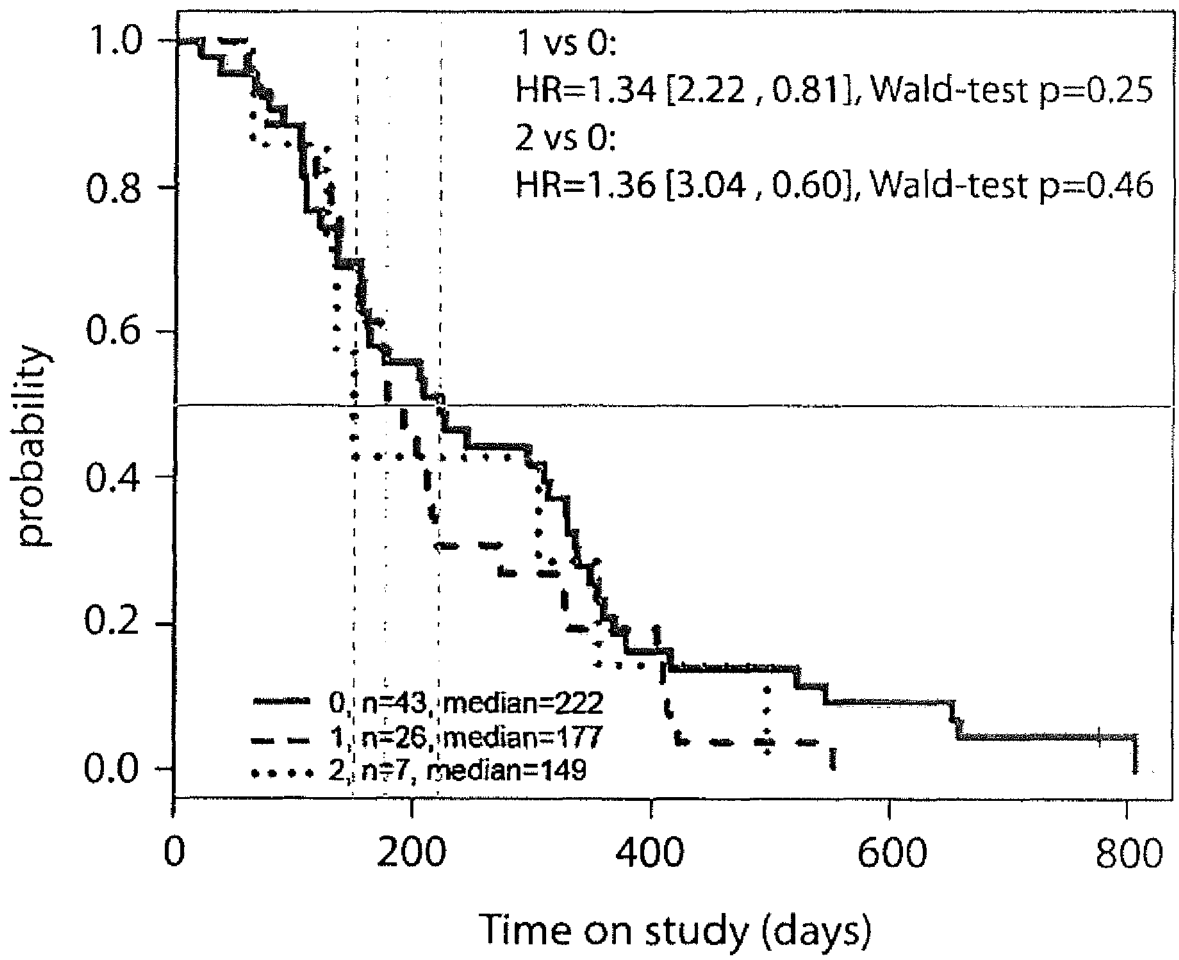


In the legend 0, 1 and 2 refer to genotypes:
0: GG (n=43, median=222)
1: TG (n=26, median=177)
2: TT (n=7, median=149)

Figure 9

Kaplan Meier Curve for Progression Free Survival rs9554316 (SEQ ID NO: 1) in bevacizumab (Avastin®) treated group (caucasians only)

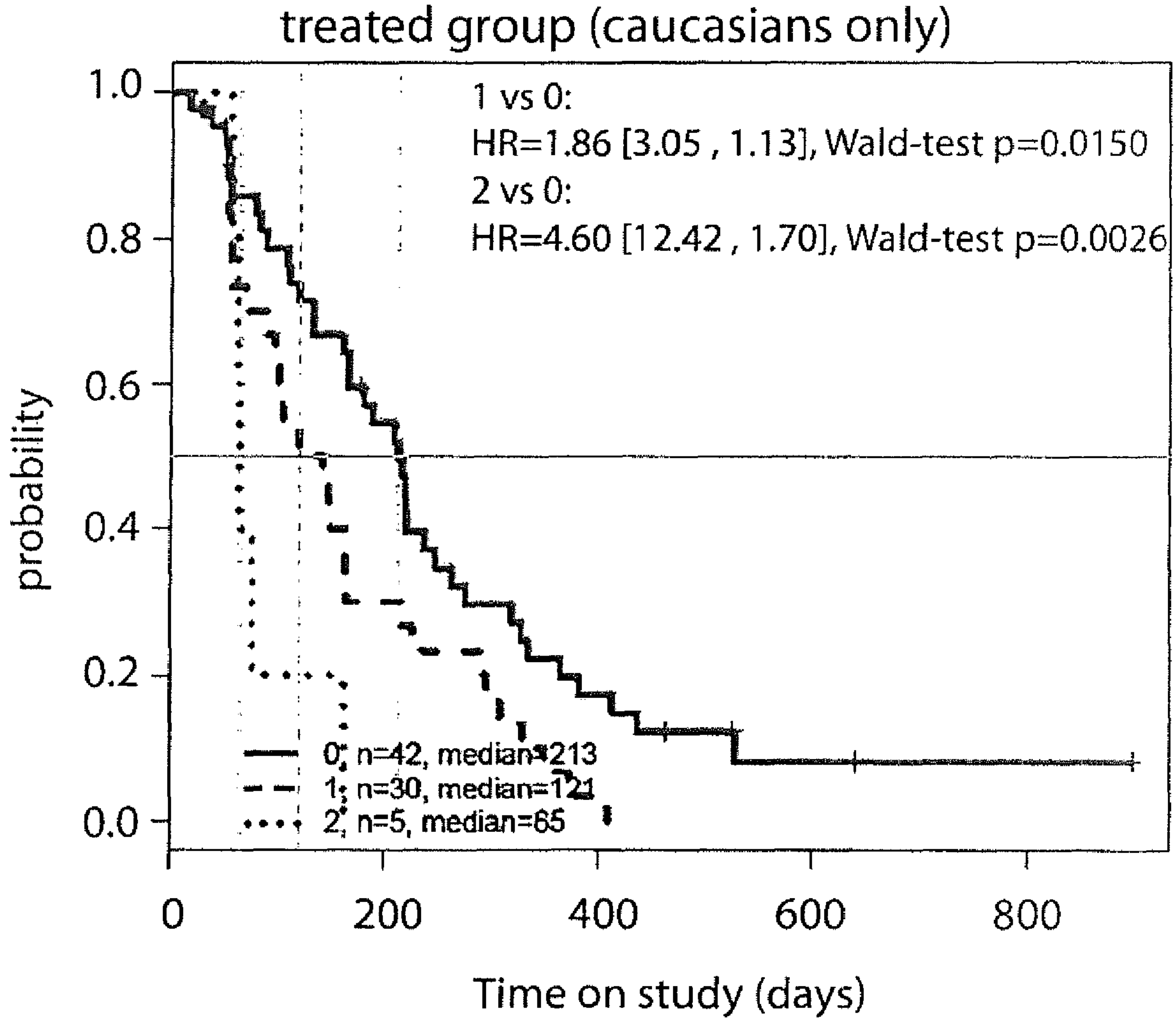


In the legend 0, 1 and 2 refer to genotypes:
0: GG (n=42, median=213)
1: TG (n=30, median=121)
2: TT (n=5, median=65)

Figure 10

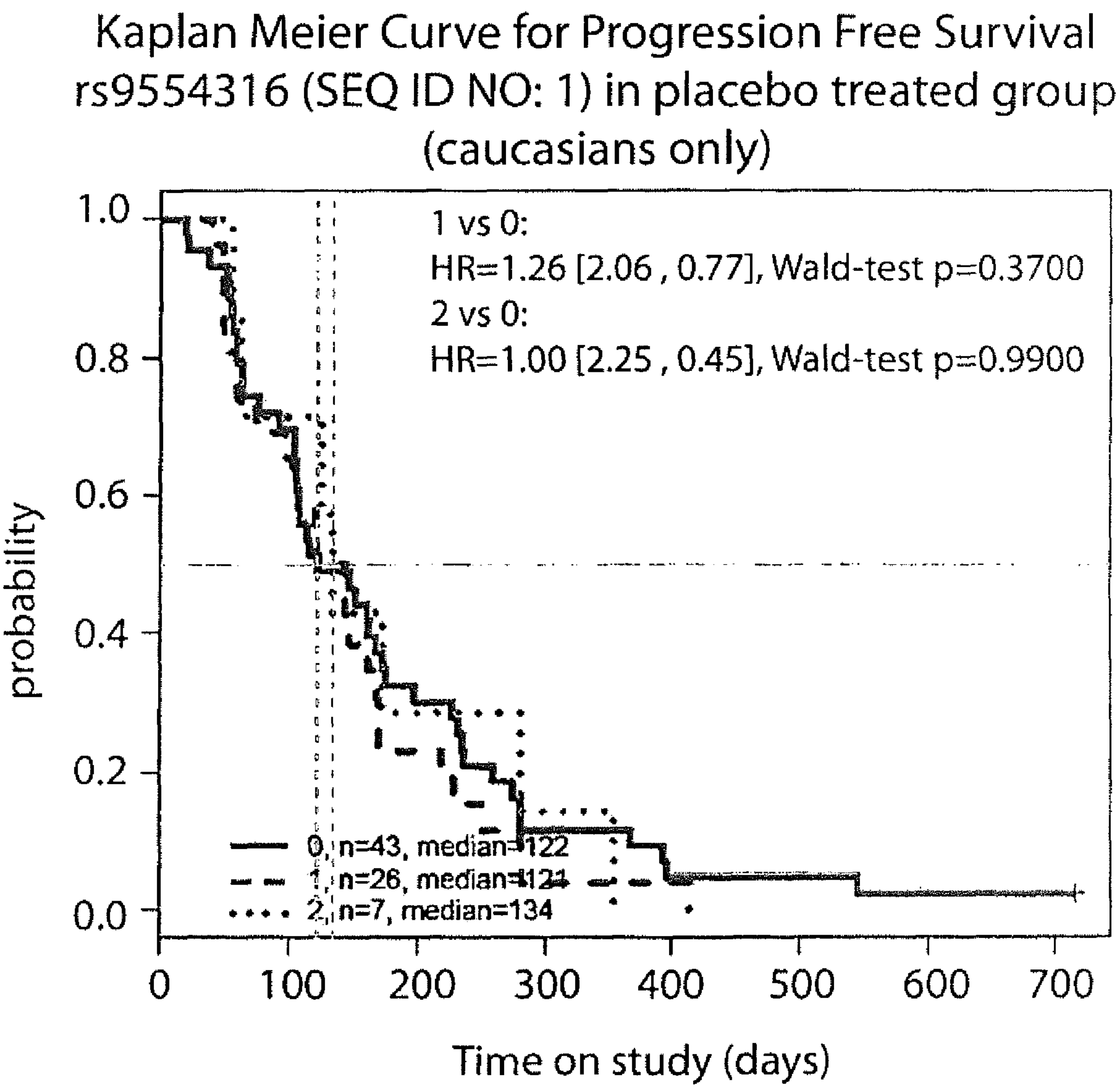


In the legend 0, 1 and 2 refer to genotypes:
0: GG (n=43, median=122)
1: TG (n=26, median=121)
2: TT (n=7, median=134)

Figure 11

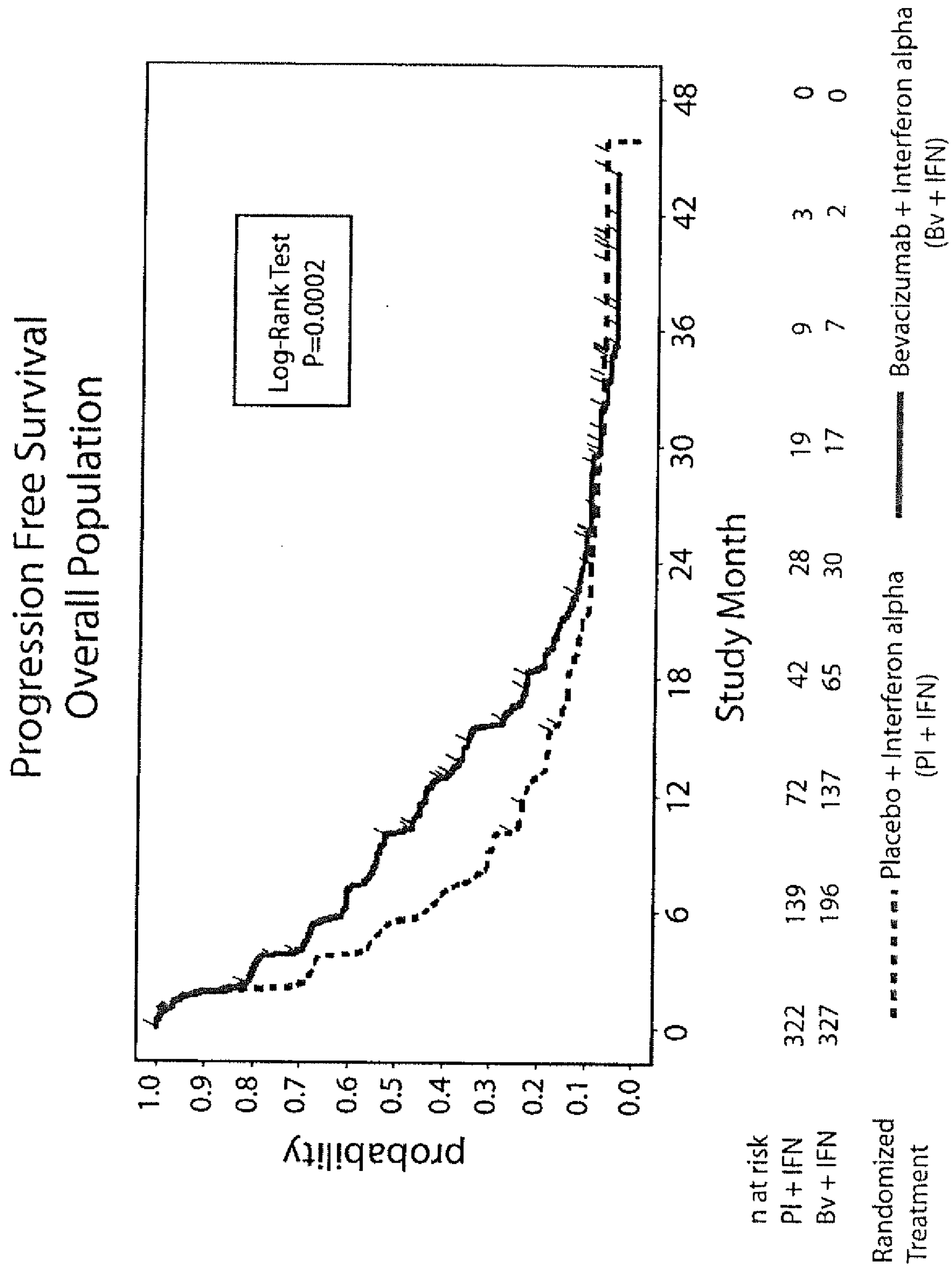
Progression Free Survival
Overall Population
Log-Rank Test
P=0.0002
probability
1.0
0.9
0.8
0.7
0.6
0.5
0.4
0.3
0.2
0.1
0.0
0
6
12
18
24
30
36
42
48
Study Month
n at risk
Pl + IFN 322 139 72 42 28 19 9 3 0
Bv + IFN 327 196 137 65 30 17 7 2 0
Randomized Treatment
Placebo + Interferon alpha (Pl + IFN)
Bevacizumab + Interferon alpha (Bv + IFN)

Figure 12

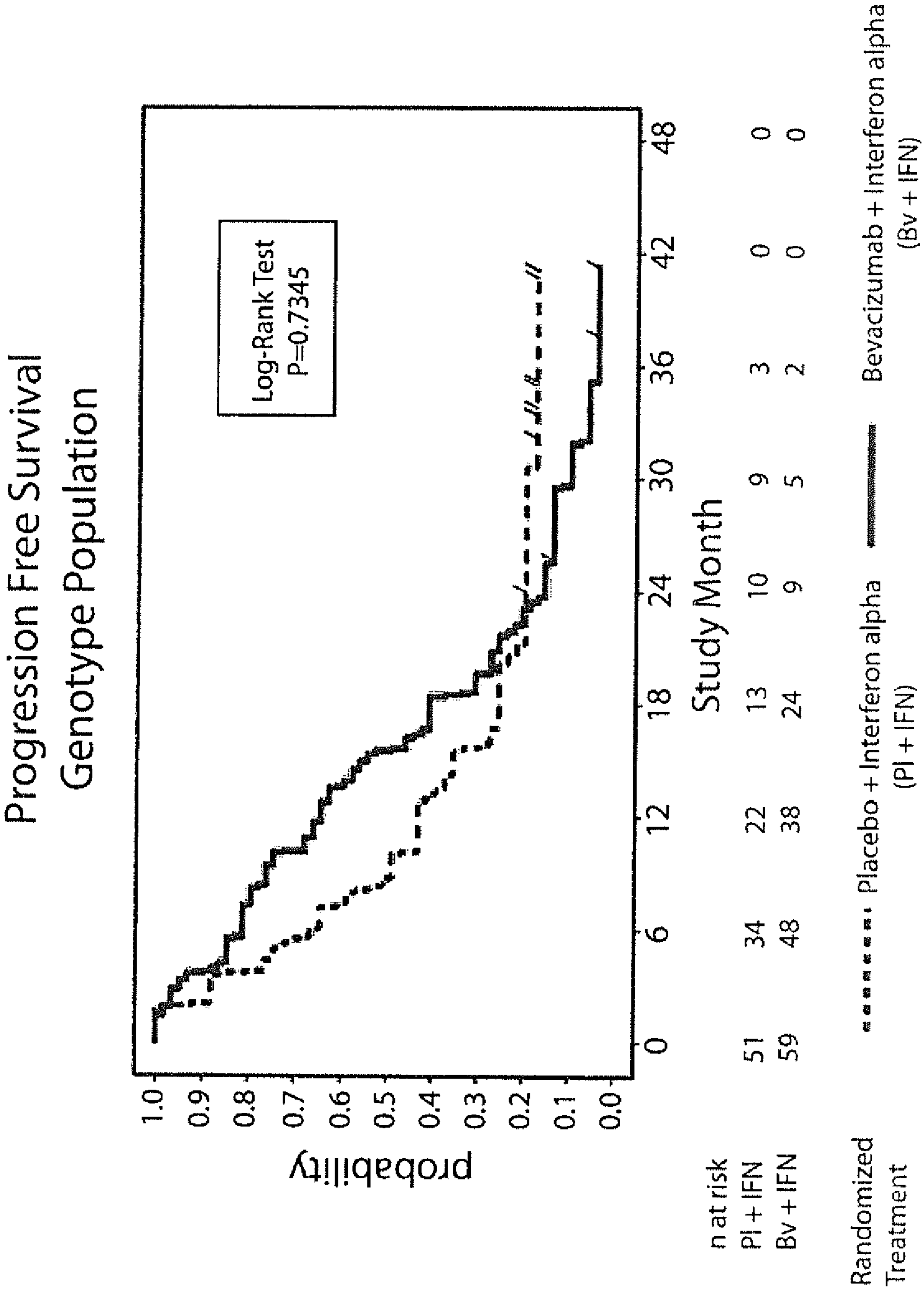
Progression Free Survival
Genotype Population
Log-Rank Test
P=0.7345
probability
1.0
0.9
0.8
0.7
0.6
0.5
0.4
0.3
0.2
0.1
0.0
0 6 12 18 24 30 36 42 48
Study Month
n at risk
Pl + IFN 51 34 22 13 10 9 3 0 0
Bv + IFN 59 48 38 24 9 5 2 0 0
Randomized Treatment
Placebo + Interferon alpha (Pl + IFN)
Bevacizumab + Interferon alpha (Bv + IFN)

Figure 13

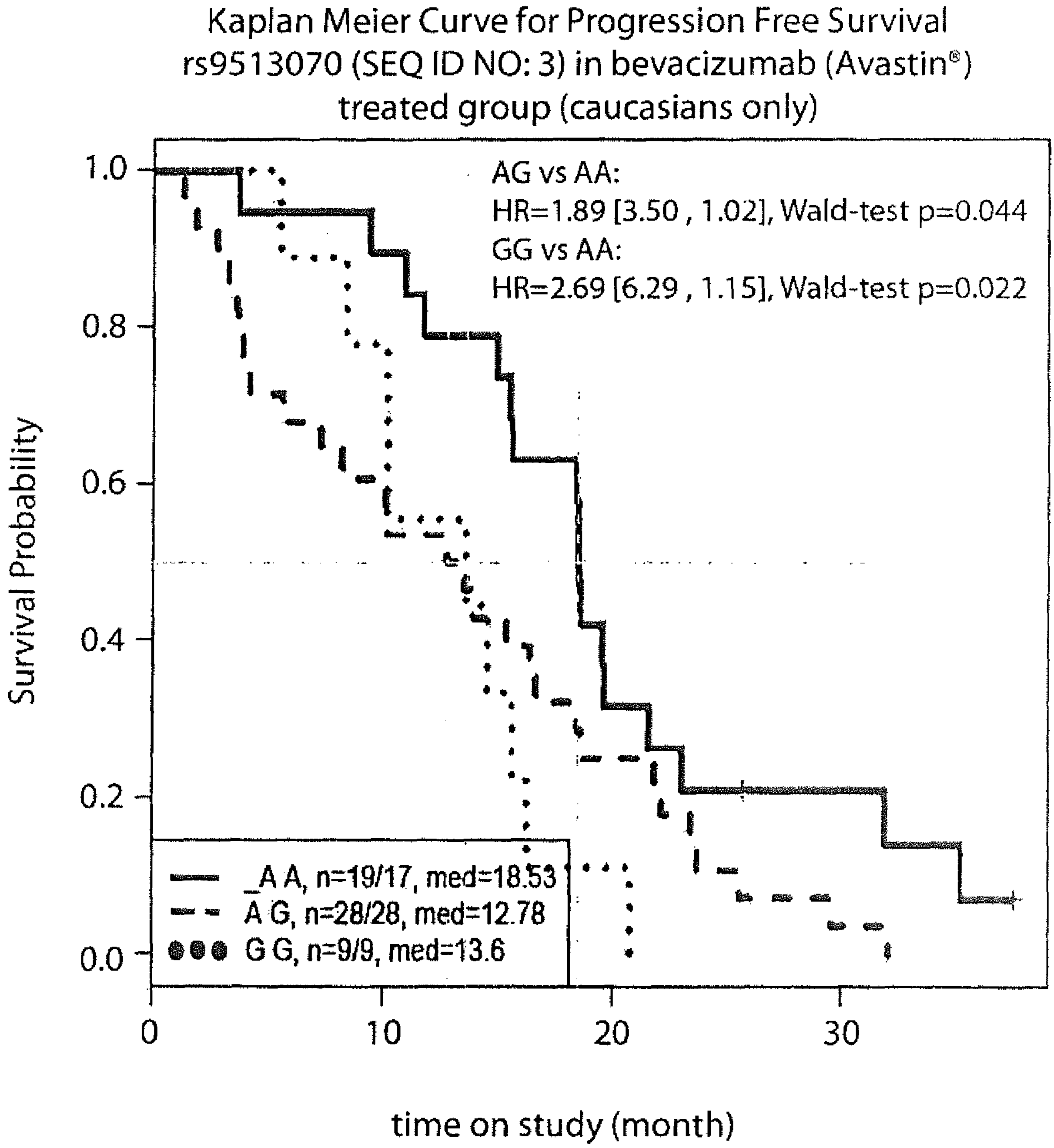


AA (n=19/17, med=18.53)
AG (n=28/28, med=12.78)
GG (n=9/9, med=13.6)

Figure 14

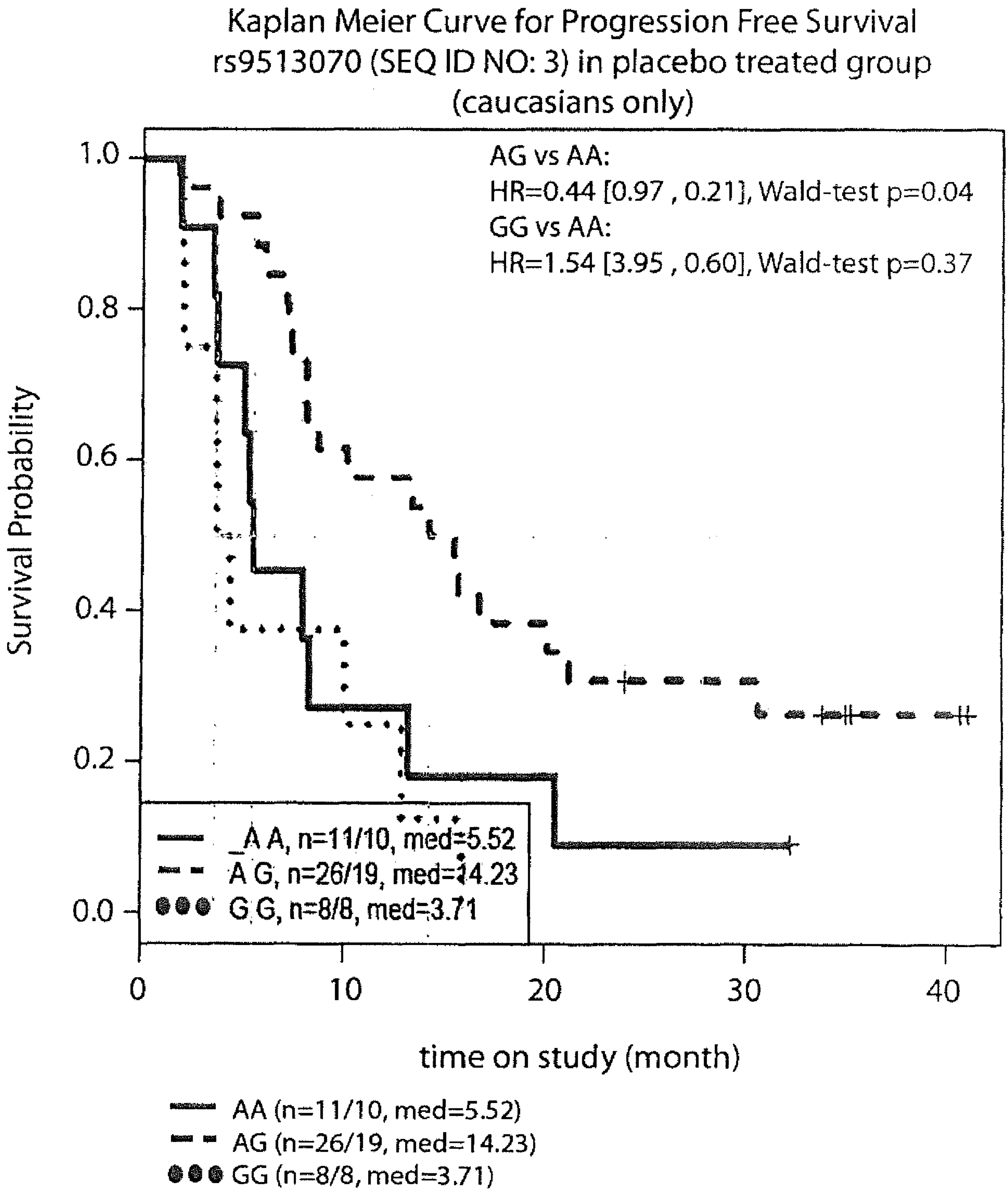
Kaplan Meier Curve for Progression Free Survival
rs9513070 (SEQ ID NO: 3) in placebo treated group
(caucasians only)
AG vs AA:
HR=0.44 [0.97 , 0.21], Wald-test p=0.04
GG vs AA:
HR=1.54 [3.95 , 0.60], Wald-test p=0.37
Survival Probability
1.0
0.8
0.6
0.4
0.2
0.0
A A, n=11/10, med=5.52
A G, n=26/19, med=14.23
G G, n=8/8, med=3.71
0
10
20
30
40
time on study (month)
AA (n=11/10, med=5.52)
AG (n=26/19, med=14.23)
GG (n=8/8, med=3.71)

Figure 15

Kaplan Meier Curve for Progression Free Survival
rs9554316 (SEQ ID NO: 1) in bevacizumab (Avastin®)
treated group (caucasians only)

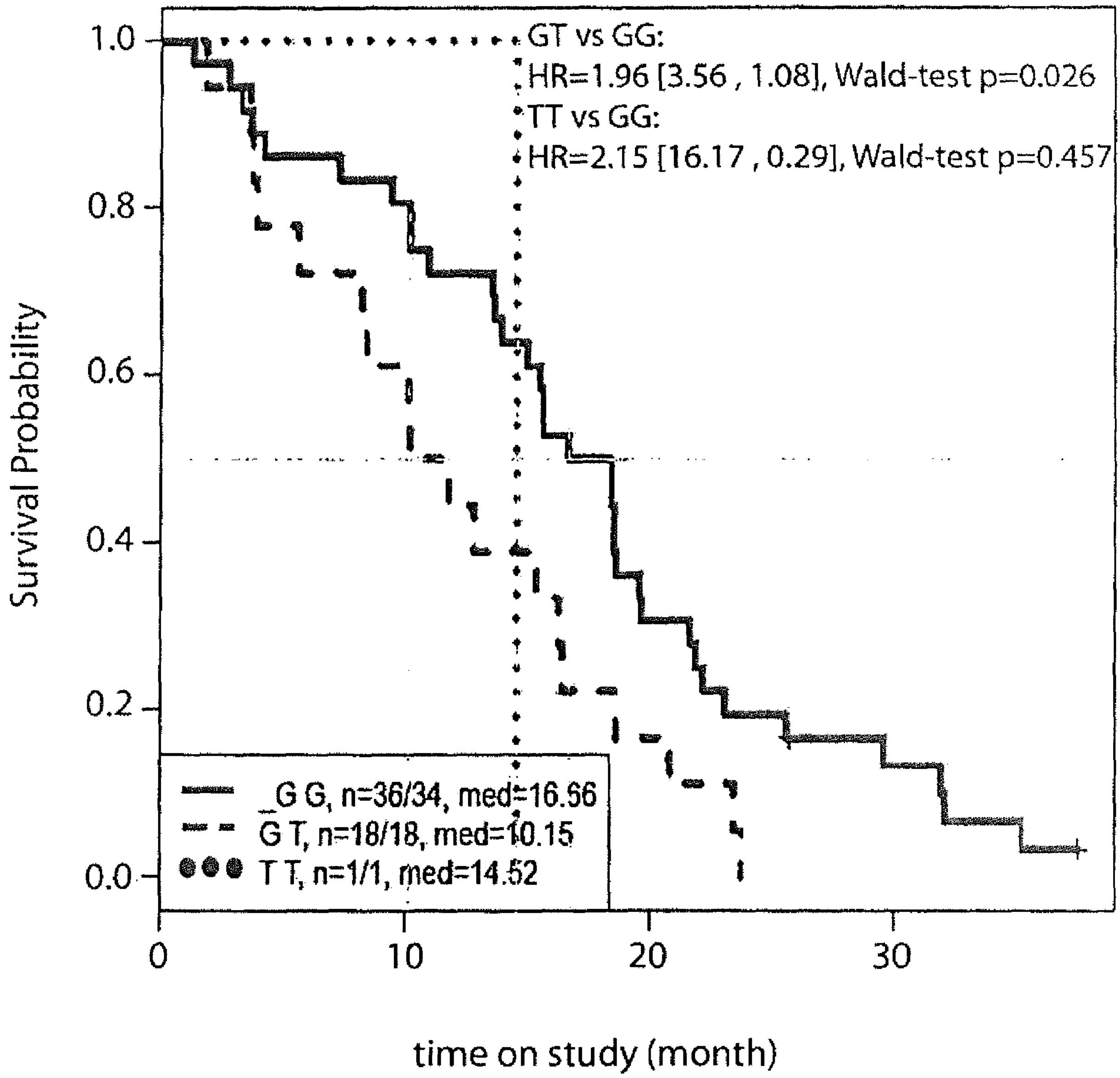


GG (n=36/34, med=16.66)
GT (n=18/18, med=10.15)
TT (n=1/1, med=14.52)

Figure 16

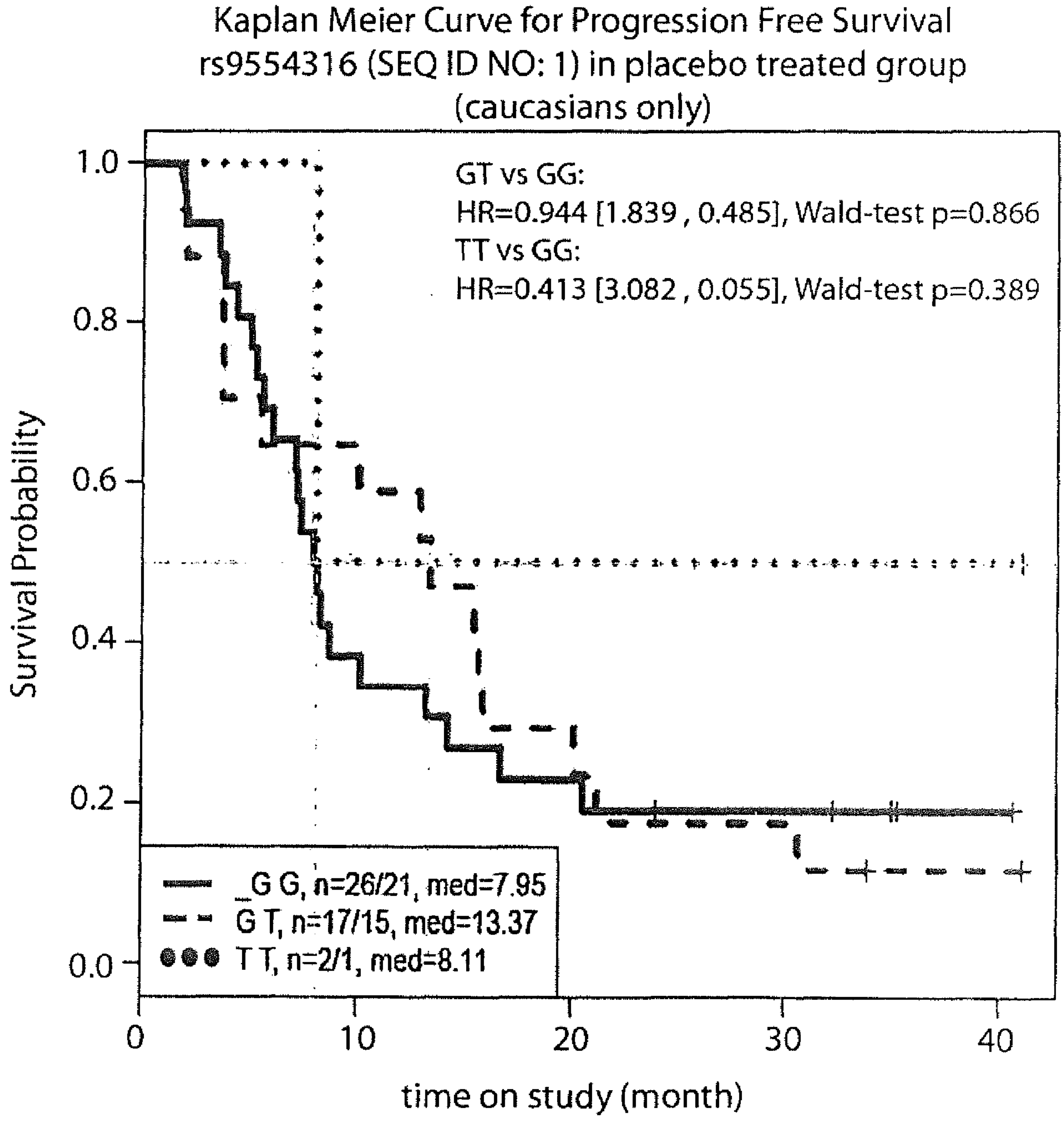


GG (n=26/21, med=7.95)
GT (n=17/15, med=13.37)
TT (n=2/1, med=8.11)

Europäisches Patentamt
European Patent Office
Office européen des brevets

# EUROPEAN SEARCH REPORT

Application Number
EP 16 19 4527

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2007/109183 A2 (NOVARTIS AG [CH]; NOVARTIS PHARMA GMBH [AT]; CULVER KENNETH WAYNE [US]) 27 September 2007 (2007-09-27) * paragraphs [0010], [0021], [0022], [0025], [0085] - [0093]; claims 1-15 * | 1-16 | INV. C12Q1/68 |
| A | US 2004/091912 A1 (SMITH JOHN C [GB]) 13 May 2004 (2004-05-13) * Examples; paragraphs [0061] - [0066] * | 1-16 | |
| A | US 2007/254295 A1 (HARVEY JEANNE [US] ET AL) 1 November 2007 (2007-11-01) * paragraph [0031]; claims 1-5,7,13-15; example 2 * | 1-16 | |
| A | PASQUALETTI GIUSEPPE ET AL: "Vascular endothelial growth factor pharmacogenetics: a new perspective for anti-angiogenic therapy", PHARMACOGENOMICS, ASHLEY PUBLICATIONS, GB LNKD- DOI:10.2217/14622416.8.1.49, vol. 8, no. 1, 1 January 2007 (2007-01-01), pages 49-66, XP008128080, ISSN: 1462-2416 * page 61, 7th bullet point of the box "Highlights"; page 60, column 2, paragraph 2 * | 1-16 | TECHNICAL FIELDS SEARCHED (IPC): C12Q |
| | -/-- | | |

The present search report has been drawn up for all claims

1

| Place of search | Date of completion of the search | Examiner |
|---|---|---|
| The Hague | 13 February 2017 | Cornelis, Karen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 16 19 4527

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | MESHINCHI SOHEIL ET AL: "Activating mutations of RTK/ras signal transduction pathway in pediatric acute myeloid leukemia", BLOOD, AMERICAN SOCIETY OF HEMATOLOGY, US LNKD- DOI:10.1182/BLOOD-2003-01-0137, vol. 102, no. 4, 15 August 2003 (2003-08-15), pages 1474-1479, XP008092855, ISSN: 0006-4971 [retrieved on 2003-04-17] * page 1475, column 2, paragraph 5 * ----- | 1-16 | |
| A | SCHNEIDER BRYAN P ET AL: "Association of vascular endothelial growth factor and vascular endothelial growth factor receptor-2 genetic polymorphisms with outcome in a trial of paclitaxel compared with paclitaxel plus bevacizumab in advanced breast cancer: ECOG 2100.", JOURNAL OF CLINICAL ONCOLOGY : OFFICIAL JOURNAL OF THE AMERICAN SOCIETY OF CLINICAL ONCOLOGY 1 OCT 2008 LNKD- PUBMED:18824714, vol. 26, no. 28, 1 October 2008 (2008-10-01), pages 4672-4678, XP002609306, ISSN: 1527-7755 * abstract * ----- | 1-16 | TECHNICAL FIELDS SEARCHED (IPC) |
| | -/-- | | |

The present search report has been drawn up for all claims

1

| Place of search | Date of completion of the search | Examiner |
|---|---|---|
| The Hague | 13 February 2017 | Cornelis, Karen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 16 19 4527

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DATABASE PROBE [Online] NCBI; 6 March 2006 (2006-03-06), "Sequence-specific oligonucleotide (SSO) for Homo sapiens variation rs9582036", XP002609307, retrieved from http://www.ncbi.nlm.nih.gov/probe Database accession no. Pr004907474 * the whole document * ----- | 1-16 | |
| A | DATABASE PROBE [Online] NCBI; 6 March 2006 (2006-03-06), "Sequence-specific oligonucleotide (SSO) for Homo sapiens variation rs9554320", XP002609308, retrieved from http://www.ncbi.nlm.nih.gov/probe Database accession no. Pr004962263.1 * the whole document * ----- | 1-16 | TECHNICAL FIELDS SEARCHED (IPC) |
| A | DATABASE PROBE [Online] NCBI; 6 March 2006 (2006-03-06), "Sequence-specific oligonucleotide (SSO) probe for Homo sapiens variation rs9554316.", XP002609309, retrieved from http://www.ncbi.nlm.nih.gov/probe Database accession no. Pr004962234.1 * the whole document * ----- -/-- | 1-16 | |

The present search report has been drawn up for all claims

1

| Place of search | Date of completion of the search | Examiner |
|---|---|---|
| The Hague | 13 February 2017 | Cornelis, Karen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 16 19 4527

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DATABASE PROBE [Online] NCBI; 6 March 2006 (2006-03-06), "Sequence-specific oligonucleotide (SSO) for Homo sapiens variation rs9513070.", XP002609310, retrieved from http://www.ncbi.nlm.nih.gov/probe Database accession no. Pr004812914.1 * the whole document * ----- | 1-16 | |
| X,P | LAMBRECHTS D ET AL: "16LBA VEGFR-1 polymorphisms as potential predictors of clinical outcome in bevacizumab-treated patients with metastatic pancreatic cancer", EUROPEAN JOURNAL OF CANCER. SUPPLEMENT, PERGAMON, OXFORD, GB LNKD- DOI:10.1016/S1359-6349(09)72051-8, vol. 7, no. 3, 1 September 2009 (2009-09-01), page 10, XP026629173, ISSN: 1359-6349 [retrieved on 2009-09-01] * abstract * ----- | 1-16 | |
| | | | TECHNICAL FIELDS SEARCHED (IPC) |

The present search report has been drawn up for all claims

1

| Place of search | Date of completion of the search | Examiner |
|---|---|---|
| The Hague | 13 February 2017 | Cornelis, Karen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 16 19 4527

This annex lists the patent family members relating to the patent documents cited in the above-mentioned European search report. The members are as contained in the European Patent Office EDP file on
The European Patent Office is in no way liable for these particulars which are merely given for the purpose of information.

13-02-2017

| Patent document cited in search report | Publication date | Patent family member(s) | Publication date |
|---|---|---|---|
| WO 2007109183 A2 | 27-09-2007 | NONE | |
| US 2004091912 A1 | 13-05-2004 | EP 1130123 A2 | 05-09-2001 |
| | | JP 2001299366 A | 30-10-2001 |
| | | US 2002192647 A1 | 19-12-2002 |
| | | US 2004091912 A1 | 13-05-2004 |
| US 2007254295 A1 | 01-11-2007 | US 2007254295 A1 | 01-11-2007 |
| | | US 2011244465 A1 | 06-10-2011 |
| | | WO 2007109571 A2 | 27-09-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **VAN CUTSEM et al.** *J. Clinc. Oncol.,* 2009, vol. 27, 2231-7
- **PE'ER, I. et al.** *Nat. Genet.,* 2006, vol. 38, 663-7
- **BARRETT, J.C. et al.** *Bioinformatics,* 2005, vol. 21, 263-5
- **ESCUDIER et al.** *Lancet,* 2007, vol. 370, 2103-2111

SEQUENCE LISTING

<110>  F. Hoffmann - La Roche AG
       Life Sciences Research Partners vzw
       VIB vzw

<120>  Responsiveness to Angiogenesis Inhibitors

<130>  R2351 EP/2 S3

<150>  EP 09 16 7184.2
<151>  2009-08-04

<160>  71

<170>  PatentIn version 3.3

<210>  1
<211>  101
<212>  DNA
<213>  Homo sapiens

<400>  1
atagacacaa attctaagaa atcgtaaaga catcattcga tttttttct kgaactcaat     60

ggaattctca gcccccagcc cttggtcatg gtctccagca a                        101

<210>  2
<211>  101
<212>  DNA
<213>  Homo sapiens

<400>  2
tgctgcgatt acaggcacga gccactgtgc ccagcaacaa tagccttctt mcaaaatgta     60

tactaaagta tttaggagta aagtgctatt atgccttaag c                        101

<210>  3
<211>  101
<212>  DNA
<213>  Homo sapiens

<400>  3
ttgcttttta tactataatg caagcttgcc caacttgtgg cccacgggct rcatgaatcc     60

caggatggct ttgagtgcag cccaacacaa actcgtaaac t                        101

<210>  4
<211>  101
<212>  DNA
<213>  Homo sapiens

<400>  4
tttccggggc tgtgagcaag gttcctgtgt gtagctgatc attctccccc mgcactgcaa     60

agccgctgtt acaaactggc ctcccaccac tcaaggcaca c                        101

<210>  5
<211>  101

<212> DNA
<213> Homo sapiens

<400> 5
agtgcaaaac atccacaaag ccccaggaaa aaaagtgtcc aagacaggca rgcaagtgca 60

atttgcacag tggagaaagc ctcacatgga cacccgaaaa g 101

<210> 6
<211> 101
<212> DNA
<213> Homo sapiens

<400> 6
acattactgg cgttggtgtt tggaagggat ctgaagaagg ggtctatcgg rgtgcactag 60

gtaccttaac ttcacgactt acatcaaaca tggaggtggc a 101

<210> 7
<211> 101
<212> DNA
<213> Homo sapiens

<400> 7
ccccttagcc cctcaacccc acatcacagg gctttagaga gaagagctgg ragacctctt 60

gtttgccagg ctatactggt tagcttctcc cattgtgatt g 101

<210> 8
<211> 101
<212> DNA
<213> Homo sapiens

<400> 8
cttcttccaa aatgtatact aaagtattta ggagtaaagt gctattatgc yttaagcctc 60

cttttaaatg gtttggcaaa aagaaaaatc cgtaacaaac a 101

<210> 9
<211> 101
<212> DNA
<213> Homo sapiens

<400> 9
ctcccagagg gacctactct ggtccgttgt gaaatcagag aatgccagac rtaggagaga 60

ctggaggcat catctcgtct gagtgcccaa tggcacagga g 101

<210> 10
<211> 101
<212> DNA
<213> Homo sapiens

<400> 10
gaggacacca cgcaccagtg acaagaacag gtcccctccc gcttccctac rctcaggcag 60

agctccccag ccctgctgtt gggcctgagt ctcctgtcca c 101

<210> 11
<211> 101
<212> DNA
<213> Homo sapiens

<400> 11
tcttcaaagg ttttgattct ttccaggctc atgaacttga aagcatttac rtatctaatg 60

aagaaacaga aagaattatc aagacaggaa aaggcatcca g 101

<210> 12
<211> 101
<212> DNA
<213> Homo sapiens

<400> 12
ttacttgtgc tgggagatgg cggttgctgg ggaaaaaacc tgagaaaccc kagttcatat 60

cctccctctg acacttctta gaggtatgcc tttagggggc g 101

<210> 13
<211> 101
<212> DNA
<213> Homo sapiens

<400> 13
aggaagtgca tgatcttttc tgacctggcc tccaagccat gcagtgtccc ktctttcaca 60

ttgcattgat cacaagtgag tcctaaagct agctctgatt c 101

<210> 14
<211> 101
<212> DNA
<213> Homo sapiens

<400> 14
cttcaagtca tttgcctgaa tgaatgattt tattcttatg ttatgaaacc sataatactt 60

tttaaaggat ttcagattct tgaagacaaa atgcctcttg c 101

<210> 15
<211> 101
<212> DNA
<213> Homo sapiens

<400> 15
gcagtgtatg agggttccaa tttctccata tcctcgccac actgacttca yctagctatc 60

ctcatgggcg taaagtggta tcttatggta atttctttcc c 101

<210> 16
<211> 101
<212> DNA
<213> Homo sapiens

<400> 16
gtggctgtct gcagggacat gctctgcatt cagagagaac actggggaga ygacacgttt 60

ctctttttgt gaaacatgct cccgtagtga ttactaaatg a 101

<210> 17
<211> 101
<212> DNA
<213> Homo sapiens

<400> 17
agggaggaca tgggaggtca tccacaaaag tgaaaaataa cactatgtct raaaacaagc 60

agggaagaga agccagtacg agccagcggg gagatgctag g 101

<210> 18
<211> 101
<212> DNA
<213> Homo sapiens

<400> 18
agaacactgg ggagacgaca cgtttctctt tttgtgaaac atgctcccgt wgtgattact 60

aaatgacatt cctcccagag catgcaacac gggcagagct a 101

<210> 19
<211> 101
<212> DNA
<213> Homo sapiens

<400> 19
agaaggacag aaatacgttt gaggagggga gaggcaaaag ccagcctccc ygcctcttaa 60

ccagaccctt gcgagtagag cggcttctgg ttactgtcac a 101

<210> 20
<211> 30
<212> DNA
<213> Artificial Sequence

<220>
<221> source
<223> /note="description of artificial sequence: an artificially synthesized primer for PCR"

<400> 20
acgttggatg actgtgccca gcaacaatag 30

<210> 21
<211> 30
<212> DNA
<213> Artificial Sequence

<220>
<221> source
<223> /note="description of artificial sequence: an artificially synthesized primer for PCR"

<400> 21
acgttggatg gcataatagc actttactcc 30

<210> 22
<211> 18
<212> DNA
<213> Artificial Sequence

<220>
<221> source
<223> /note="description of artificial sequence: an artificially synthesized probe"

<400> 22
agcaacaata gccttctt 18

<210> 23
<211> 19
<212> DNA
<213> Artificial Sequence

<220>
<221> source
<223> /note="description of artificial sequence: an artificially synthesized probe or PCR extension product"

<400> 23
agcaacaata gccttcttc 19

<210> 24
<211> 19
<212> DNA
<213> Artificial Sequence

<220>
<221> source
<223> /note="description of artificial sequence: an artificially synthesized probe or PCR extension product"

<400> 24
agcaacaata gccttctta 19

<210> 25
<211> 29
<212> DNA
<213> Artificial Sequence

<220>
<221> source
<223> /note="description of artificial sequence: an artificially synthesized primer for PCR"

<400> 25
acgttggatg atcgtaaaga catcattcg 29

<210> 26
<211> 30
<212> DNA
<213> Artificial Sequence

<220>
<221> source
<223> /note="description of artificial sequence: an artificially synthesized primer for PCR"

<400> 26
acgttggatg tgctggagac catgaccaag 30

<210> 27
<211> 24
<212> DNA
<213> Artificial Sequence

<220>
<221> source
<223> /note="description of artificial sequence: an artificially synthesized probe"

<400> 27
aagacatcat tcgatttttt ttct 24

<210> 28
<211> 25
<212> DNA
<213> Artificial Sequence

<220>
<221> source
<223> /note="description of artificial sequence: an artificially synthesized probe or PCR extension product"

<400> 28
aagacatcat tcgatttttt ttctg 25

<210> 29
<211> 25
<212> DNA
<213> Artificial Sequence

<220>
<221> source
<223> /note="description of artificial sequence: an artificially synthesized probe or PCR extension product"

<400> 29
aagacatcat tcgatttttt ttctt 25

<210> 30
<211> 30
<212> DNA
<213> Artificial Sequence

<220>
<221> source
<223> /note="description of artificial sequence: an artificially synthesized primer for PCR"

<400> 30
acgttggatg gtgggaggcc agtttgtaac 30

```
<210>  31
<211>  30
<212>  DNA
<213>  Artificial Sequence

<220>
<221>  source
<223>  /note="description of artificial sequence: an artificially
       synthesized primer for PCR"

<400>  31
acgttggatg agcaaggttc ctgtgtgtag                                        30


<210>  32
<211>  18
<212>  DNA
<213>  Artificial Sequence

<220>
<221>  source
<223>  /note="description of artificial sequence: an artificially
       synthesized probe"

<400>  32
acagcggctt tgcagtgc                                                     18


<210>  33
<211>  19
<212>  DNA
<213>  Artificial Sequence

<220>
<221>  source
<223>  /note="description of artificial sequence: an artificially
       synthesized probe or PCR extension product"

<400>  33
acagcggctt tgcagtgcg                                                    19


<210>  34
<211>  19
<212>  DNA
<213>  Artificial Sequence

<220>
<221>  source
<223>  /note="description of artificial sequence: an artificially
       synthesized probe or PCR extension product"

<400>  34
acagcggctt tgcagtgct                                                    19


<210>  35
<211>  30
<212>  DNA
<213>  Artificial Sequence
```

<220>
<221> source
<223> /note="description of artificial sequence: an artificially synthesized primer for PCR"

<400> 35
acgttggatg gcaagcttgc ccaacttgtg 30

<210> 36
<211> 30
<212> DNA
<213> Artificial Sequence

<220>
<221> source
<223> /note="description of artificial sequence: an artificially synthesized primer for PCR"

<400> 36
acgttggatg gtttgtgttg ggctgcactc 30

<210> 37
<211> 17
<212> DNA
<213> Artificial Sequence

<220>
<221> source
<223> /note="description of artificial sequence: an artificially synthesized probe"

<400> 37
cgtgtggccc acgggct 17

<210> 38
<211> 18
<212> DNA
<213> Artificial Sequence

<220>
<221> source
<223> /note="description of artificial sequence: an artificially synthesized probe or PCR extension product"

<400> 38
cgtgtggccc acgggcta 18

<210> 39
<211> 18
<212> DNA
<213> Artificial Sequence

<220>
<221> source
<223> /note="description of artificial sequence: an artificially synthesized probe or PCR extension product"

<400> 39
cgtgtggccc acgggctg 18

<210> 40
<211> 101
<212> DNA
<213> Homo sapiens

<400> 40
tgtgtttttt taaaattttt taaaaaccaa tttcttaata ctttacattt yattattaat 60

atttatactt ctaagtattt ttcctaagga aaaagtactt c 101

<210> 41
<211> 101
<212> DNA
<213> Homo sapiens

<400> 41
tactgggaga aaaattatgg agcatcaact gggtaaacta taatacaaac raacgttaag 60

cgtgaaggga tagaaacata gggcagtgtt tgtgtaaaaa t 101

<210> 42
<211> 101
<212> DNA
<213> Homo sapiens

<400> 42
taaataattt catagaagtt cagtatagta cgtaattctt gtaagagtat ygtatgcaag 60

ctctctgtat gccacccact gttagttcaa attgagattt t 101

<210> 43
<211> 101
<212> DNA
<213> Homo sapiens

<400> 43
tgtatgccac ccactgttag ttcaaattga gatttttgtt ttgttttgtt yttaagaaca 60

taccaagaaa ataccagtga attgttggat atgaattccc t 101

<210> 44
<211> 101
<212> DNA
<213> Homo sapiens

<400> 44
aaattgagat ttttgttttg ttttgttctt aagaacatac caagaaaata ycagtgaatt 60

gttggatatg aattccctgt tagttgattt aaatcctttc t 101

<210> 45
<211> 101
<212> DNA
<213> Homo sapiens

<400> 45

gagcccagag ttttgagaca ggcctgggca acatagtgag accttgtctc yacaaaaagt 60

aaaatcagcc aggcatggtg tcacgcacct gtagccccag c 101

<210> 46
<211> 101
<212> DNA
<213> Homo sapiens

<400> 46
ggagcacctt cacatggcct ccatgtggct taggtttctc caagtatggc ygctgggtcc 60

ctggcgggaa ctgttgaaga gtgagtgtac caagactcag g 101

<210> 47
<211> 101
<212> DNA
<213> Homo sapiens

<400> 47
ttcaaaagtc agggaaaaga ggtaaaacca gcaaaagaga caggtagcct rtgagatggg 60

aagaagaaag tatcatgtcc tggaaccaaa ctgaagttgt t 101

<210> 48
<211> 101
<212> DNA
<213> Homo sapiens

<400> 48
ataagtgtgt tcctttaaag cactttaaat agttgctggc acatattggg yactcaaatg 60

atagcaatga tagcaattac tagctatcac tgcttcccaa a 101

<210> 49
<211> 101
<212> DNA
<213> Homo sapiens

<400> 49
tgggaaaaag aaggataata taggtatgtt tcaaaacaaa tatattttag yctgagatta 60

agcccttcca ccttttaaaa tgggttttct cctatgaaaa g 101

<210> 50
<211> 101
<212> DNA
<213> Homo sapiens

<400> 50
aacattagta gcatctggtt ggattttggg tttttttttt ccccctccct yaagtgttct 60

tagctcgcag aatagaagaa tggccatccc tgtgttggga t 101

<210> 51
<211> 101
<212> DNA

<213> Homo sapiens

<400> 51
agtttccaga gccatgagaa catttctaaa ctgacgtgac attgattcct maagcattct 60

tcaagtcatt tgcctgaatg aatgatttta ttcttatgtt a 101

<210> 52
<211> 101
<212> DNA
<213> Homo sapiens

<400> 52
tggcatggct acacggctaa atattattta atggcataat cgagtgctag ygccaggctt 60

gtattttgtg tgtgtgtgtg attacagatt gggagaattg g 101

<210> 53
<211> 101
<212> DNA
<213> Homo sapiens

<400> 53
ccatctcggc ctcccagagt gctgggatta caggcgtgag ccactgtgcc yggccgcaag 60

cagtattttc tgactgctgc agcttcttcc catatcttcc c 101

<210> 54
<211> 101
<212> DNA
<213> Homo sapiens

<400> 54
tatgtaaaga cttgaaggtt tgagaaagtc caaagcagtt tttggtccaa rgctgatgtg 60

actttgtacc tctgagttat ttgcgaccac agcctgggta t 101

<210> 55
<211> 101
<212> DNA
<213> Homo sapiens

<400> 55
atgcttaaaa tagtggcact tataagcacc aaataactat ttaatcagta ytattcaata 60

ctgttataat aaaaactaat ttcaggccag gcatggaggc t 101

<210> 56
<211> 101
<212> DNA
<213> Homo sapiens

<400> 56
tagtggcact tataagcacc aaataactat ttaatcagta ctattcaata ytgttataat 60

aaaaactaat ttcaggccag gcatggaggc tcacgcctgt a 101

<210> 57
<211> 101
<212> DNA
<213> Homo sapiens

<400> 57
ctgcaatctc cgcttcatag gctcaagtga ttctcatgcc tcagcctccc ragtagctgc 60

caccaggccc ggctaacttt tgtattttta gtagagacgg g 101

<210> 58
<211> 101
<212> DNA
<213> Homo sapiens

<400> 58
aacagatctt tgttttagaa agaagaaaag acaatccaga aacaaccagg kggactctag 60

acacccatgg aggaggtgat cagagctcat gagagtacca g 101

<210> 59
<211> 101
<212> DNA
<213> Homo sapiens

<400> 59
tagctctata gtaggcagaa atatccttta agttcattaa aggttctcag rtcttattta 60

aagaaatgta ccccaatgct agcaatgatt acaaccttgc a 101

<210> 60
<211> 101
<212> DNA
<213> Homo sapiens

<400> 60
cgttacacgt ttttgttgct aagttattcc atttccatgg cgagaaatgt ygatgaaaaa 60

caacactagc ttgaggctga tgcattaatc tttcatacat a 101

<210> 61
<211> 101
<212> DNA
<213> Homo sapiens

<400> 61
acaatccctt ttcttccacc tagaatcatg caatagaaat accagtctac rctcttcaaa 60

acaaaacccc cagacatcaa aagacagtat ctatgcatat c 101

<210> 62
<211> 101
<212> DNA
<213> Homo sapiens

<400> 62
ccagttgaaa aagggggaaa aaaatcaaat taccttcccc caacagatta waaaggaaat 60

accaaacagc cacaaagaag ctcgaaacct tttctatcgc c 101

<210> 63
<211> 101
<212> DNA
<213> Homo sapiens

<400> 63
ctctaaaagc caataccaat ttcagcactg ggtccactga aattctatcc raagatatca 60

gaacttcagt gatttgtgtc cctccaaaca ctatgcaaaa c 101

<210> 64
<211> 101
<212> DNA
<213> Homo sapiens

<400> 64
acatataaaa agaattatat acaatgccca agtgggattt ttttcaggaa kgcaagattg 60

gtttaacatc caaaaaccaa tgtaattact atatcatgtc a 101

<210> 65
<211> 101
<212> DNA
<213> Homo sapiens

<400> 65
taaaatggca gaagaaggat ttgacaaaaa tccaagctcc tttcgtgatg waaacactca 60

ggaaaatggg aattgcactg aatttcctca acctaataaa t 101

<210> 66
<211> 101
<212> DNA
<213> Homo sapiens

<400> 66
agaaattaaa gaagatctac taagtgggaa acatctcatg ttcatggatt rgaagactta 60

atattgttaa gatgttaatg tcccccaaat tgatctacag a 101

<210> 67
<211> 101
<212> DNA
<213> Homo sapiens

<400> 67
atacatgaaa gaacctcaaa aatattatgc tgagtgaaag aaattagtca yaaaagacta 60

caaattatgt attctattta tataaaatat ccatgagaca a 101

<210> 68
<211> 101
<212> DNA
<213> Homo sapiens

```
<400>  68
ataaggggtg atagctaaag gctatggagt ttattttgag acaatgacat rttctaaaat      60

tgaccgtgat gatgatggtt gcatatatct gtcaatatgc t                        101


<210>  69
<211>  101
<212>  DNA
<213>  Homo sapiens

<400>  69
tgtcaatatg ctacaaacca ctgaatcatc attttaaatg ggtgtggtat rtaaattata      60

tctcaataaa acttattttg aattaaaaca gaggtttagt c                        101


<210>  70
<211>  101
<212>  DNA
<213>  Homo sapiens

<400>  70
aataagaagt gcctctttct aaaaaagagc tctttgccta ctacctgcct ktcctcagaa      60

gaaagacatt gctttttgtt ttgttttgtt ttgtttgttt t                        101


<210>  71
<211>  101
<212>  DNA
<213>  Homo sapiens

<400>  71
ccttgaagct attactgacc aatcagaata ctatcactaa ggatctctct ygcctccttc      60

ctcccctcat ccctcagtct ctcttctcct tctccctctt c                        101
```